# EUROPEAN PATENT SPECIFICATION

(11) **EP 4 396 173 B1**
(45) Date of publication and mention of the grant of the patent: **12.08.2026**
(21) Application number: 22769969.1
(22) Date of filing: 30.08.2022
(51) Int. Cl.: C07D 333/72, C07D 317/66, C07D 333/66, C07D 209/08, C07C 235/74, C07D 263/56, C07D 277/64, C07D 307/82, C07D 317/46, A01N 43/12, A01N 43/76, A01N 37/36, A01N 43/38, A01N 43/78, A01N 43/30, C07C 235/16, A01P 13/00

(54) **HERBICIDAL MALONAMIDES CONTAINING A CONDENSED RING SYSTEM**
HERBIZIDE MALONAMIDE MIT EINEM KONDENSIERTEN RINGSYSTEM
MALONAMIDES HERBICIDES CONTENANT UN SYSTÈME CYCLIQUE CONDENSÉ

(30) Priority: 31.08.2021 EP 21193999
(43) Date of publication of application: 10.07.2024
(73) Proprietor: BASF Agricultural Solutions Deutschland GmbH, 67117 Limburgerhof (DE)
(72) Inventor: HEINRICH, Marc, 67056 Ludwigshafen (DE); KORDES, Markus, 67056 Ludwigshafen (DE); SEISER, Tobias, 67056 Ludwigshafen (DE); ZIMMERMANN, Gunther, 67056 Ludwigshafen (DE); NEWTON, Trevor William, 67117 Limburgerhof (DE); KRAEMER, Gerd, 67117 Limburgerhof (DE)
(74) Representative: BASF IP Association
(86) International application number: PCT/EP2022/074088
(87) International publication number: WO 2023/031200

(56) References cited:
- WO-A1-2012/130798
- WO-A1-2019/145245
- WO-A1-2021/170464
- WO-A2-87/05898
- CN-A- 112 624 989
- US-A- 3 072 473
- US-A1- 2005 054 633
- US-A1- 2006 122 168
- US-A1- 2007 225 273
- ZA-B- 9 692

## Description

The present invention relates to malonamide compounds containing a condensed ring system and compositions comprising the same. The invention also relates to the use of the malonamide compounds containing a condensed ring system or the corresponding compositions for controlling unwanted vegetation. Furthermore, the invention relates to methods of applying the malonamide compounds containing a condensed ring system or the corresponding compositions.

### BACKGROUND OF THE INVENTION

For the purpose of controlling unwanted vegetation, especially in crops, there is an ongoing need for new herbicides that have high activity and selectivity together with a substantial lack of toxicity for humans and animals.
WO 2012/130798, WO 2014/04882, WO 2014/048882, WO 2018/228985,
WO 2018/228986, WO 2019/034602, WO 2019/145245, WO 2020/114932,
WO 2020/114934 and WO 2020/182723 describe 3-phenylisoxazoline-5-carboxamides and their use as herbicides.

WO 87/05898 describes the use of malonic acid derivatives for retarding plant growth.

Malonic acid derivatives are also described in US 3,072,473 as plant growth regulants.

CN 112624989 A relates to compounds of following formula: 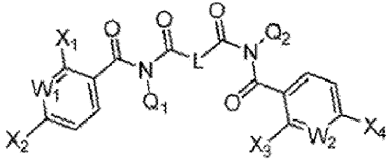 where W₁ and W₂ are nitrogen or carbon ring atoms, Q₁ and Q₂ are specific 5-membered hetaryl rings and L is a specific bridging group. The compounds are said to have herbicidal activity.

ZA 9692 B relates inter alia to malonsulfonylamides of formula R-SO₂-NH-CO-CH₂-CO-NR1R2, where R is phenyl, chlorophenyl or tolyl, R1 is H and R2 is butyl, phenyl, tolyl, chlorophenyl, bromophenyl, methoxyphenyl, benzyl, or pyridyl, or both R1 and R2 are methyl or both R1 and R2 are ethyl. The compounds are said to have herbicidal activity.

WO 2021/170464 relates to malonamide derivatives carrying an optionally substituted phenyl ring on one of the amide nitrogen atoms and an O-bound radical on the central carbon atom of the malonamide moiety of following formula: 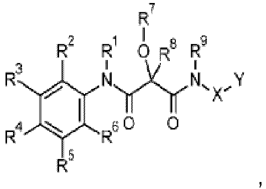 and to the use thereof as herbicides.

The compounds of the prior art often suffer from insufficient herbicidal activity, in particular at low application rates, and/or unsatisfactory selectivity resulting in a low compatibility with crop plants.

Accordingly, it is an object of the present invention to provide further malonamide compounds having a strong herbicidal activity, in particular even at low application rates, a sufficiently low toxicity for humans and animals and/or a high compatibility with crop plants. The malonamide compounds should also show a broad activity spectrum against a large number of different unwanted plants.

These and further objectives are achieved by the compounds of formula (I) defined below including their agriculturally acceptable salts, stereoisomers and tautomers.

### SUMMARY OF THE INVENTION

Accordingly, the present invention provides compounds of formula (I) 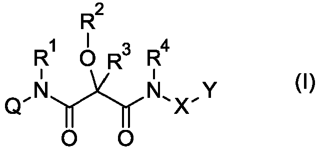 wherein the substituents have the following meanings:
- Q: is selected from bicyclic condensed ring systems of formula Q1 to Q13: 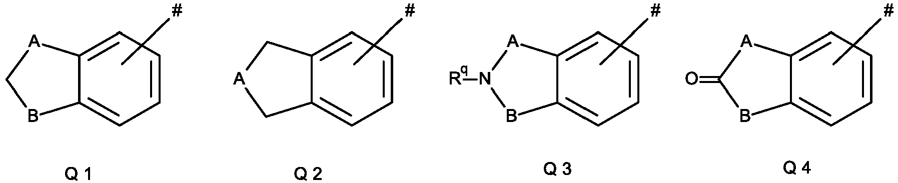 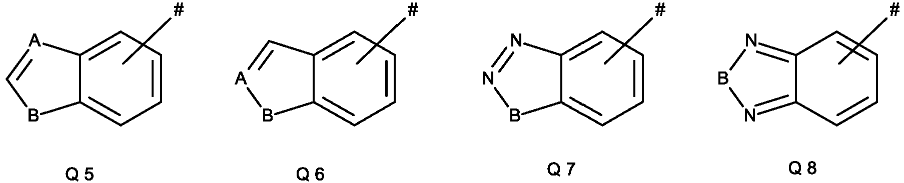 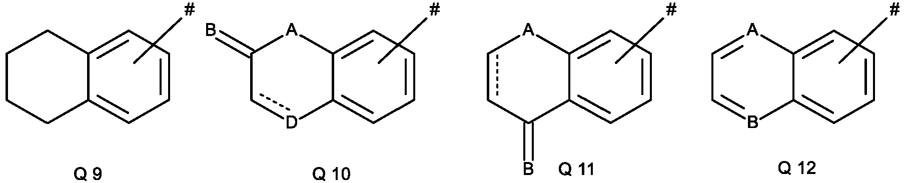 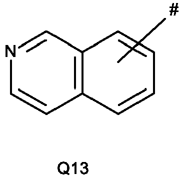
where

| | |
|---|---|
| in Q1: | A is CH₂, NR^{q}, O, S, S(O) or S(O)₂; and |
| | B is CH₂, NR^{q}, O, S, S(O) or S(O)₂; |
| in Q2: | A is NR^{q}, O, S, S(O) or S(O)₂; |
| in Q3: | A is CH₂, C(=O), NR^{q}, O, S, S(O) or S(O)₂; and |
| | B is CH₂, C(=O), NR^{q}, O, S, S(O) or S(O)₂; |
| in Q4: | A is CH₂, NR^{q}, O, S, S(O) or S(O)₂; and |
| | B is CH₂, NR^{q}, O, S, S(O) or S(O)₂; |
| in Q5: | A is CH or N; and |
| | B is CH₂, NR^{q}, O, S, S(O) or S(O)₂; |
| in Q6: | A is CH or N; and |
| | B is CH₂, NR^{q}, O, S, S(O) or S(O)₂; |
| in Q7: | B is CH₂, NR^{q}, O, S, S(O) or S(O)₂; |
| in Q8: | B is CH₂, NR^{q}, O, S, S(O) or S(O)₂; |
| in Q10: | the dashed line indicates a single bond or a double bond |
| | A is NR^{q} or O; |
| | B is O; and |
| | D is CH₂, NR^{q} or O if the dashed line indicates a single bond; and is CH or N the dashed line indicates a double bond; |
| in Q11: | the dashed line indicates a single bond or a double bond |
| | A is NR^{q} or O; and |
| | B is O; |
| in Q12: | A is CH or N; and |
| | B is CH or N; |

where
- R^{q}: is hydrogen, (C₁-C₃)-alkyl, (C₁-C₃)-haloalkyl, (C₁-C₃)-alkoxy, (C₁-C₃)-haloalkoxy, phenyl-(C₁-C₃)-alkyl, (C₁-C₄)-alkylcarbonyl or (C₁-C₄)-alkoxycarbonyl; and
- #: is the attachment point to NR¹;
where rings Q1 to Q13 carry k substituents R^{Q1}; where R^{Q1} is selected from the group consisting of halogen, cyano, (C₁-C₃)-alkyl, (C₁-C₃)-haloalkyl, (C₁-C₃)-alkoxy and (C₁-C₃)-haloalkoxy; where R^{q} is hydrogen or (C₁-C₃)-alkyl; and where k is 0, 1, 2 or 3;
- R¹: is hydrogen;
- R²: is (C₁-C₆)-alkyl;
- R³: is hydrogen or halogen;
- R⁴: is hydrogen;
- X: is a bond; and Y is Z; where Z is a five- or six-membered saturated or partly unsaturated carbocyclic ring which is substituted by m radicals CO₂R^{e}, where R^{e} is hydrogen or (C₁-C₆)-alkyl and m is 1 or 2; or
- X: is a divalent unit (X¹); and Y is (C₁-C₄)-alkyl which is substituted by m radicals CO₂R^{e}, where R^{e} is hydrogen or (C₁-C₆)-alkyl and m is 1 or 2;
where the divalent unit (X¹) has the following formula: 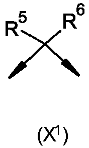
where R⁵ and R⁶ are independently of each other hydrogen or (C₁-C₆)-alkyl;
and the agriculturally acceptable salts, the stereoisomers and the tautomers thereof.

The invention also relates to a composition comprising at least one compound of formula (I) and at least one auxiliary which is customary for formulating crop protection compounds.

The present invention also provides combinations comprising at least one compound of formula (I) (component A) and at least one further compound selected from the herbicidal compounds B (component B; different from A) and safeners C (component C).

The invention relates moreover to the use of a compound of formula (I) or of said compositions for controlling unwanted vegetation, and to a method for controlling unwanted vegetation which comprises allowing a herbicidally effective amount of at least one compound of formula (I) or of said compositions to act on plants, their seed and/or their habitat.

### DETAILED DESCRIPTION OF THE INVENTION

### Definitions:

Depending on the kind of substituents, the compounds of formula (I) may have one or more centers of chirality, in which case they may be present as mixtures of enantiomers or diastereomers but also in the form of the pure enantiomers or pure diastereomers. The invention provides both the pure enantiomers or pure diastereomers of the compounds of formula I, and their mixtures and the use according to the invention of the pure enantiomers or pure diastereomers of the compound of formula I or its mixtures. Suitable compounds of formula I also include all possible geometrical stereoisomers (cis/trans isomers) as a specific form of diastereomers and mixtures thereof. Cis/trans isomers may be present with respect to an alkene, carbon-nitrogen double-bond, nitrogen-sulfur double bond, amide group or a cyclic, non-aromatic moiety. The term "stereoisomer(s)" encompasses both optical isomers, such as enantiomers or diastereomers existing due to more than one stereogenic center in the molecule, as well as geometrical isomers (cis/trans isomers). Just by way of example, a stereogenic center is the C atom carrying R⁵ and R⁶ in X¹ to X⁶, provided of course that R⁵ and R⁶ are different. Another example for a stereogenic center is the C atom carrying OR² and R³.

If the above-mentioned herbicidal compounds B and/or the safeners C have one or more stereogenic centres they may also be present as enantiomers or diastereomers, and it is possible to use both the pure enantiomers and diastereomers or their mixtures.

If the compounds of formula (I), the herbicidal compounds B and/or the safeners C as described herein have ionizable functional groups, they can also be employed in the form of their agriculturally acceptable salts. Suitable are, in general, the salts of those cations and the acid addition salts of those acids whose cations and anions, respectively, have no adverse effect on the activity of the active compounds.

Preferred cations are the ions of the alkali metals, preferably of lithium, sodium and potassium, of the alkaline earth metals, preferably of calcium and magnesium, and of the transition metals, preferably of manganese, copper, zinc and iron, further ammonium and substituted ammonium in which one to four hydrogen atoms are replaced by C₁-C₄-alkyl, hydroxy-C₁-C₄-alkyl, C₁-C₄-alkoxy-C₁-C₄-alkyl, hydroxy-C₁-C₄-alkoxy-C₁-C₄-alkyl, phenyl or benzyl, preferably ammonium, methylammonium, isopropylammonium, dimethylammonium, diethylammonium, diisopropylammonium, trimethylammonium, triethylammonium, tris(isopropyl)ammonium, heptylammonium, dodecylammonium, tetradecylammonium, tetramethylammonium, tetraethylammonium, tetrabutylammonium, 2-hydroxyethylammonium (olamine salt), 2-(2-hydroxyeth-1-oxy)eth-1-ylammonium (diglycolamine salt), di(2-hydroxyeth-1-yl)ammonium (diolamine salt), tris(2-hydroxyethyl)ammonium (trolamine salt), tris(2-hydroxypropyl)ammonium, benzyltrimethylammonium, benzyltriethylammonium, N,N,N-trimethylethanolammonium (choline salt), furthermore phosphonium ions, sulfonium ions, preferably tri(C₁-C₄-alkyl)sulfonium, such as trimethylsulfonium, and sulfoxonium ions, preferably tri(C₁-C₄-alkyl)sulfoxonium, and finally the salts of polybasic amines such as N,N-bis-(3-aminopropyl)methylamine and diethylenetriamine.

Anions of useful acid addition salts are primarily chloride, bromide, fluoride, iodide, hydrogensulfate, methylsulfate, sulfate, dihydrogenphosphate, hydrogen phosphate, nitrate, bicarbonate, carbonate, hexafluorosilicate, hexafluorophosphate, benzoate and also the anions of C₁-C₄-alkanoic acids, preferably formate, acetate, propionate and butyrate.

The compounds (I) may be present in form of different tautomers. For instance, the depicted keto form of the malonamide moiety N(R¹)-C(=O)-C(OR²)(R³)-C(=O)-N(R⁴) can be in equilibrium with its enol forms N(R¹)-C(OH)=C(OR²)-C(=O)-N(R⁴) and N(R¹)-C(=O)-C(OR²)=C(OH)-N(R⁴) (keto-enol tautomery) if R³ is hydrogen. The same applies if ring Z contains a C(=O) group as ring member neighboured to a CH ring member.

Also if R^{Q1} is hydroxyl ring Q can be present in the corresponding keto form. For instance, if Q is contains a condensed pyridine ring carrying in 2-position to the nitrogen ring atom an OH group, ring Q can be present as its tautomeric form 1,2-dihydropyridin-2-one (condensed to another ring, of course).

Moreover, if ring Z is a lactam, i.e. contains an amide group as ring member (= unsubstituted, secondary nitrogen ring atom neighboured to a carbon ring atom carrying an oxo group), this ring moiety -N(H)-C(=O)- can be in equilibrium with its tautomeric form -N=C(OH)-.

The same applies to the two mandatorily present amide groups of the malonamide moiety -N(R¹)-C(=O)-C(OR²)(R³)-C(=O)-N(R⁴)- if one or both of R¹ and R⁴ are hydrogen:
If only R¹ is hydrogen, the malonamide moiety can be present as
   -N(H)-C(=O)-C(OR²)(R³)-C(=O)-N(R⁴)- or as -N=C(OH)-C(OR²)(R³)-C(=O)-N(R⁴)- or as a mixture of the two forms;
If only R⁴ is hydrogen, the malonamide moiety can be present as
   -N(R¹)-C(=O)-C(OR²)(R³)-C(=O)-N(H)- or as -N(R¹)-C(=O)-C(OR²)(R³)-C(OH)=Nor as a mixture of the two forms;
If both of R¹ and R⁴ are hydrogen, the malonamide moiety can be present as
   -N(H)-C(=O)-C(OR²)(R³)-C(=O)-N(H)- or as -N=C(OH)-C(OR²)(R³)-C(=O)-N(H)- or as
   -N(H)-C(=O)-C(OR²)(R³)-C(OH)=N- or as -N=C(OH)-C(OR²)(R³)-C(OH)=N- or as mixture of two, three all four of the above forms.

The amount in which the one or other tautomeric form is present depends on the complete molecular structure and even stronger on the surrounding conditions (presence or absence of solvent, type of solvent, pH, temperature etc.).

The term "undesired vegetation" ("weeds") is understood to include any vegetation growing in non-crop-areas or at a crop plant site or locus of seeded and otherwise desired crop, where the vegetation is any plant species, including their germinant seeds, emerging seedlings and established vegetation, other than the seeded or desired crop (if any). Weeds, in the broadest sense, are plants considered undesirable in a particular location.

The organic moieties mentioned in the above definitions of the variables are - like the term halogen - collective terms for individual listings of the individual group members. The prefix Cₙ-Cₘ indicates in each case the possible number of carbon atoms in the group.

The term "halogen" denotes in each case fluorine, bromine, chlorine or iodine, in particular fluorine, chlorine or bromine.

The term "partially or completely halogenated" will be taken to mean that 1 or more, e.g. 1, 2, 3, 4 or 5 or all of the hydrogen atoms of a given radical have been replaced by a halogen atom, in particular by fluorine or chlorine. A partially or completely halogenated radical is termed below also "halo-radical". For example, partially or completely halogenated alkyl is also termed haloalkyl.

The term "alkyl" as used herein (and in the alkyl moieties of other groups comprising an alkyl group, e.g. alkoxy, alkylamino, dialkylamino, alkylcarbonyl, alkoxycarbonyl, alkylthio, alkylsulfonyl and alkoxyalkyl) denotes in each case a straight-chain or branched alkyl group having usually from 1 to 12 carbon atoms (= C₁-C₁₂-alkyl), frequently from 1 to 6 carbon atoms (= C₁-C₆-alkyl), in particular 1 to 4 carbon atoms (= C₁-C₄-alkyl) and especially from 1 to 3 carbon atoms (= C₁-C₃-alkyl) or 1 or 2 carbon atoms (= C₁-C₂-alkyl). C₁-C₂-Alkyl is methyl or ethyl. C₁-C₃-Alkyl is methyl, ethyl, n-propyl or iso-propyl. Examples of C₁-C₄-alkyl are methyl, ethyl, n-propyl, iso-propyl, n-butyl, 2-butyl (= sec-butyl), isobutyl and tert-butyl. Examples for C₁-C₆-alkyl are, in addition to those mentioned for C₁-C₄-alkyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. Examples for C₁-C₈-alkyl are, in addition to those mentioned for C₁-C₆-alkyl, n-heptyl, 1-methylhexyl, 2-methylhexyl, 3-methylhexyl, 4-methylhexyl, 5-methylhexyl, 1-ethylpentyl, 2-ethylpentyl, 3-ethylpentyl, n-octyl, 1-methylheptyl, 2-methylheptyl, 1-ethylhexyl, 2-ethylhexyl, 1,2-dimethylhexyl, 1-propylpentyl and 2-propylpentyl. Examples for C₁-C₁₂-alkyl are, apart those mentioned for C₁-C₈-alkyl, nonyl, decyl, 2-propylheptyl, 3-propylheptyl, undecyl, dodecyl and positional isomers thereof.

The term "haloalkyl" as used herein (and in the haloalkyl moieties of other groups comprising a haloalkyl group, e.g. haloalkoxy, haloalkylthio, haloalkylcarbonyl, haloalkylsulfonyl and haloalkylsulfinyl), which is also expressed as "alkyl which is partially or fully halogenated", denotes in each case a straight-chain or branched alkyl group having usually from 1 to 6 carbon atoms (= C₁-C₆-haloalkyl), more frequently 1 to 3 carbon atoms (= C₁-C₃-haloalkyl), as defined above, wherein the hydrogen atoms of this group are partially or totally replaced with halogen atoms. Preferred haloalkyl moieties are selected from C₁-C₃-haloalkyl, specifically from C₁-C₂-haloalkyl, in particular from fluorinated C₁-C₂-alkyl. Examples for C₁-C₂-haloalkyl are fluoromethyl, difluoromethyl, trifluoromethyl, chloromethyl, dichloromethyl, trichloromethyl, chlorofluoromethyl, dichlorofluoromethyl, chlorodifluoromethyl, bromomethyl,1-fluoroethyl, 2-fluoroethyl, 2,2-difluoroethyl, 2,2,2-trifluoroethyl, pentafluoroethyl, 1-chloroethyl, 2-chloroethyl, 2,2,-dichloroethyl, 2,2,2-trichloroethyl, 2-chloro-2-fluoroethyl, 2-chloro-2,2-difluoroethyl, 2,2-dichloro-2-fluoroethyl, or 1-bromoethyl. Examples for C₁-C₃-haloalkyl are, in addition to those mentioned for C₁-C₂-haloalkyl, 1-fluoropropyl, 2-fluoropropyl, 3-fluoropropyl, 3,3-difluoropropyl, 3,3,3-trifluoropropyl, heptafluoropropyl, 1,1,1-trifluoroprop-2-yl, or 3-chloropropyl.

The term "hydroxyalkyl" denotes in each case a straight-chain or branched alkyl group having usually from 1 to 6 carbon atoms (= C₁-C₆-hydroxyalkyl), more frequently 1 to 3 carbon atoms (= C₁-C₃-hydroxyalkyl), as defined above, wherein one hydrogen atom of this group is replaced with a hydroxyl group. Examples are hydroxymethyl, 1-hydroxyethyl, 2-hydroxyethyl, 1-hydroxypropyl, 2-hydroxypropyl, 3-hydroxypropyl, or 1-hydroxy-2-propyl.

The term "cyanoalkyl" denotes in each case a straight-chain or branched alkyl group having usually from 1 to 6 carbon atoms (= C₁-C₆-cyanoalkyl), more frequently 1 to 3 carbon atoms (= C₁-C₃-cyanoalkyl), as defined above, wherein one hydrogen atom of this group is replaced with a cyano group. Examples are cyanomethyl, 1-cyanoethyl, 2-cyanoethyl, 1-cyanopropyl, 2-cyanopropyl, 3-cyanopropyl, or 1-cyano-2-propyl.

The term "alkenyl" as used herein denotes in each case a monounsaturated straight-chain or branched hydrocarbon radical having usually 2 to 12 (= C₂-C₁₂-alkenyl), preferably 2 to 6 carbon atoms (= C₂-C₆-alkenyl), e.g. 3 to 6 carbon atoms (= C₃-C₆-alkenyl), in particular 2 to 4 carbon atoms (= C₂-C₄-alkenyl) or 2 or 3 carbon atoms (= C₂-C₃-alkenyl), and a double bond in any position, for example C₂-C₃-alkenyl, such as ethenyl, 1-propenyl, 2-propenyl or 1-methylethenyl; C₂-C₄-alkenyl, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl or 2-methyl-2-propenyl; C₂-C₆-alkenyl, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, or 1-ethyl-2-methyl-2-propenyl; or C₂-C₁₂-alkenyl, such as the radicals mentioned for C₂-C₆-alkenyl and additionally 1-heptenyl, 2-heptenyl, 3-heptenyl, 1-octenyl, 2-octenyl, 3-octenyl, 4-octenyl, the nonenyls, decenyls, undecenyls, dodecenyls and the positional isomers thereof. Examples for C₃-C₆-alkenyl are those mentioned above for C₂-C₆-alkenyl, except for ethenyl.

The term "haloalkenyl" as used herein, which may also be expressed as "alkenyl which is substituted by halogen", and the haloalkenyl moieties in haloalkenyloxy refers to unsaturated straight-chain or branched hydrocarbon radicals having 2 to 6 (= C₂-C₆-haloalkenyl) or 2 to 4 (= C₂-C₄-haloalkenyl) or 2 to 3 (= C₂-C₃-haloalkenyl) carbon atoms and a double bond in any position, where some or all of the hydrogen atoms in these groups are replaced by halogen atoms as mentioned above, in particular fluorine, chlorine and bromine, for example chlorovinyl, or chloroallyl.

The term "alkynyl" as used herein denotes unsaturated straight-chain or branched hydrocarbon radicals having usually 2 to 12 (= C₂-C₁₂-alkynyl), frequently 2 to 6 (= C₂-C₆-alkynyl), preferably 2 to 4 carbon atoms (= C₂-C₄-alkynyl) or 2 to 3 carbon atoms (= C₂-C₃-alkynyl) and a triple bond in any position, for example C₂-C₃-alkynyl, such as ethynyl, 1-propynyl or 2-propynyl; C₂-C₄-alkynyl, such as ethynyl, 1-propynyl or 2-propynyl, 1-butynyl, 2-butynyl, 3-butynyl, or 1-methyl-2-propynyl; C₂-C₆-alkynyl, such as ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl, or 1-ethyl-1-methyl-2-propynyl.

The term "haloalkynyl" as used herein, which is also expressed as "alkynyl which is substituted by halogen", refers to unsaturated straight-chain or branched hydrocarbon radicals having usually 2 to 6 carbon atoms (= C₂-C₆-haloalkynyl), preferabyl 2 to 4 carbon atoms (= C₂-C₄-haloalkynyl) or 2 or 3 carbon atoms (= C₂-C₃-haloalkynyl), and a triple bond in any position (as mentioned above), where some or all of the hydrogen atoms in these groups are replaced by halogen atoms as mentioned above, in particular fluorine, chlorine and bromine.

The term "cycloalkyl" as used herein (and in the cycloalkyl moieties of other groups comprising a cycloalkyl group, e.g. cycloalkoxy and cycloalkylalkyl) denotes in each case a mono- or bicyclic, saturated cycloaliphatic radical having usually from 3 to 6 carbon atoms (= C₃-C₆-cycloalkyl), 3 to 5 carbon atoms (= C₃-C₅-cycloalkyl) or 3 to 4 carbon atoms (= C₃-C₄-cycloalkyl) **as (only) ring members.** Examples of monocyclic saturated cycloaliphatic radicals having 3 or 4 carbon atoms comprise cyclopropyl and cyclobutyl. Examples of monocyclic saturated cycloaliphatic radicals having 3 to 5 carbon atoms comprise cyclopropyl, cyclobutyl and cyclopentyl. Examples of monocyclic saturated cycloaliphatic radicals having 3 to 6 carbon atoms comprise cyclopropyl, cyclobutyl, cyclopentyl and cyclohexyl. C₅-C₆-Cycloalkyl is cyclopentyl or cyclohexyl. Examples of bicyclic radicals having 5 or 6 carbon atoms comprise bicyclo[1.1.1]pentyl and bicyclo[2.1.1]hexyl. Preferably, cycloalkyl is monocyclic.

The term "halocycloalkyl" as used herein (and in the halocycloalkyl moieties of other groups comprising an halocycloalkyl group) denotes in each case a mono- or bicyclic cycloaliphatic radical having usually from 3 to 8 carbon atoms ("C₃-C₈-halocycloalkyl"), preferably 3 to 5 carbon atoms ("C₃-C₅-halocycloalkyl"), wherein at least one, e.g. 1, 2, 3, 4 or 5 of the hydrogen atoms are replaced by halogen, in particular by fluorine or chlorine. Examples are 1- and 2- fluorocyclopropyl, 1,2-, 2,2- and 2,3-difluorocyclopropyl, 1,2,2-trifluorocyclopropyl, 2,2,3,3-tetrafluorocyclpropyl, 1- and 2-chlorocyclopropyl, 1,2-, 2,2- and 2,3-dichlorocyclopropyl, 1,2,2-trichlorocyclopropyl, 2,2,3,3-tetrachlorocyclpropyl, 1-,2- and 3-fluorocyclopentyl, 1,2-, 2,2-, 2,3-, 3,3-, 3,4-, 2,5-difluorocyclopentyl, 1-,2- and 3-chlorocyclopentyl, 1,2-, 2,2-, 2,3-, 3,3-, 3,4-, or 2,5-dichlorocyclopentyl.

The term "alkoxy" as used herein denotes in each case a straight-chain or branched alkyl group usually having from 1 to 6 carbon atoms (= C₁-C₆-alkoxy), preferably 1 to 3 carbon atoms (= C₁-C₃-alkoxy), in particular 1 or 2 carbon atoms (= C₁-C₂-alkoxy), which is bound to the remainder of the molecule via an oxygen atom. C₁-C₂-Alkoxy is methoxy or ethoxy. C₁-C₃-Alkoxy is additionally, for example, n-propoxy or 1-methylethoxy (isopropoxy). C₁-C₆-Alkoxy is additionally, for example, butoxy, 1-methylpropoxy (sec-butoxy), 2-methylpropoxy (isobutoxy) or 1,1-dimethylethoxy (tert-butoxy), pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy.

The term "haloalkoxy" as used herein denotes in each case a straight-chain or branched alkoxy group, as defined above, having from 1 to 6 carbon atoms (= C₁-C₆-haloalkoxy), preferably 1 to 3 carbon atoms (= C₁-C₃-haloalkoxy), in particular 1 or 2 carbon atoms (= C₁-C₂-haloalkoxy), wherein the hydrogen atoms of this group are partially or totally replaced with halogen atoms, in particular fluorine atoms (in this case, the radical is also termed fluorinated alkoxy). C₁-C₂-Haloalkoxy is, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromo-ethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy or OC₂F_{5.} C₁-C₃-Haloalkoxy is additionally, for example, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy or 1-(CH₂Br)-2-bromoethoxy. C₁-C₆-Haloalkoxy is additionally, for example, 4-fluorobutoxy, 4-chlorobutoxy, 4-bromobutoxy or nonafluorobutoxy , 5-fluoropentoxy, 5-chloropentoxy, 5-brompentoxy, 5-iodopentoxy, undecafluoropentoxy, 6-fluorohexoxy, 6-chlorohexoxy, 6-bromohexoxy, 6-iodohexoxy or dodecafluorohexoxy.

The term "cyanoalkoxy" denotes in each case a straight-chain or branched alkyl group having usually from 1 to 6 carbon atoms (= C₁-C₆-cyanoalkoxy), more frequently 1 to 3 carbon atoms (= C₁-C₃-cyanoalkoxy), as defined above, wherein one hydrogen atom of this group is replaced with a cyano group. Examples are cyanomethoxy, 1-cyanoethoxy, 2-cyanoethoxy, 1-cyanopropoxy, 2-cyanopropoxy, 3-cyanopropoxy, or 1-cyano-2-propoxy.

The term "alkenyloxy" denotes an alkenyl group, as defined above, attached via an oxygen atom to the remainder of the molecule. C₂-C₆-Alkenyloxy is a C₂-C₆-alkenyl group, as defined above, attached via an oxygen atom to the remainder of the molecule. C₃-C₆-Alkenyloxy is a C₃-C₆-alkenyl group, as defined above, attached via an oxygen atom to the remainder of the molecule.

The term "haloalkenyloxy" denotes a haloalkenyl group, as defined above, attached via an oxygen atom to the remainder of the molecule. C₂-C₆-Haloalkenyloxy is a C₂-C₆-haloalkenyl group, as defined above, attached via an oxygen atom to the remainder of the molecule. C₃-C₆-Haloalkenyloxy is a C₃-C₆-haloalkenyl group, as defined above, attached via an oxygen atom to the remainder of the molecule.

The term "alkynyloxy" denotes an alkynyl group, as defined above, attached via an oxygen atom to the remainder of the molecule. C₂-C₆-Alkynyloxy is a C₂-C₆-alkynyl group, as defined above, attached via an oxygen atom to the remainder of the molecule. C₃-C₆-Alkynyloxy is a C₃-C₆-alkynyl group, as defined above, attached via an oxygen atom to the remainder of the molecule.

The term "haloalkynyloxy" denotes a haloalkynyl group, as defined above, attached via an oxygen atom to the remainder of the molecule. C₂-C₆-Haloalkynyloxy is a C₂-C₆-haloalkynyl group, as defined above, attached via an oxygen atom to the remainder of the molecule. C₃-C₆-Haloalkynyloxy is a C₃-C₆-haloalkynyl group, as defined above, attached via an oxygen atom to the remainder of the molecule.

The term "cycloalkoxy" denotes a cycloalkyl group, as defined above, attached via an oxygen atom to the remainder of the molecule. C₃-C₆-Cycloalkoxy is a C₃-C₆-cycloalkyl group, as defined above, attached via an oxygen atom to the remainder of the molecule. Examples of C₃-C₆-cycloalkoxy comprise cyclopropoxy, cyclobutoxy, cyclopentoxy and cyclohexoxy.

The term "C₁-C₃-alkoxy-C₁-C₃-alkyl" denotes an alkyl group having 1 to 3 carbon atoms, as defined above, where one hydrogen atom is replaced by a C₁-C₃-alkoxy group, as defined above. Examples are methoxymethyl, ethoxymethyl, propoxymethyl, isopropoxymethyl, 1-methoxyethyl, 1-ethoxyethyl, 1-propoxyethyl, 1-isopropoxyethyl, 2-methoxyethyl, 2-ethoxyethyl, 2-propoxyethyl, 2-isopropoxyethyl, 1-methoxypropyl, 1-ethoxypropyl, 1-propoxypropyl, 1-isopropoxypropyl, 2-methoxypropyl, 2-ethoxypropyl, 2-propoxypropyl, 2-isopropoxypropyl, 3-methoxypropyl, 3-ethoxypropyl, 3-propoxypropyl, or 3-isopropoxypropyl,.

The term "C₃-C₅-cycloalkyl-C₁-C₃-alkoxy" as used herein, refers to an alkoxy group having 1 to 3 carbon atoms, as defined above, where one hydrogen atom is replaced by a C₃-C₅-cycloalkyl group, as defined above. Examples are cyclopropyl-methoxy, cyclobutylmethoxy, cyclopentylmethoxy, 1-cyclopropylethoxy, 2-cyclopropylethoxy, 1-cyclobutylethoxy, 2-cyclobutylethoxy, 1-cyclopentylethoxy, 2-cyclopentylethoxy, 1-cyclopropylpropoxy, 2-cyclopropylpropoxy, 3-cyclopropylpropoxy, 1-cyclobutylpropoxy, 2-cyclobutylpropoxy, 3-cyclobutylpropoxy, 1-cyclopentylpropoxy, 2-cyclopentylpropoxy, or 3-cyclopentylpropoxy.

The term "alkoxy-alkoxy" as used herein, refers to an alkoxy group, as defined above, where one hydrogen atom is replaced by another alkoxy group, as defined above. The term "C₁-C₃-alkoxy-C₁-C₃-alkoxy" as used herein, refers to an alkoxy group having 1 to 3 carbon atoms, as defined above, where one hydrogen atom is replaced by a C₁-C₃-alkoxy group, as defined above. Examples are methoxymethoxy, ethoxymethoxy, propoxymethoxy, isopropoxymethoxy, 1-methoxyethoxy, 1-ethoxyethoxy, 1-propoxyethoxy, 1-isopropoxyethoxy, 2-methoxyethoxy, 2-ethoxyethoxy, 2-propoxyethoxy, 2-isopropoxyethoxy, 1-methoxypropoxy, 1-ethoxypropoxy, 1-propoxypropoxy, 1-isopropoxypropoxy, 2-methoxypropoxy, 2-ethoxypropoxy, 2-propoxypropoxy, 2-isopropoxypropoxy, 3-methoxypropoxy, 3-ethoxypropoxy, 3-propoxypropoxy, or 3-isopropoxypropoxy,.

The term "alkylthio" (also alkylsulfanyl, "alkyl-S" or "alkyl-S(O)ₖ" (wherein k is 0) as used herein denotes in each case a straight-chain or branched saturated alkyl group as defined above, usually comprising 1 to 6 carbon atoms (= C₁-C₆-alkylthio), preferably 1 to 3 carbon atoms (= C₁-C₃-alkylthio), which is attached via a sulfur atom at any position in the alkyl group. C₁-C₂-Alkylthio is methylthio or ethylthio. C₁-C₃-Alkylthio is additionally, for example, n-propylthio or 1-methylethylthio (isopropylthio). C₁-C₆-Alkylthio is additionally, for example, butylthio, 1-methylpropylthio (sec-butylthio), 2-methylpropylthio (isobutylthio), 1,1-di-methylethylthio (tert-butylthio), pentylthio, 1-methylbutylthio, 2-methylbutylthio, 3-methylbutylthio, 1,1-dimethylpropylthio, 1,2-dimethylpropylthio, 2,2-dimethylpropylthio, 1-ethylpropylthio, hexylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, 2-ethylbutylthio, 1,1,2-trimethylpropylthio, 1,2,2-trimethylpropylthio, 1-ethyl-1-methylpropylthio or 1-ethyl-2-methylpropylthio.

The term "haloalkylthio" as used herein refers to an alkylthio group as defined above wherein the hydrogen atoms are partially or completely substituted by fluorine, chlorine, bromine and/or iodine. C₁-C₂-Haloalkylthio is, for example, SCH₂F, SCHF₂, SCF₃, SCH₂Cl, SCHCl₂, SCCl₃, chlorofluoromethylthio, dichlorofluoromethylthio, chlorodifluoromethylthio, 2-fluoroethylthio, 2-chloroethylthio, 2-bromoethylthio, 2-iodoethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio or SC₂F₅. C₁-C₄-Haloalkylthio is additionally, for example, 2-fluoropropylthio, 3-fluoropropylthio, 2,2-difluoropropylthio, 2,3-difluoropropylthio, 2-chloropropylthio, 3-chloropropylthio, 2,3-dichloropropylthio, 2-bromopropylthio, 3-bromopropylthio, 3,3,3-trifluoropropylthio, 3,3,3-trichloropropylthio, SCH₂-C₂F₅, SCF₂-C₂F₅, 1-(CH₂F)-2-fluoroethylthio, 1-(CH₂Cl)-2-chloroethylthio, 1-(CH₂Br)-2-bromoethylthio, 4-fluorobutylthio, 4-chlorobutylthio, 4-bromobutylthio or nonafluorobutylthio. C₁-C₆-Haloalkylthio is additionally, for example, 5-fluoropentylthio, 5-chloropentylthio, 5-brompentylthio, 5-iodopentylthio, undecafluoropentylthio, 6-fluorohexylthio, 6-chlorohexylthio, 6-bromohexylthio, 6-iodohexylthio or dodecafluorohexylthio.

The terms "alkylsulfinyl" and "alkyl-S(O)ₖ" (wherein k is 1) are equivalent and, as used herein, denote an alkyl group, as defined above, attached via a sulfinyl [S(O)] group. For example, the term "C₁-C₂-alkylsulfinyl" refers to a C₁-C₂-alkyl group, as defined above, attached via a sulfinyl [S(O)] group. The term "C₁-C₃-alkylsulfinyl" refers to a C₁-C₃-alkyl group, as defined above, attached via a sulfinyl [S(O)] group. The term "C₁-C₆-alkylsulfinyl" refers to a C₁-C₆-alkyl group, as defined above, attached via a sulfinyl [S(O)] group. C₁-C₂-alkylsulfinyl is methylsulfinyl or ethylsulfinyl. C₁-C₃-alkylsulfinyl is additionally, for example, n-propylsulfinyl or 1-methylethylsulfinyl (isopropylsulfinyl). C₁-C₆-alkylsulfinyl is additionally, for example, butylsulfinyl, 1-methylpropylsulfinyl (sec-butylsulfinyl), 2-methylpropylsulfinyl (isobutylsulfinyl), 1,1-dimethylethylsulfinyl (tert-butylsulfinyl), pentylsulfinyl, 1-methylbutylsulfinyl, 2-methylbutylsulfinyl, 3-methylbutylsulfinyl, 1,1-dimethylpropylsulfinyl, 1,2-dimethylpropylsulfinyl, 2,2-dimethylpropylsulfinyl, 1-ethylpropylsulfinyl, hexylsulfinyl, 1-methylpentylsulfinyl, 2-methylpentylsulfinyl, 3-methylpentylsulfinyl, 4-methylpentylsulfinyl, 1,1-dimethylbutylsulfinyl, 1,2-dimethylbutylsulfinyl, 1,3-dimethylbutylsulfinyl, 2,2-dimethylbutylsulfinyl, 2,3-dimethylbutylsulfinyl, 3,3-dimethylbutylsulfinyl, 1-ethylbutylsulfinyl, 2-ethylbutylsulfinyl, 1,1,2-trimethylpropylsulfinyl, 1,2,2-trimethylpropylsulfinyl, 1-ethyl-1-methylpropylsulfinyl or 1-ethyl-2-methylpropylsulfinyl.

The terms "alkylsulfonyl" and "alkyl-S(O)ₖ" (wherein k is 2) are equivalent and, as used herein, denote an alkyl group, as defined above, attached via a sulfonyl [S(O)₂] group. The term "C₁-C₂-alkylsulfonyl" refers to a C₁-C₂-alkyl group, as defined above, attached via a sulfonyl [S(O)₂] group. The term "C₁-C₃-alkylsulfonyl" refers to a C₁-C₃-alkyl group, as defined above, attached via a sulfonyl [S(O)₂] group. The term "C₁-C₆-alkylsulfonyl" refers to a C₁-C₆-alkyl group, as defined above, attached via a sulfonyl [S(O)₂] group. C₁-C₂-alkylsulfonyl is methylsulfonyl or ethylsulfonyl. C₁-C₃-alkylsulfonyl is additionally, for example, n-propylsulfonyl or 1-methylethylsulfonyl (isopropylsulfonyl). C₁-C₆-alkylsulfonyl is additionally, for example, butylsulfonyl, 1-methylpropylsulfonyl (sec-butylsulfonyl), 2-methylpropylsulfonyl (isobutylsulfonyl), 1,1-dimethylethylsulfonyl (tert-butylsulfonyl), pentylsulfonyl, 1-methylbutylsulfonyl, 2-methylbutylsulfonyl, 3-methylbutylsulfonyl, 1,1-dimethylpropylsulfonyl, 1,2-dimethylpropylsulfonyl, 2,2-dimethylpropylsulfonyl, 1-ethylpropylsulfonyl, hexylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3-dimethylbutylsulfonyl, 2,2-dimethylbutylsulfonyl, 2,3-dimethylbutylsulfonyl, 3,3-dimethylbutylsulfonyl, 1-ethylbutylsulfonyl, 2-ethylbutylsulfonyl, 1,1,2-trimethylpropylsulfonyl, 1,2,2-trimethylpropylsulfonyl, 1-ethyl-1-methylpropylsulfonyl or 1-ethyl-2-methylpropylsulfonyl.

The substituent "oxo" replaces a CH₂ group by a C(=O) group.

The suffix "-carbonyl" in a group denotes in each case that the group is bound to the remainder of the molecule via a carbonyl (C=O) group. This is the case e.g. in alkylcarbonyl, haloalkylcarbonyl, aminocarbonyl, alkylaminocarbonyl, dialkylaminocarbonyl, alkoxycarbonyl, haloalkoxycarbonyl.

The term "alkylcarbonyl" denotes an alkyl group, as defined above, attached via a carbonyl [C(=O)] group to the remainder of the molecule. C₁-C₃-Alkylcarbonyl is a C₁-C₃-alkyl group, as defined above, attached via a carbonyl [C(=O)] group to the remainder of the molecule. C₁-C₄-Alkylcarbonyl is a C₁-C₄-alkyl group, as defined above, attached via a carbonyl [C(=O)] group to the remainder of the molecule. Examples for C₁-C₃-alkylcarbonyl are acetyl (methylcarbonyl), propionyl (ethylcarbonyl), propylcarbonyl and isopropylcarbonyl. Examples for C₁-C₄-alkylcarbonyl are acetyl (methylcarbonyl), propionyl (ethylcarbonyl), propylcarbonyl, isopropylcarbonyl or n-butylcarbonyl.

The term "alkoxycarbonyl" denotes an alkoxy group, as defined above, attached via a carbonyl [C(=O)] group to the remainder of the molecule. C₁-C₃-Alkoxycarbonyl is a C₁-C₃-alkoxy group, as defined above, attached via a carbonyl [C(=O)] group to the remainder of the molecule. Examples for C₁-C₃-alkoxycarbonyl are methoxycarbonyl, ethoxycarbonyl, propoxycarbonyl and isopropoxycarbonyl. C₁-C₆-Alkoxycarbonyl is a C₁-C₆-alkoxy group, as defined above, attached via a carbonyl [C(=O)] group to the remainder of the molecule. Examples for C₁-C₆-alkoxycarbonyl are, in addition to those listed for C₁-C₃-alkoxycarbonyl, n-butoxycarbonyl, sec-butoxycarbonyl, isobutoxycarbonyl, tert-butoxycarbonyl, pentoxycarbonyl and hexoxycarbonyl.

The term "alkoxycarbonyl-alkyl" denotes an alkyl group, as defined above, in which one hydrogen atom is replaced by an alkoxycarbonyl group, as defined above. C₁-C₆-Alkoxycarbonyl-C₁-C₆-alkyl is a C₁-C₆-alkyl group, as defined above, in which one hydrogen atom is replaced by a C₁-C₆-alkoxycarbonyl group, as defined above.

### Aminocarbonyl is a group H₂NC(O)-

The term "C₁-C₄-alkylaminocarbonyl" denotes a group C₁-C₄-alkyl-N(H)-C(O)-. Examples are methylaminocarbonyl, ethylaminocarbonyl, propylaminocarbonyl, isopropylaminocarbonyl, n-butylaminocarbonyl, sec-butylaminocarbonylisobutylaminocarbonyl and tert-butylaminocarbonyl.

The term "di-(C₁-C₄-alkyl)-aminocarbonyl" denotes a group (C₁-C₄-alkyl)₂N-C(O)-. Examples are dimethylaminocarbonyl, diethylaminocarbonyl, ethylmethylaminocarbonyl, dipropylaminocarbonyl, diisopropylaminocarbonyl, methylpropylaminocarbonyl, methylisopropylaminocarbonyl, ethylpropylaminocarbonyl, ethylisopropylaminocarbonyl, n-butyl-methylaminocarbonyl, n-butyl-ethylaminocarbonyl, n-butyl-propylaminocarbonyl, di-n-butylaminocarbonyl, 2-butyl-methylaminocarbonyl, 2-butyl-ethylaminocarbonyl, 2-butyl-propylaminocarbonyl, isobutyl-methylaminocarbonyl, ethyl-isobutylaminocarbonyl, isobutyl-propylaminocarbonyl, tert-butyl-methylaminocarbonyl, tert-butyl-ethylaminocarbonyl, or tert-butyl-propylaminocarbonyl.

Benzyloxycarbonyl is also known as the group Cbz or Z; Fluorenyloxycarbonyl is also known as Fmoc and allyloxycarbonyl is also known as Alloc.

Phenyl-(C₁-C₃-alkyl) is a C₁-C₃-alkyl group, as defined above, in which one hydrogen atom is replaced by a phenyl ring (i.e. the attachment to the remainder of the molecule is via the alkyl group). Examples are benzyl, 1-phenylethyl, 2-phenylethyl, 1-phenylpropyl, 2-phenylpropyl, 3-phenylpropyl or 2-phenyl-2-propyl.

Furanyl-(C₁-C₃-alkyl) is a C₁-C₃-alkyl group, as defined above, in which one hydrogen atom is replaced by a 2-or 3-furanyl ring (i.e. the attachment to the remainder of the molecule is via the alkyl group). Examples are furan-2-yl-methyl, furan-3-yl-methyl, 1-(furan-2-yl)-ethyl, 1-(furan-3-yl)-ethyl, 2-(furan-2-yl)-ethyl, or 2-(furan-3-yl)-ethyl.

Phenylthio is a phenyl ring attached via an S atom to the remainder of the molecule.

Phenylsulfinyl is a phenyl ring attached via a S(O) group to the remainder of the molecule.

Phenylsulfonyl is a phenyl ring attached via a S(O)₂ group to the remainder of the molecule.

Bicyclic rings in terms of the present invention contain two rings which have at least one ring atom in common. The term comprises condensed (fused) ring systems, in which the two rings have two neighboring ring atoms in common, as well as spiro systems, in which the rings have only one ring atom in common, and bridged systems with at least three ring atoms in common. If not specified otherwise, the bicyclic rings can be carbocyclic, containing only carbon atoms as ring members, as well as heterocyclic, containing at least one heteroatom or heteroatom group generally selected from N, O S, S(O), and S(O)₂ as ring member(s). Further details are given below.

Polycyclic rings contain three or more rings, each of which having at least one ring atom in common with at least one of the other rings of the polycyclic system. The rings can be condensed, spiro-bound or bridged; mixed systems (e.g. one ring is spiro-bound to a condensed system, or a bridged system is condensed with another ring) are also possible. If not specified otherwise, the polycyclic rings can be carbocyclic, containing only carbon atoms as ring members, as well as heterocyclic, containing at least one heteroatom or heteroatom group generally selected from N, O S, S(O), and S(O)₂ as ring member(s). Further details are given below.

Carbocyclic ring contain only carbon atoms as ring members; heterocyclic rings contain also at least one N, O and/or S atom as ring member(s).

An unsaturated carbocycle contains at least one C-C double bond(s). An unsaturated heterocycle contains at least one C-C and/or C-N and/or N-N double bond(s). Partially unsaturated carbocyclic rings contain less than the maximum number of C-C double bond(s) allowed by the ring size. Partially unsaturated heterocyclic rings contain less than the maximum number of C-C and/or C-N and/or N-N double bond(s) allowed by the ring size. A fully (or maximally) unsaturated carbocyclic ring contains as many conjugated C-C double bonds as allowed by the size(s) of the ring(s). Not encompassed in the definition of Z is however phenyl. A fully (or maximally) unsaturated heterocycle contains as many conjugated C-C and/or C-N and/or N-N double bonds as allowed by the size(s) of the ring(s). Maximally unsaturated 5- or 6-membered heteromonocyclic rings are generally aromatic. Exceptions are maximally unsaturated 6-membered rings containing O, S, SO and/or SO₂ as ring members, such as pyran and thiopyran, which are not aromatic.

Examples for 3-, 4-, 5-, 6-, 7- or 8-membered saturated monocyclic carbocyclic rings are cyclopropyl, cyclobutyl, cyclopentyl, cyclohexyl, cycloheptyl and cyclooctyl.

Examples for 3-, 4-, 5-, 6-, 7- or 8-membered partly unsaturated or fully unsaturated monocyclic carbocyclic rings are cycloprop-1-enyl, cycloprop-2-enyl, cyclobut-1-enyl, cyclobut-2-enyl, cyclobutadienyl, cyclopent-1-enyl, cyclopent-2-enyl, cyclopent-3-enyl, cyclopenta-1,3-dienyl, cyclopenta-1,4-dienyl, cyclopenta-2,4-dienyl, cyclohex-1-enyl, cyclohex-2-enyl, cyclohex-3-enyl, cyclohexa-1,3-dienyl, cyclohexa-1,4-dienyl, cyclohexa-1,5-dienyl, cyclohexa-2,4-dienyl, cyclohexa-2,5-dienyl, cyclohept-1-enyl, cyclohept-2-enyl, cyclohept-3-enyl, cyclohept-4-enyl, cyclohepta-1,3-dienyl, cyclohepta-1,4-dienyl, cyclohepta-1,5-dienyl, cyclohepta-1,6-dienyl, cyclohepta-2,4-dienyl, cyclohepta-2,5-dienyl, cyclohepta-2,6-dienyl, cyclohepta-3,5-dienyl, cyclohepta-1,3,5-trienyl, cyclooct-1-enyl, cyclooct-2-enyl, cyclooct-3-enyl, cyclooct-4-enyl, cyclooct-5-enyl, cyclooct-6-enyl, cyclooct-7-enyl, cycloocta-1,3-dienyl, cycloocta-1,4-dienyl, cycloocta-1,5-dienyl, cycloocta-1,6-dienyl, cycloocta-1,7-dienyl, cycloocta-2,4-dienyl, cycloocta-2,5-dienyl, cycloocta-2,6-dienyl, cycloocta-2,7-dienyl, cycloocta-3,5-dienyl, cycloocta-3,6-dienyl, cycloocta-1,3,5-trienyl, cycloocta-1,3,7-trienyl, cycloocta-2,4,6-trienyl, cyclooctatetraenyl.

Examples for 3-, 4-, 5-, 6-, 7- or 8-membered saturated, partly unsaturated, fully unsaturated or aromatic heterocyclic rings are:
3-, 4-, 5-, 6-, 7- or 8-membered monocyclic saturated heterocycles: e.g. oxiran-2-yl, thiiran-2-yl, aziridin-1-yl, aziridin-2-yl, oxetan-2-yl, oxetan-3-yl, thietan-2-yl, thietan-3-yl, 1-oxothietan-2-yl, 1-oxothietan-3-yl, 1,1-dioxothietan-2-yl, 1,1-dioxothietan-3-yl, azetidin-1-yl, azetidin-2-yl, azetidin-3-yl, tetrahydrofuran-2-yl, tetrahydrofuran-3-yl, tetrahydrothien-2-yl, tetrahydrothien-3-yl, 1-oxotetrahydrothien-2-yl, 1,1-dioxotetrahydrothien-2-yl, 1-oxotetrahydrothien-3-yl, 1,1-dioxotetrahydrothien-3-yl, 1,3-dioxolan-2-yl, 1,3-dioxolan-4-yl, 1,3-dithiolan-2-yl, 1,3-dithiolan-4-yl, 1,3-oxathiolan-2-yl, 1,3-oxathiolan-4-yl, 1,3-oxathiolan-5-yl, pyrrolidin-1-yl, pyrrolidin-2-yl, pyrrolidin-3-yl, pyrazolidin-1-yl, pyrazolidin-3-yl, pyrazolidin-4-yl, pyrazolidin-5-yl, imidazolidin-1-yl, imidazolidin-2-yl, imidazolidin-4-yl, oxazolidin-2-yl, oxazolidin-3-yl, oxazolidin-4-yl, oxazolidin-5-yl, isoxazolidin-2-yl, isoxazolidin-3-yl, isoxazolidin-4-yl, isoxazolidin-5-yl, thiazolidin-2-yl, thiazolidin-3-yl, thiazolidin-4-yl, thiazolidin-5-yl, isothiazolidin-2-yl, isothiazolidin-3-yl, isothiazolidin-4-yl, isothiazolidin-5-yl, 1,2,4-oxadiazolidin-3-yl, 1,2,4-oxadiazolidin-5-yl, 1,2,4-thiadiazolidin-3-yl, 1,2,4-thiadiazolidin-5-yl, 1,3,4-oxadiazolidin-2-yl, 1,3,4-thiadiazolidin-2-yl, 1,2,4-triazolidin-1-yl, 1,2,4-triazolidin-3-yl, 1,2,4-triazolidin-4-yl, 2-tetrahydropyranyl, 3-tetrahydropyranyl, 4-tetrahydropyranyl, 1,3-dioxan-2-yl, 1,3-dioxan-5-yl, 1,4-dioxan-2-yl, piperidin-1-yl, piperidin-2-yl, piperidin-3-yl, piperidin-4-yl, hexahydropyridazin-1-yl, hexahydropyridazin-3-yl, hexahydropyridazin-4-yl, hexahydropyrimidin-1-yl, hexahydropyrimidin-2-yl, hexahydropyrimidin-4-yl, hexahydropyrimidin-5-yl, piperazin-1-yl, piperazin-2-yl, 1,3,5-hexahydrotriazin-1-yl, 1,3,5-hexahydrotriazin-2-yl, 1,2,4-hexahydrotriazin-1-yl, 1,2,4-hexahydrotriazin-2-yl, 1,2,4-hexahydrotriazin-3-yl, 1,2,4-hexahydrotriazin-4-yl, 1,2,4-hexahydrotriazin-5-yl, 1,2,4-hexahydrotriazin-6-yl, morpholin-2-yl, morpholin-3-yl, morpholin-4-yl, thiomorpholin-2-yl, thiomorpholin-3-yl, thiomorpholin-4-yl, 1-oxothiomorpholin-2-yl, 1-oxothiomorpholin-3-yl, 1-oxothiomorpholin-4-yl, 1,1-dioxothiomorpholin-2-yl, 1,1-dioxothiomorpholin-3-yl, 1,1-dioxothiomorpholin-4-yl, azepan-1-, -2-, -3- or -4-yl, oxepan-2-, -3-, -4- or -5-yl, hexahydro-1,3-diazepinyl, hexahydro-1,4-diazepinyl, hexahydro-1,3-oxazepinyl, hexahydro-1,4-oxazepinyl, hexahydro-1,3-dioxepinyl, hexahydro-1,4-dioxepinyl, oxocane, thiocane, azocanyl, [1,3]diazocanyl, [1,4]diazocanyl, [1,5]diazocanyl, or [1,5]oxazocanyl;
3-, 4-, 5-, 6-, 7- or 8-membered partially unsaturated heteromonocyclic rings: 2,3-dihydrofuran-2-yl, 2,3-dihydrofuran-3-yl, 2,4-dihydrofuran-2-yl, 2,4-dihydrofuran-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihy-dropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-, 3-, 4-, 5- or 6-di- or tetrahydropyridinyl, 3-di- or tetrahydropyridazinyl, 4-di- or tetrahydropyridazinyl, 2-di- or tetrahydropyrimidinyl, 4-di- or tetrahydropyrimidinyl, 5-di- or tetrahydropyrimidinyl, di- or tetrahydropyrazinyl, 1,3,5-di- or tetrahydrotriazin-2-yl, 1,2,4-di- or tetrahydrotriazin-3-yl, 2,3,4,5-tetrahydro[1H]azepin-1-, -2-, -3-, -4-, -5-, -6- or -7-yl, 3,4,5,6-tetrahydro[2H]azepin-2-, - 3-, -4-, -5-, -6- or -7-yl, 2,3,4,7-tetrahydro[1H]azepin-1-, -2-, -3-, -4-, -5-, -6- or -7-yl, 2,3,6,7-tetrahydro[1H]azepin-1-, -2-, -3-, -4-, -5-, -6- or -7-yl, tetrahydrooxepinyl, such as 2,3,4,5-tetrahydro[1H]oxepin-2-, -3-, -4-, -5-, -6- or -7-yl, 2,3,4,7-tetrahydro[1H]oxepin-2-, -3-, -4-, -5-, -6- or -7-yl, 2,3,6,7-tetrahydro[1H]oxepin-2-, -3-, -4-, -5-, -6- or -7-yl, tetrahydro-1,3-diazepinyl, tetrahydro-1,4-diazepinyl, tetrahydro-1,3-oxazepinyl, tetrahydro-1,4-oxazepinyl, tetrahydro-1,3-dioxepinyl, tetrahydro-1,4-dioxepinyl, 1,2,3,4,5,6-hexahydroazocine, 2,3,4,5,6,7-hexahydroazocine, 1,2,3,4,5,8-hexahydroazocine, 1,2,3,4,7,8-hexahydroazocine, 1,2,3,4,5,6-hexahydro-[1,5]diazocine, or 1,2,3,4,7,8-hexahydro-[1,5]diazocine;
3-, 4-, 5-, 6-, 7- or 8-membered maximally unsaturated (but not aromatic) heteromonocyclic rings: pyran-2-yl, pyran-3-yl, pyran-4-yl, thiopryran-2-yl, thiopryran-3-yl, thiopryran-4-yl, 1-oxothiopryran-2-yl, 1-oxothiopryran-3-yl, 1-oxothiopryran-4-yl, 1,1-dioxothiopryran-2-yl, 1,1-dioxothiopryran-3-yl, 1,1-dioxothiopryran4-yl, 2H-oxazin-2-yl, 2H-oxazin-3-yl, 2H-oxazin-4-yl, 2H-oxazin-5-yl, 2H-oxazin-6-yl, 4H-oxazin-3-yl, 4H-oxazin-4-yl, 4H-oxazin-5-yl, 4H-oxazin-6-yl, 6H-oxazin-3-yl, 6H-oxazin-4-yl, 7H-oxazin-5-yl, 8H-oxazin-6-yl, 2H-1,3-oxazin-2-yl, 2H-1,3-oxazin-4-yl, 2H-1,3-oxazin-5-yl, 2H-1,3-oxazin-6-yl, 4H-1,3-oxazin-2-yl, 4H-1,3-oxazin-4-yl, 4H-1,3-oxazin-5-yl, 4H-1,3-oxazin-6-yl, 6H-1,3-oxazin-2-yl, 6H-1,3-oxazin-4-yl, 6H-1,3-oxazin-5-yl, 6H-1,3-oxazin-6-yl, 2H-1,4-oxazin-2-yl, 2H-1,4-oxazin-3-yl, 2H-1,4-oxazin-5-yl, 2H-1,4-oxazin-6-yl, 4H-1,4-oxazin-2-yl, 4H-1,4-oxazin-3-yl, 4H-1,4-oxazin-4-yl, 4H-1,4-oxazin-5-yl, 4H-1,4-oxazin-6-yl, 6H-1,4-oxazin-2-yl, 6H-1,4-oxazin-3-yl, 6H-1,4-oxazin-5-yl, 6H-1,4-oxazin-6-yl, 1,4-dioxine-2-yl, 1,4-oxathiin-2-yl, 1H-azepine, 1H-[1,3]-diazepine, 1H-[1,4]-diazepine, [1,3]diazocine, [1,5]diazocine, or [1,5]diazocine
5- or 6-membered monocyclic aromatic heterocyclic rings: e.g. 2-furyl, 3-furyl, 2 thienyl, 3-thienyl, 1-pyrrolyl, 2-pyrrolyl, 3-pyrrolyl, 1¬-pyrazolyl, 3¬-pyrazolyl, 4 pyrazolyl, 5-pyrazolyl, 1 imidazolyl, 2-imidazolyl, 4-imidazolyl, 5-imidazolyl, 2-oxazolyl, 4-oxazolyl, 5 oxazolyl, 3-isoxazolyl, 4-isoxazolyl, 5-isoxazolyl, 2-thiazolyl, 4-thiazolyl, 5 thiazolyl, 3-isothiazolyl, 4-isothiazolyl, 5-isothiazolyl, 1,3,4-triazol-1-yl, 1,3,4-triazol-2-yl, 1,3,4-triazol-3-yl, 1,2,3-triazol-1-yl, 1,2,3-triazol-2-yl, 1,2,3-triazol-4-yl, 1,2,5-oxadiazol-3-yl, 1,2,3-oxadiazol-4-yl, 1,2,3-oxadiazol-5-yl, 1,3,4-oxadiazol-2-yl, 1,2,5-thiadiazol-3-yl, 1,2,3-thiadiazol-4-yl, 1,2,3-thiadiazol-5-yl, 1,3,4-thiadiazol-2-yl, 1,2,3,4-tetrazol-1-yl, 1,2,3,4-tetrazol-2-yl, 1,2,3,4-[1H]-tetrazol-5-yl, 1,2,3,4-[2H]-tetrazol-5-yl, 2-pyridinyl, 3-pyridinyl, 4 pyridinyl, 1-oxopyridin-2-yl, 1-oxopyridin-3-yl, 1-oxopyridin-4-yl, 3-pyridazinyl, 4-pyridazinyl, 2-pyrimidinyl, 4 pyrimidinyl, 5-pyrimidinyl, 2-pyrazinyl, 1,3,5-triazin-2-yl, 1,2,4-triazin-3-yl, 1,2,4-triazin-5-yl, 1,2,3,4-tetrazin-1-yl, 1,2,3,4-tetrazin-2-yl, or 1,2,3,4-tetrazin-5-yl.

Bicyclic rings are 4- to 8-membered, preferably 5- to 8-membered.

Examples for 5- to 8-membered bicyclic spirocyclic saturated carbocyclic rings comprise spiro[2.2]pentyl, spiro[2.3]hexyl, spiro[2.4]heptyl, spiro[3.3]heptyl, spiro[4.4]nonyl, or spiro[5.5]undecyl.

Examples of 5- to 8-membered bicyclic condensed saturated carbocyclic rings comprise bicyclo[3.1.0]hexyl, bicyclo[3.2.0]heptyl, bicyclo[3.3.0]octyl, 1,2,3,3a,4,5,6,6a-octahydropentalenyl, or bicyclo[4.2.0]octyl.

Examples of 5- to 8-membered bicyclic bridged saturated carbocyclic rings comprise bicyclo[1.1.1]pentyl, bicyclo[2.1.1]hexyl, bicyclo[2.2.1]heptyl, bicyclo-[3.1.1]heptyl, bicyclo[2.2.2]octyl, or bicyclo[3.2.1]octyl.

An example for a 5- to 8-membered polycyclic saturated carbocyclic is cubyl.

An example for a 5- to 8-membered partly unsaturated bicyclic bridged carbocyclic ring is bicyclo[2.2.2]oct-2-enyl.

Examples for saturated 5- to 8-membered bicyclic condensed heterocyclic rings are:

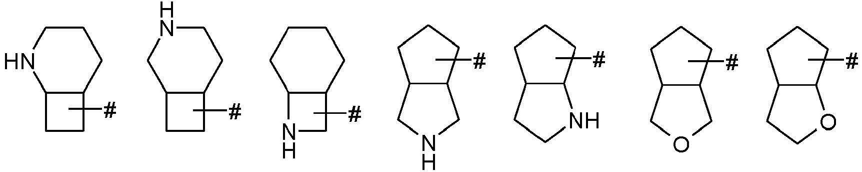

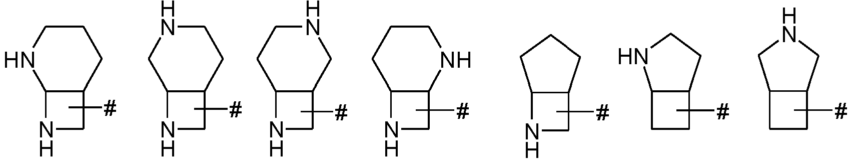

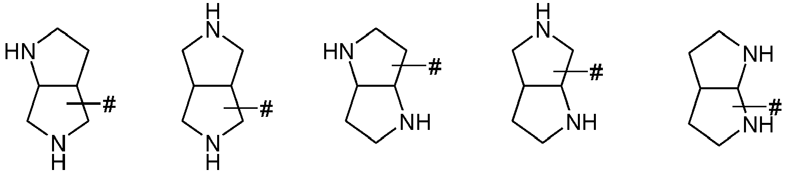

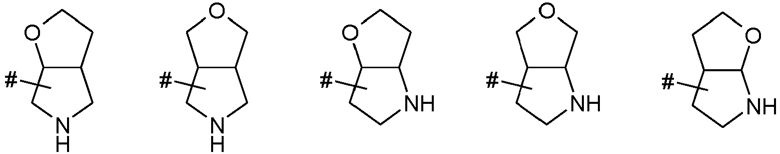

Examples for saturated 5- to 8-membered bicyclic spirocyclic heterocyclic rings are:

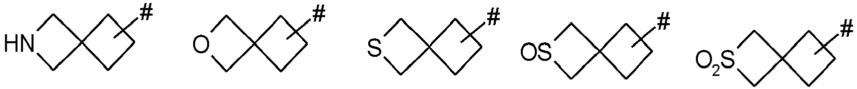

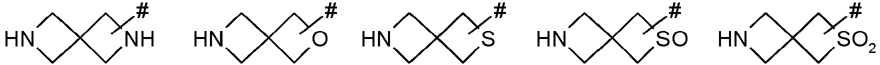

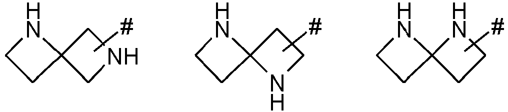

Examples for saturated 5- to 8-membered bicyclic bridged heterocyclic rings are:

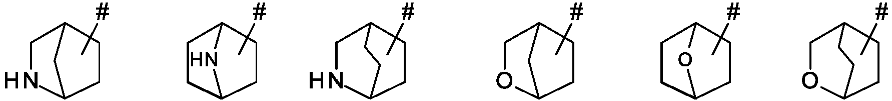

Examples for partly unsaturated 5- to 8-membered bicyclic bridged heterocyclic rings are: 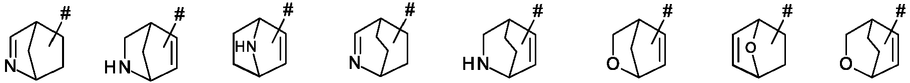 In the above structures # denotes the attachment point to the remainder of the molecule. The attachment point is not restricted to the ring on which this is shown, but can be on either of the two rings, and may be on a carbon or on a nitrogen ring atom. If the rings carry one or more substituents, these may be bound to carbon and/or to nitrogen ring atoms.

Aromatic condensed ring systems are throughout aromatic. In partially aromatic condensed ring systems, an aromatic ring is condensed to a nonaromatic ring.

Examples for bicyclic or tricyclic carbocyclic aromatic ring systems are naphthyl, anthracenyl and phenanthrenyl.

Examples for bicyclic or tricyclic carbocyclic partially aromatic ring systems are indanyl, indenyl, tetralinyl, 1,2-dihydrotetralinyl, 1,4-dihydrotetralinyl, 9H-fluorenyl and 9,10-dihydroanthracenyl.

Examples for bicyclic or tricyclic heterocyclic aromatic ring systems are indolyl, 2H-isoindolyl, benzofuranyl, benzothiophenyl, benzimidazolyl, 1H-indazolyl, 1,3-benzoxazolyl, 1,2-benzoxazolyl, 1,3-benzothiazolyl, 1,2-benzothiazolyl, quinolinyl, isoquinolinyl, quinoxalinyl, cinnolinyl, quinazolinyl, 1,8-naphthyridinyl, 1,5-naphthyridinyl, 1H-pyrrolo[3,2-b]pyridinyl, 1H-pyrrolo[3,2-c]pyridinyl, dibenzofurayl, dibenzothiophenyl, 9H-carbazolyl, acridinyl, or phenazinyl.

Examples for bicyclic or tricyclic heterocyclic partially aromatic ring systems are 2,3-dihydrobenzofuranyl, 1,3-dihydroisobenzofuranyl, 2,3-dihydrobenzothiophenyl, 1-oxo-2,3-dihydrobenzothiophenyl, 1,1-dioxo-2,3-dihydrobenzothiophenyl, 1,3-dihydro-2-benzothiophenyl, 2-oxo-1,3-dihydro-2-benzothiophenyl, 2,2-dioxo-1,3-dihydro-2-benzothiophenyl, indolinyl, isoindolinyl, 1,3-benzodioxolyl, 1,3-benzodithiolyl, 1-oxo-1,3-benzodithiolyl, 1,1-dioxo-1,3-benzodithiolyl, 1,3-dioxo-1,3-benzodithiolyl, 1,1,3,3-tetraoxo-1,3-benzoxathiolyl, 2,3-dihydro-1H-benzimidazolyl, 2,3-dihydro-1H-indazolyl, 2,3-dihydro-1,3-benzoxazolyl, 2,3-dihydro-1,2-benzoxazolyl, 2,3-dihydro-1,3-benzothiazolyl, 2,3-dihydro-1,2-benzothiazolyl, chromanyl, iso-chromanyl, 4H-1,3-benzodioxinyl, 2,3-dihydro-1,4-benzodioxinyl, 4H-chromenyl, 2H-chromenyl, 1,2,3,4-tetrahydroquinolinyl, or 1,2,3,4-tetrahydroisoquinolinyl.

The remarks made below as to preferred embodiments of the variables (substituents) of the compounds of formula I are valid on their own as well as preferably in combination with each other, as well as in combination with the stereoisomers, tautomers or salts thereof. The remarks made below concerning preferred embodiments of the variables further are valid on their own as well as preferably in combination with each other concerning the compounds of formulae I, where applicable, as well as concerning the uses and methods according to the invention and the composition according to the invention.

R¹ is hydrogen.

R⁴ is hydrogen.

R² is (C₁-C₆)-alkyll, preferably (C₁-C₄)-alkyl. In particular, R² is methyl or ethyl; specifically methyl.

R³ is hydrogen or halogen; especially hydrogen or fluorine, and is in particular hydrogen.

In the divalent radical (X¹), the orientation within the molecule is as depicted, the left arrow representing the bond to the adjacent nitrogen atom and the right arrow representing the bond to Y.

When X is a bond ("X⁰"), the compound (I) can also be depicted as follows:

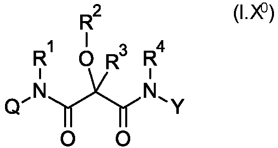

When X is a divalent radical of the formula (X¹), the compound (I) can also be depicted as follows:

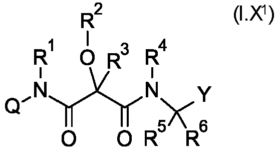

In the divalent radical (X¹), R⁵-R⁶, independently of each other, are are hydrogen or (C₁-C₆)-alkyl, and specifically hydrogen or methyl.

Non-exhaustive examples for suitable divalent radicals (X¹) are CH₂, CH(CH₃), CH(CH₂CH₃), C(CH₃)₂, and C(iPr)CH₃ . iPr is isopropyl.

X is a bond or the divalent unit (X¹). In the latter, R⁵ and R⁶, independently of each other, are hydrogen or (C₁-C₆)-alkyl, and are preferably hydrogen or methyl. Even more preferably, one of R⁵ and R⁶ is hydrogen and the other is hydrogen or methyl. In particular, one of R⁵ and R⁶ is hydrogen and the other is methyl, X¹ thus being in particular CH(CH₃).

Z is a five- or six-membered saturated or partly unsaturated carbocyclic ring which is substituted by m radicals CO₂R^{e}. where m is 1 or 2, in particular 1.

Examples for five- or six-membered saturated or partly unsaturated carbocyclic rings are cyclopentyl, cyclopent-1-en-1-yl, cyclopent-2-en-1-yl, cyclopent-3-en-1-yl, cyclopenta-1,3-dien-1-yl, cyclopenta-1,4-dien-1-yl, cyclopenta-2,4-dien-1-yl, cyclohexyl, cyclohex-1-en-1-yl, cyclohex-2-en-1-yl, cyclohex-3-en-1-yl, cyclohexa-1,3-dien-1-yl, cyclohexa-1,4-dien-1-yl, cyclohexa-1,5-dien-1-yl, cyclohexa-2,4-dien-1-yl and cyclohexa-2,5-dien-1-yl. Among these, preference is given to cyclopentyl, cyclopent-1-en-1-yl, cyclopent-2-en-1-yl, cyclopent-3-en-1-yl and cyclohexyl. A specific example is cyclopent-2-en-1-yl.

Non-exhaustive examples for such rings are the following structures:

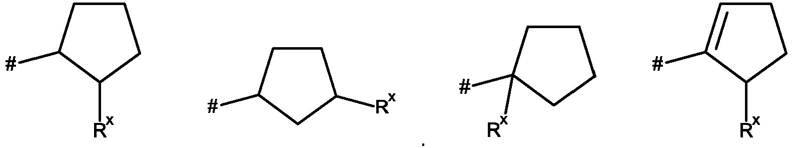

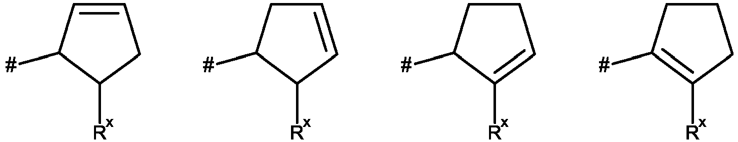

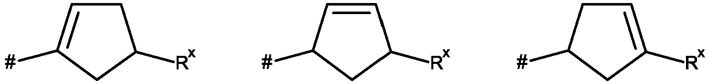

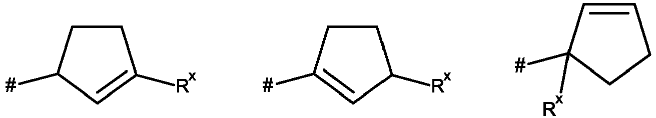

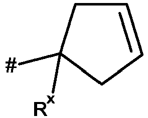

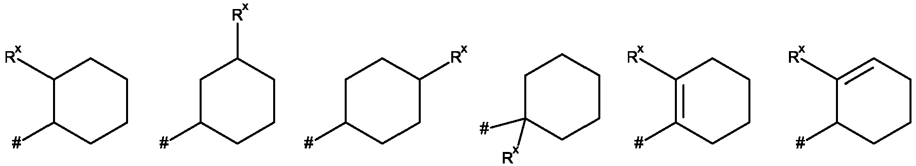

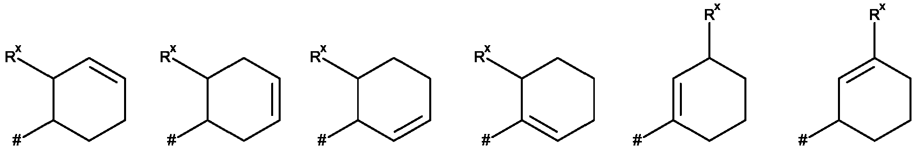

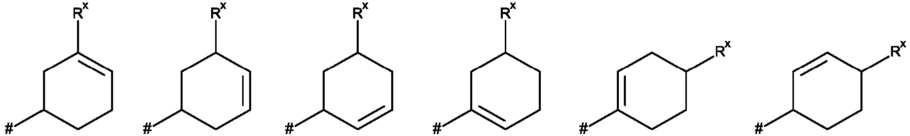

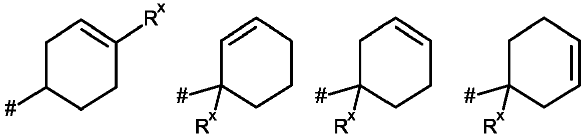

In the above structures, # stands for the attachment point to the remainder of the molecule and R^{x} stands for CO₂R^{e}.

R^{e} is hydrogen or (C₁-C₆)-alkyl, preferably (C₁-C₄)-alkyl. m is in this context 1 or 2, preferably 1. Examples for such rings are the above structures wherein R^{x} stands for CO₂R^{e}, where R^{e} is hydrogen or (C₁-C₆)-alkyl, preferably (C₁-C₄)-alkyl.

In particular, Z is a five- or six-membered partly unsaturated carbocyclic ring which is substituted by m radicals CO₂R^{e}, where R^{e} is hydrogen or (C₁-C₆)-alkyl, preferably (C₁-C₄)-alkyl. m is in this context 1 or 2, preferably 1.

More particularly, Z is a five-membered partly unsaturated carbocyclic ring which is substituted by one radical CO₂R^{e}, where R^{e} is hydrogen or (C₁-C₆)-alkyl, preferably (C₁-C₄)-alkyl. Examples for such rings are 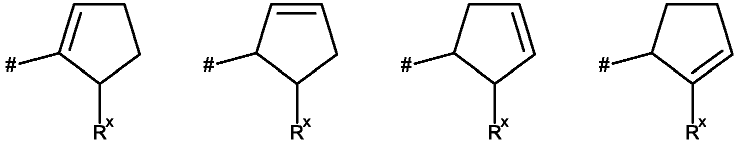 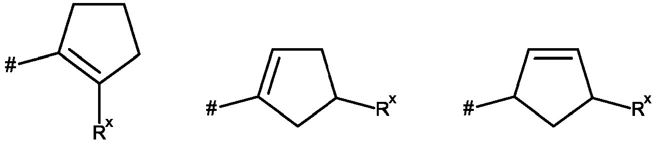 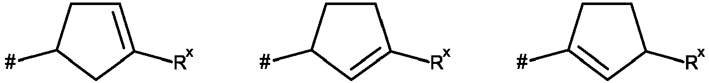 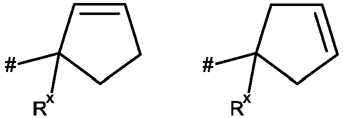 wherein R^{x} stands for CO₂R^{e}, where R^{e} is hydrogen or (C₁-C₆)-alkyl.

Specifically, Z is 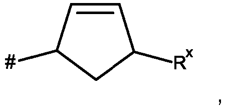 where R^{x} is CO₂R^{e}, where R^{e} is hydrogen or (C₁-C₆)-alkyl and is in particular (C₁-C₄)-alkyl.

In another preferred embodiment, Y is (C₁-C₄)-alkyl which is substituted by m radicals CO₂R^{e}, where R^{e} is hydrogen or (C₁-C₆)-alkyl and m is 1 or 2. Preferably, Y is (C₁-C₄)-alkyl which is substituted by one radical CO₂R^{e}, where R^{e} is hydrogen or (C₁-C₆)-alkyl, where R^{e} is preferably (C₁-C₄)-alkyl.

In a preferred embodiment, X is a bond and Y is Z, where Z has one of the above general or preferred meanings.

In another preferred embodiment,
- X: is a divalent unit (X¹), where R⁵ and R⁶ are as defined above; and
- Y: is (C₁-C₄)-alkyl which is substituted by m radicals selected from the group consisting of CO₂R^{e}, where R^{e} is hydrogen or (C₁-C₆)-alkyl and m is 1 or 2.

More preferably,
- X: is a divalent unit (X¹), where R⁵ and R⁶ are independently hydrogen or methyl; and
- Y: is (C₁-C₄)-alkyl which is substituted by m radicals CO₂R^{e}, where R^{e} is hydrogen or (C₁-C₆)-alkyl, where R^{e} is preferably (C₁-C₄)-alkyl, and m is 1 or 2.
m in this context is preferably 1.

Even more preferably,
- X: is a divalent unit (X¹), where one of R⁵ and R⁶ is hydrogen and the other is hydrogen or methyl, preferably methyl; and
- Y: is (C₁-C₄)-alkyl which is substituted by m (preferably 1) radicals CO₂R^{e}, where R^{e} is hydrogen or (C₁-C₆)-alkyl, where R^{e} is preferably (C₁-C₄)-alkyl.

Q is selected from rings of formulae Q1 to Q13 as shown above.

Q1 to Q13 carry k substituents R^{Q1}; where R^{Q1} is selected from the group consisting of halogen, cyano, (C₁-C₃)-alkyl, (C₁-C₃)-haloalkyl, (C₁-C₃)-alkoxy and (C₁-C₃)-haloalkoxy and preferably from halogen and (C₁-C₃)-alkyl; and where k is 0, 1, 2 or 3, preferably 0, 1 or 2.

Preferably, Q is a bicyclic condensed ring system of formula Q1, Q2, Q5 or Q12.
In Q1, A and B are preferably O;
in Q2, A is preferably S, S(O) or S(O)₂; more preferably S(O)₂;
in Q5 A is preferably CH or N and B is preferably NR^{q}, O or S;
in Q12 A and B are preferably CH.

Preferably, rings Q1, Q2, Q5 and Q12 carry k substituents R^{Q1}; where R^{Q1} is selected from the group consisting of halogen and (C₁-C₃)-alkyl; more preferably halogen; where k is 0, 1 or 2. R^{q} is preferably hydrogen or (C₁-C₃)-alkyl; more preferably (C₁-C₃)-alkyl.

In a preferred embodiment
- Q: is a bicyclic condensed ring system of formula Q1 to Q13; where rings Q1 to Q13 carry k substituents R^{Q1}; where R^{Q1} is selected from the group consisting of halogen, cyano, (C₁-C₃)-alkyl, (C₁-C₃)-haloalkyl, (C₁-C₃)-alkoxy and (C₁-C₃)-haloalkoxy; where R^{q} is hydrogen or (C₁-C₃)-alkyl; and where k is 0, 1, 2 or 3;
- R¹: hydrogen;
- R²: is (C₁-C₆)-alkyl;
- R³: is hydrogen or halogen;
- R⁴: is hydrogen;
- X: is a bond; and Y is Z; where Z is a five- or six-membered saturated or partly unsaturated carbocyclic ring which is substituted by m radicals CO₂R^{e}, where R^{e} is hydrogen or (C₁-C₆)-alkyl and m is 1 or 2; or
- X: is a divalent unit (X¹), where R⁵ and R⁶ are independently of each other hydrogen or (C₁-C₆)-alkyl; and Y is (C₁-C₄)-alkyl which is substituted by m radicals CO₂R^{e}, where R^{e} is (C₁-C₆)-alkyl and m is 1 or 2.

More preferably,
- Q: is a bicyclic condensed ring system of formula Q1, Q2, Q5 or Q12; where in Q1 A and B are O; in Q2 A is S, S(O) or S(O)₂, preferably S(O)₂; in Q5 A is CH or N and B is NR^{q}, O or S; and in Q12 A and B are CH; where rings Q1, Q2, Q5 and Q12 carry k substituents R^{Q1}; where R^{Q1} is selected from the group consisting of halogen and (C₁-C₃)-alkyl; where R^{q} is hydrogen or (C₁-C₃)-alkyl; and where k is 0, 1 or 2;
- R¹: hydrogen;
- R²: is methyl or ethyl;
- R³: is hydrogen;
- R⁴: is hydrogen;
- X: is a bond; and Y is Z; where Z is a five-membered partly unsaturated carbocyclic ring which is substituted by one radical CO₂R^{e}, where R^{e} is (C₁-C₆)-alkyl; or
- X: is a divalent unit (X¹), where R⁵ and R⁶ are independently of each other hydrogen or methyl; and Y is (C₁-C₄)-alkyl which is substituted by one radical CO₂R^{e}, where R^{e} is (C₁-C₆)-alkyl.

Specifically,
- Q: is a bicyclic condensed ring system of formula Q1, Q2, Q5 or Q12; where in Q1 A and B are O; in Q2 A is S, S(O) or S(O)₂; in Q5 A is CH or N and B is NR^{q}, O or S; where rings Q1, Q2, Q5 and Q12 carry k substituents R^{Q1}; where R^{Q1} is selected from the group consisting of halogen and (C₁-C₃)-alkyl; where k is 0, 1 or 2;
- R¹: hydrogen;
- R²: is methyl or ethyl;
- R³: is hydrogen;
- R⁴: is hydrogen;
- X: is a bond; and Y is Z; where Z is a five-membered partly unsaturated carbocyclic ring which is substituted by one radical CO₂R^{e}, where R^{e} is (C₁-C₆)-alkyl.

Particularly preferably,
- Q: is a bicyclic condensed ring system of formula Q1, Q2, Q5 or Q12; where in Q1 A and B are O; in Q2 A is S(O)₂; in Q5 A is CH or N and B is NR^{q}, O or S; where rings Q1, Q2, Q5 and Q12 carry k substituents R^{Q1}; where R^{Q1} is halogen;
where R^{q} is (C₁-C₃)-alkyl; and where k is 0, 1 or 2;
- R¹: hydrogen;
- R²: is methyl;
- R³: is hydrogen;
- R⁴: is hydrogen;
- X: is a bond; and Y is Z; where Z is a five-membered partly unsaturated carbocyclic ring which is substituted by one radical CO₂R^{e}, where R^{e} is (C₁-C₄)-alkyl; or
- X: is a divalent unit (X¹), where one of R⁵ and R⁶ is hydrogen and the other is hydrogen or methyl; preferably methyl; and Y is (C₁-C₄)-alkyl which is substituted by one radical CO₂R^{e}, where R^{e} is (C₁-C₄)-alkyl.

In the above particular and specific embodiments, the five-membered partly unsaturated carbocyclic ring Z is preferably a ring Z10 (depicted below), wherein # denotes the attachment point to the remainder of the molecule.

Compounds (I), wherein R¹ and R⁴ are hydrogen and R², R³ and X-Y in combination have the meanings as defined in each line of table A below are particularly preferred.

**Table A**

| No. | R² | R³ | -X-Y |
|---|---|---|---|
| 1. | CH₃ | H | -CH(CH₃)-CH₂-C(=O)O-CH₃ |
| 2. | CH₃ | F | -CH(CH₃)-CH₂-C(=O)O-CH₃ |
| 3. | Et | H | -CH(CH₃)-CH₂-C(=O)O-CH₃ |
| 4. | Et | F | -CH(CH₃)-CH₂-C(=O)O-CH₃ |
| 5. | CH₃ | H | -CH(CH₃)-CH₂-C(=O)O-CH₂CH₃ |
| 6. | CH₃ | F | -CH(CH₃)-CH₂-C(=O)O-CH₂CH₃ |
| 7. | Et | H | -CH(CH₃)-CH₂-C(=O)O-CH₂CH₃ |
| 8. | Et | F | -CH(CH₃)-CH₂-C(=O)O-CH₂CH₃ |
| 9. | CH₃ | H | -CH(CH₃)-CH₂-C(=O)O-CH₂CH₂CH₃ |
| 10. | CH₃ | F | -CH(CH₃)-CH₂-C(=O)O-CH₂CH₂CH₃ |
| 11. | Et | H | -CH(CH₃)-CH₂-C(=O)O-CH₂CH₂CH₃ |
| 12. | Et | F | -CH(CH₃)-CH₂-C(=O)O-CH₂CH₂CH₃ |
| 13. | CH₃ | H | -CH(CH₃)-CH₂-C(=O)O-CH(CH₃)₂ |
| 14. | CH₃ | F | -CH(CH₃)-CH₂-C(=O)O-CH(CH₃)₂ |
| 15. | Et | H | -CH(CH₃)-CH₂-C(=O)O-CH(CH₃)₂ |
| 16. | Et | F | -CH(CH₃)-CH₂-C(=O)O-CH(CH₃)₂ |
| 17. | CH₃ | H | -CH(CH₃)-CH₂CH₂-C(=O)O-CH₃ |
| 18. | CH₃ | F | -CH(CH₃)-CH₂CH₂-C(=O)O-CH₃ |
| 19. | Et | H | -CH(CH₃)-CH₂CH₂-C(=O)O-CH₃ |
| 20. | Et | F | -CH(CH₃)-CH₂CH₂-C(=O)O-CH₃ |
| 21. | CH₃ | H | -CH(CH₃)-CH₂CH₂-C(=O)O-CH₂CH₃ |
| 22. | CH₃ | F | -CH(CH₃)-CH₂CH₂-C(=O)O-CH₂CH₃ |
| 23. | Et | H | -CH(CH₃)-CH₂CH₂-C(=O)O-CH₂CH₃ |
| 24. | Et | F | -CH(CH₃)-CH₂CH₂-C(=O)O-CH₂CH₃ |
| 25. | CH₃ | H | -CH(CH₃)-CH₂CH₂-C(=O)O-CH₂CH₂CH₃ |
| 26. | CH₃ | F | -CH(CH₃)-CH₂CH₂-C(=O)O-CH₂CH₂CH₃ |
| 27. | Et | H | -CH(CH₃)-CH₂CH₂-C(=O)O-CH₂CH₂CH₃ |
| 28. | Et | F | -CH(CH₃)-CH₂CH₂-C(=O)O-CH₂CH₂CH₃ |
| 29. | CH₃ | H | -CH(CH₃)-CH₂CH₂-C(=O)O-CH(CH₃)₂ |
| 30. | CH₃ | F | -CH(CH₃)-CH₂CH₂-C(=O)O-CH(CH₃)₂ |
| 31. | Et | H | -CH(CH₃)-CH₂CH₂-C(=O)O-CH(CH₃)₂ |
| 32. | Et | F | -CH(CH₃)-CH₂CH₂-C(=O)O-CH(CH₃)₂ |
| 33. | CH₃ | H | Z2, wherein R^{e} is CH₃ |
| 34. | CH₃ | F | Z2, wherein R^{e} is CH₃ |
| 35. | Et | H | Z2, wherein R^{e} is CH₃ |
| 36. | Et | F | Z2, wherein R^{e} is CH₃ |
| 37. | CH₃ | H | Z2, wherein R^{e} is ethyl |
| 38. | CH₃ | F | Z2, wherein R^{e} is ethyl |
| 39. | Et | H | Z2, wherein R^{e} is ethyl |
| 40. | Et | F | Z2, wherein R^{e} is ethyl |
| 41. | CH₃ | H | Z2, wherein R^{e} is n-propyl |
| 42. | CH₃ | F | Z2, wherein R^{e} is n-propyl |
| 43. | Et | H | Z2, wherein R^{e} is n-propyl |
| 44. | Et | F | Z2, wherein R^{e} is n-propyl |
| 45. | CH₃ | H | Z2, wherein R^{e} is isopropyl |
| 46. | CH₃ | F | Z2, wherein R^{e} is isopropyl |
| 47. | Et | H | Z2, wherein R^{e} is isopropyl |
| 48. | Et | F | Z2, wherein R^{e} is isopropyl |
| 49. | CH₃ | H | Z3, wherein R^{e} is CH₃ |
| 50. | CH₃ | F | Z3, wherein R^{e} is CH₃ |
| 51. | Et | H | Z3, wherein R^{e} is CH₃ |
| 52. | Et | F | Z3, wherein R^{e} is CH₃ |
| 53. | CH₃ | H | Z3, wherein R^{e} is ethyl |
| 54. | CH₃ | F | Z3, wherein R^{e} is ethyl |
| 55. | Et | H | Z3, wherein R^{e} is ethyl |
| 56. | Et | F | Z3, wherein R^{e} is ethyl |
| 57. | CH₃ | H | Z3, wherein R^{e} is n-propyl |
| 58. | CH₃ | F | Z3, wherein R^{e} is n-propyl |
| 59. | Et | H | Z3, wherein R^{e} is n-propyl |
| 60. | Et | F | Z3, wherein R^{e} is n-propyl |
| 61. | CH₃ | H | Z3, wherein R^{e} is isopropyl |
| 62. | CH₃ | F | Z3, wherein R^{e} is isopropyl |
| 63. | Et | H | Z3, wherein R^{e} is isopropyl |
| 64. | Et | F | Z3, wherein R^{e} is isopropyl |
| 65. | CH₃ | H | Z4, wherein R^{e} is CH₃ |
| 66. | CH₃ | F | Z4, wherein R^{e} is CH₃ |
| 67. | Et | H | Z4, wherein R^{e} is CH₃ |
| 68. | Et | F | Z4, wherein R^{e} is CH₃ |
| 69. | CH₃ | H | Z4, wherein R^{e} is ethyl |
| 70. | CH₃ | F | Z4, wherein R^{e} is ethyl |
| 71. | Et | H | Z4, wherein R^{e} is ethyl |
| 72. | Et | F | Z4, wherein R^{e} is ethyl |
| 73. | CH₃ | H | Z4, wherein R^{e} is n-propyl |
| 74. | CH₃ | F | Z4, wherein R^{e} is n-propyl |
| 75. | Et | H | Z4, wherein R^{e} is n-propyl |
| 76. | Et | F | Z4, wherein R^{e} is n-propyl |
| 77. | CH₃ | H | Z4, wherein R^{e} is isopropyl |
| 78. | CH₃ | F | Z4, wherein R^{e} is isopropyl |
| 79. | Et | H | Z4, wherein R^{e} is isopropyl |
| 80. | Et | F | Z4, wherein R^{e} is isopropyl |
| 81. | CH₃ | H | Z5, wherein R^{e} is CH₃ |
| 82. | CH₃ | F | Z5, wherein R^{e} is CH₃ |
| 83. | Et | H | Z5, wherein R^{e} is CH₃ |
| 84. | Et | F | Z5, wherein R^{e} is CH₃ |
| 85. | CH₃ | H | Z5, wherein R^{e} is ethyl |
| 86. | CH₃ | F | Z5, wherein R^{e} is ethyl |
| 87. | Et | H | Z5, wherein R^{e} is ethyl |
| 88. | Et | F | Z5, wherein R^{e} is ethyl |
| 89. | CH₃ | H | Z5, wherein R^{e} is n-propyl |
| 90. | CH₃ | F | Z5, wherein R^{e} is n-propyl |
| 91. | Et | H | Z5, wherein R^{e} is n-propyl |
| 92. | Et | F | Z5, wherein R^{e} is n-propyl |
| 93. | CH₃ | H | Z5, wherein R^{e} is isopropyl |
| 94. | CH₃ | F | Z5, wherein R^{e} is isopropyl |
| 95. | Et | H | Z5, wherein R^{e} is isopropyl |
| 96. | Et | F | Z5, wherein R^{e} is isopropyl |
| 97. | CH₃ | H | Z6, wherein R^{e} is CH₃ |
| 98. | CH₃ | F | Z6, wherein R^{e} is CH₃ |
| 99. | Et | H | Z6, wherein R^{e} is CH₃ |
| 100. | Et | F | Z6, wherein R^{e} is CH₃ |
| 101. | CH₃ | H | Z6, wherein R^{e} is ethyl |
| 102. | CH₃ | F | Z6, wherein R^{e} is ethyl |
| 103. | Et | H | Z6, wherein R^{e} is ethyl |
| 104. | Et | F | Z6, wherein R^{e} is ethyl |
| 105. | CH₃ | H | Z6, wherein R^{e} is n-propyl |
| 106. | CH₃ | F | Z6, wherein R^{e} is n-propyl |
| 107. | Et | H | Z6, wherein R^{e} is n-propyl |
| 108. | Et | F | Z6, wherein R^{e} is n-propyl |
| 109. | CH₃ | H | Z6, wherein R^{e} is isopropyl |
| 110. | CH₃ | F | Z6, wherein R^{e} is isopropyl |
| 111. | Et | H | Z6, wherein R^{e} is isopropyl |
| 112. | Et | F | Z6, wherein R^{e} is isopropyl |
| 113. | CH₃ | H | Z7, wherein R^{e} is CH₃ |
| 114. | CH₃ | F | Z7, wherein R^{e} is CH₃ |
| 115. | Et | H | Z7, wherein R^{e} is CH₃ |
| 116. | Et | F | Z7, wherein R^{e} is CH₃ |
| 117. | CH₃ | H | Z7, wherein R^{e} is ethyl |
| 118. | CH₃ | F | Z7, wherein R^{e} is ethyl |
| 119. | Et | H | Z7, wherein R^{e} is ethyl |
| 120. | Et | F | Z7, wherein R^{e} is ethyl |
| 121. | CH₃ | H | Z7, wherein R^{e} is n-propyl |
| 122. | CH₃ | F | Z7, wherein R^{e} is n-propyl |
| 123. | Et | H | Z7, wherein R^{e} is n-propyl |
| 124. | Et | F | Z7, wherein R^{e} is n-propyl |
| 125. | CH₃ | H | Z7, wherein R^{e} is isopropyl |
| 126. | CH₃ | F | Z7, wherein R^{e} is isopropyl |
| 127. | Et | H | Z7, wherein R^{e} is isopropyl |
| 128. | Et | F | Z7, wherein R^{e} is isopropyl |
| 129. | CH₃ | H | Z8, wherein R^{e} is CH₃ |
| 130. | CH₃ | F | Z8, wherein R^{e} is CH₃ |
| 131. | Et | H | Z8, wherein R^{e} is CH₃ |
| 132. | Et | F | Z8, wherein R^{e} is CH₃ |
| 133. | CH₃ | H | Z8, wherein R^{e} is ethyl |
| 134. | CH₃ | F | Z8, wherein R^{e} is ethyl |
| 135. | Et | H | Z8, wherein R^{e} is ethyl |
| 136. | Et | F | Z8, wherein R^{e} is ethyl |
| 137. | CH₃ | H | Z8, wherein R^{e} is n-propyl |
| 138. | CH₃ | F | Z8, wherein R^{e} is n-propyl |
| 139. | Et | H | Z8, wherein R^{e} is n-propyl |
| 140. | Et | F | Z8, wherein R^{e} is n-propyl |
| 141. | CH₃ | H | Z8, wherein R^{e} is isopropyl |
| 142. | CH₃ | F | Z8, wherein R^{e} is isopropyl |
| 143. | Et | H | Z8, wherein R^{e} is isopropyl |
| 144. | Et | F | Z8, wherein R^{e} is isopropyl |
| 145. | CH₃ | H | Z9, wherein R^{e} is CH₃ |
| 146. | CH₃ | F | Z9, wherein R^{e} is CH₃ |
| 147. | Et | H | Z9, wherein R^{e} is CH₃ |
| 148. | Et | F | Z9, wherein R^{e} is CH₃ |
| 149. | CH₃ | H | Z9, wherein R^{e} is ethyl |
| 150. | CH₃ | F | Z9, wherein R^{e} is ethyl |
| 151. | Et | H | Z9, wherein R^{e} is ethyl |
| 152. | Et | F | Z9, wherein R^{e} is ethyl |
| 153. | CH₃ | H | Z9, wherein R^{e} is n-propyl |
| 154. | CH₃ | F | Z9, wherein R^{e} is n-propyl |
| 155. | Et | H | Z9, wherein R^{e} is n-propyl |
| 156. | Et | F | Z9, wherein R^{e} is n-propyl |
| 157. | CH₃ | H | Z9, wherein R^{e} is isopropyl |
| 158. | CH₃ | F | Z9, wherein R^{e} is isopropyl |
| 159. | Et | H | Z9, wherein R^{e} is isopropyl |
| 160. | Et | F | Z9, wherein R^{e} is isopropyl |
| 161. | CH₃ | H | Z10, wherein R^{e} is CH₃ |
| 162. | CH₃ | F | Z10, wherein R^{e} is CH₃ |
| 163. | Et | H | Z10, wherein R^{e} is CH₃ |
| 164. | Et | F | Z10, wherein R^{e} is CH₃ |
| 165. | CH₃ | H | Z10, wherein R^{e} is ethyl |
| 166. | CH₃ | F | Z10, wherein R^{e} is ethyl |
| 167. | Et | H | Z10, wherein R^{e} is ethyl |
| 168. | Et | F | Z10, wherein R^{e} is ethyl |
| 169. | CH₃ | H | Z10, wherein R^{e} is n-propyl |
| 170. | CH₃ | F | Z10, wherein R^{e} is n-propyl |
| 171. | Et | H | Z10, wherein R^{e} is n-propyl |
| 172. | Et | F | Z10, wherein R^{e} is n-propyl |
| 173. | CH₃ | H | Z10, wherein R^{e} is isopropyl |
| 174. | CH₃ | F | Z10, wherein R^{e} is isopropyl |
| 175. | Et | H | Z10, wherein R^{e} is isopropyl |
| 176. | Et | F | Z10, wherein R^{e} is isopropyl |
| 177. | CH₃ | H | Z11, wherein R^{e} is CH₃ |
| 178. | CH₃ | F | Z11, wherein R^{e} is CH₃ |
| 179. | Et | H | Z11, wherein R^{e} is CH₃ |
| 180. | Et | F | Z11, wherein R^{e} is CH₃ |
| 181. | CH₃ | H | Z11, wherein R^{e} is ethyl |
| 182. | CH₃ | F | Z11, wherein R^{e} is ethyl |
| 183. | Et | H | Z11, wherein R^{e} is ethyl |
| 184. | Et | F | Z11, wherein R^{e} is ethyl |
| 185. | CH₃ | H | Z11, wherein R^{e} is n-propyl |
| 186. | CH₃ | F | Z11, wherein R^{e} is n-propyl |
| 187. | Et | H | Z11, wherein R^{e} is n-propyl |
| 188. | Et | F | Z11, wherein R^{e} is n-propyl |
| 189. | CH₃ | H | Z11, wherein R^{e} is isopropyl |
| 190. | CH₃ | F | Z11, wherein R^{e} is isopropyl |
| 191. | Et | H | Z11, wherein R^{e} is isopropyl |
| 192. | Et | F | Z11, wherein R^{e} is isopropyl |
| 193. | CH₃ | H | Z12, wherein R^{e} is CH₃ |
| 194. | CH₃ | F | Z12, wherein R^{e} is CH₃ |
| 195. | Et | H | Z12, wherein R^{e} is CH₃ |
| 196. | Et | F | Z12, wherein R^{e} is CH₃ |
| 197. | CH₃ | H | Z12, wherein R^{e} is ethyl |
| 198. | CH₃ | F | Z12, wherein R^{e} is ethyl |
| 199. | Et | H | Z12, wherein R^{e} is ethyl |
| 200. | Et | F | Z12, wherein R^{e} is ethyl |
| 201. | CH₃ | H | Z12, wherein R^{e} is n-propyl |
| 202. | CH₃ | F | Z12, wherein R^{e} is n-propyl |
| 203. | Et | H | Z12, wherein R^{e} is n-propyl |
| 204. | Et | F | Z12, wherein R^{e} is n-propyl |
| 205. | CH₃ | H | Z12, wherein R^{e} is isopropyl |
| 206. | CH₃ | F | Z12, wherein R^{e} is isopropyl |
| 207. | Et | H | Z12, wherein R^{e} is isopropyl |
| 208. | Et | F | Z12, wherein R^{e} is isopropyl |
| 209. | CH₃ | H | Z13, wherein R^{e} is CH₃ |
| 210. | CH₃ | F | Z13, wherein R^{e} is CH₃ |
| 211. | Et | H | Z13, wherein R^{e} is CH₃ |
| 212. | Et | F | Z13, wherein R^{e} is CH₃ |
| 213. | CH₃ | H | Z13, wherein R^{e} is ethyl |
| 214. | CH₃ | F | Z13, wherein R^{e} is ethyl |
| 215. | Et | H | Z13, wherein R^{e} is ethyl |
| 216. | Et | F | Z13, wherein R^{e} is ethyl |
| 217. | CH₃ | H | Z13, wherein R^{e} is n-propyl |
| 218. | CH₃ | F | Z13, wherein R^{e} is n-propyl |
| 219. | Et | H | Z13, wherein R^{e} is n-propyl |
| 220. | Et | F | Z13, wherein R^{e} is n-propyl |
| 221. | CH₃ | H | Z13, wherein R^{e} is isopropyl |
| 222. | CH₃ | F | Z13, wherein R^{e} is isopropyl |
| 223. | Et | H | Z13, wherein R^{e} is isopropyl |
| 224. | Et | F | Z13, wherein R^{e} is isopropyl |
| 225. | CH₃ | H | Z14, wherein R^{e} is CH₃ |
| 226. | CH₃ | F | Z14, wherein R^{e} is CH₃ |
| 227. | Et | H | Z14, wherein R^{e} is CH₃ |
| 228. | Et | F | Z14, wherein R^{e} is CH₃ |
| 229. | CH₃ | H | Z14, wherein R^{e} is ethyl |
| 230. | CH₃ | F | Z14, wherein R^{e} is ethyl |
| 231. | Et | H | Z14, wherein R^{e} is ethyl |
| 232. | Et | F | Z14, wherein R^{e} is ethyl |
| 233. | CH₃ | H | Z14, wherein R^{e} is n-propyl |
| 234. | CH₃ | F | Z14, wherein R^{e} is n-propyl |
| 235. | Et | H | Z14, wherein R^{e} is n-propyl |
| 236. | Et | F | Z14, wherein R^{e} is n-propyl |
| 237. | CH₃ | H | Z14, wherein R^{e} is isopropyl |
| 238. | CH₃ | F | Z14, wherein R^{e} is isopropyl |
| 239. | Et | H | Z14, wherein R^{e} is isopropyl |
| 240. | Et | F | Z14, wherein R^{e} is isopropyl |
| 241. | CH₃ | H | Z15, wherein R^{e} is CH₃ |
| 242. | CH₃ | F | Z15, wherein R^{e} is CH₃ |
| 243. | Et | H | Z15, wherein R^{e} is CH₃ |
| 244. | Et | F | Z15, wherein R^{e} is CH₃ |
| 245. | CH₃ | H | Z15, wherein R^{e} is ethyl |
| 246. | CH₃ | F | Z15, wherein R^{e} is ethyl |
| 247. | Et | H | Z15, wherein R^{e} is ethyl |
| 248. | Et | F | Z15, wherein R^{e} is ethyl |
| 249. | CH₃ | H | Z15, wherein R^{e} is n-propyl |
| 250. | CH₃ | F | Z15, wherein R^{e} is n-propyl |
| 251. | Et | H | Z15, wherein R^{e} is n-propyl |
| 252. | Et | F | Z15, wherein R^{e} is n-propyl |
| 253. | CH₃ | H | Z15, wherein R^{e} is isopropyl |
| 254. | CH₃ | F | Z15, wherein R^{e} is isopropyl |
| 255. | Et | H | Z15, wherein R^{e} is isopropyl |
| 256. | Et | F | Z15, wherein R^{e} is isopropyl |

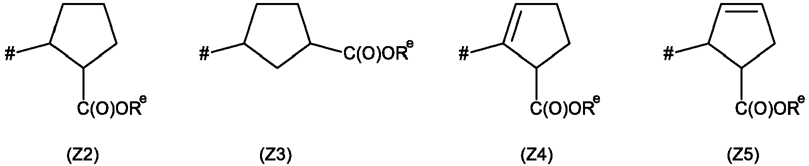

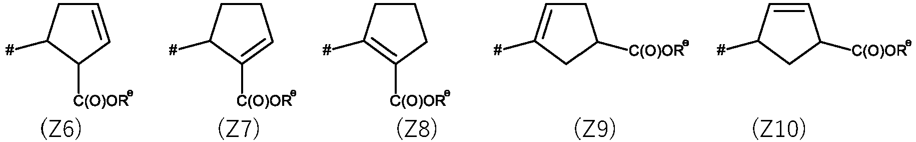

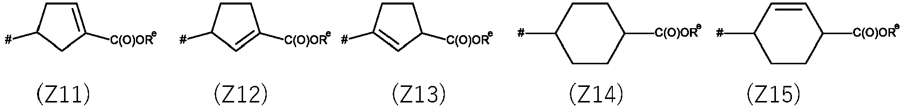

In Z2 to Z15, # denotes the attachment point to NR⁴.

Among rings Z2 to Z15, particular preference is given to rings Z10.

Particularly preferred are moreover compounds (I) in which for a single compound R¹ and R⁴ are hydrogen, R², R³ and-X-Y have one of the meanings as defined in a single line of table A and Q is Q.1, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.1, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.1, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.1, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.1, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.1, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.1, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.1, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.1, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.1, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.2, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.2, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.2, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.2, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.2, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.2, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.2, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.2, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.2, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.2, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.3, where n is 0, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 0, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 0, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 0, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 0, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 0, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 0, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 0, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.3, where n is 0, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.3, where n is 0, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.3, where n is 1, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 1, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 1, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 1, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 1, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 1, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 1, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 1, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.3, where n is 1, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.3, where n is 1, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.3, where n is 2, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 2, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 2, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 2, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 2, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 2, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 2, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.3, where n is 2, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.3, where n is 2, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.3, where n is 2, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.4, where n is 0, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 0, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 0, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 0, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 0, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 0, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 0, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 0, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.4, where n is 0, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.4, where n is 0, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.4, where n is 1, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 1, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 1, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 1, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 1, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 1, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 1, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 1, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.4, where n is 1, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.4, where n is 1, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.4, where n is 2, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 2, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 2, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 2, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 2, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 2, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 2, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.4, where n is 2, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.4, where n is 2, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.4, where n is 2, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is H, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is H, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is H, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is H, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is H, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is H, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is H, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is H, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is H, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is H, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is CH₃, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is CH₃, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is CH₃, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is CH₃, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is CH₃, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is CH₃, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is CH₃ H, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is CH₃, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is CH₃, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.5, where R^{q} is CH₃, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is H, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is H, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is H, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is H, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is H, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is H, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is H, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is H, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is H, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is H, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is CH₃, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is CH₃, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is CH₃, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is CH₃, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is CH₃, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is CH₃, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is CH₃ H, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is CH₃, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is CH₃, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.6, where R^{q} is CH₃, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are H, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are H, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, wherₑ R^{q14} and R^{q15} are H, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, wherₑ R^{q14} and R^{q15} are H, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are H, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are H, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are H, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are H, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are H, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are H, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are F, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are F, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, wherₑ R^{q14} and R^{q15} are F, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, wherₑ R^{q14} and R^{q15} are F, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are F, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are F, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are F, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are F, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are F, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are F, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.7, wherₑ R^{q14} and R^{q15} are CH₃, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are CH₃, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are CH₃, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are CH₃, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are CH₃, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are CH₃, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are CH₃, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are CH₃, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are CH₃, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.7, where R^{q14} and R^{q15} are CH₃, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are H, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are H, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are H, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are H, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are H, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are H, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are H, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are H, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are H, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are H, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are F, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are F, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are F, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are F, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are F, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are F, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are F, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are F, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{a15} are F, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are F, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are CH₃, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are CH₃, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are CH₃, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are CH₃, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are CH₃, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are CH₃, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are CH₃, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are CH₃, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{a15} are CH₃, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.8, where R^{q14} and R^{q15} are CH₃, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.9, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.9, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.9, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.9, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.9, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.9, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.9, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.9, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.9, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.9, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.10, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.10, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.10, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.10, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.10, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.10, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.10, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.10, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.10, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.10, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.11, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.11, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.11, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.11, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.11, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.11, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.11, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.11, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.11, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.11, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.12, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.12, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.12, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.12, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.12, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.12, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.12, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.12, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.12, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.12, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.13, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.13, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.13, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.13, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.13, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.13, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.13, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.13, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.13, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.13, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.14, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.14, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.14, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.14, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.14, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.14, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.14, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.14, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.14, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.14, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.15, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.15, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.15, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.15, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.15, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.15, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.15, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.15, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.15, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.15, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.16, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.16, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.16, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.16, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.16, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.16, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.16, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.16, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.16, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.16, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is H, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is H, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is H, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is H, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is H, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is H, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is H, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is H, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is H, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is H, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is CH₃, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is CH₃, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is CH₃, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is CH₃, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is CH₃, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is CH₃, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is CH₃, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is CH₃, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is CH₃, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.17, where R^{q} is CH₃, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is H, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is H, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is H, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is H, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is H, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is H, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is H, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is H, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is H, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is H, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is CH₃, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is CH₃, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is CH₃, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is CH₃, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is CH₃, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is CH₃, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is CH₃, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is CH₃, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is CH₃, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.18, where R^{q} is CH₃, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is H, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is H, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is H, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is H, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is H, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is H, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is H, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is H, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is H, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is H, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is CH₃, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is CH₃, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is CH₃, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is CH₃, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is CH₃, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is CH₃, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is CH₃, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is CH₃, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is CH₃, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.19, where R^{q} is CH₃, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is H, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is H, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is H, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is H, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is H, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is H, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is H, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is H, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is H, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is H, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is CH₃, R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is CH₃, R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is CH₃, R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is CH₃, R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is CH₃, R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is CH₃, R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is CH₃, R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is CH₃, R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is CH₃, R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.20, where R^{q} is CH₃, R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.21, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.21, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.21, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.21, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.21, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.21, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.21, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.21, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.21, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.21, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.22, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.22, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.22, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.22, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.22, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.22, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.22, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.22, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.22, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.22, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.23, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.23, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.23, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.23, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.23, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.23, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.23, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.23, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.23, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.23, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.24, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.24, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.24, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.24, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.24, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.24, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.24, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.24, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.24, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.24, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.25, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.25, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.25, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.25, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.25, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.25, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.25, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.25, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.25, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.25, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.26, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.26, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.26, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.26, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.26, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.26, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.26, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.26, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.26, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.26, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.27, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.27, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.27, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.27, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.27, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.27, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.27, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.27, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.27, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.27, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.28, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.28, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.28, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.28, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.28, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.28, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.28, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.28, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.28, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.28, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.29, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.29, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.29, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.29, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.29, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.29, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.29, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.29, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.29, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.29, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.30, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.30, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.30, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.30, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.30, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.30, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.30, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.30, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.30, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.30, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.31, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.31, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.31, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.31, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.31, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.31, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.31, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.31, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.31, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.31, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.32, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.32, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.32, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.32, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.32, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.32, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.32, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.32, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.32, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.32, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.33, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.33, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.33, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.33, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.33, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.33, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.33, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.33, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.33, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.33, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.34, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.34, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.34, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.34, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.34, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.34, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.34, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.34, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.34, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.34, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.35, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.35, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.35, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.35, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.35, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.35, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.35, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.35, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.35, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.35, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.36, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.36, where R^{a11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.36, where R^{a11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.36, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.36, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.36, where R^{q12} is Cl and R^{a11} and R^{q13} are H.

Particularly preferred are moreover compounds (I) in which for a single compound R¹ and R⁴ are hydrogen, R², R³ and-X-Y have one of the meanings as defined in a single line of table A and Q is Q.36, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.36, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.36, where R^{q13} is Cl and R^{a11} and R^{q12} are H.

Particularly preferred are moreover compounds (I) in which for a single compound R¹ and R⁴ are hydrogen, R², R³ and-X-Y have one of the meanings as defined in a single line of table A and Q is Q.36, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.37, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.37, where R^{a11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.37, where R^{a11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.37, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.37, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.37, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.37, where R^{q12} is CH₃ and R^{a11} and R^{q13} are H.

Particularly preferred are moreover compounds (I) in which for a single compound R¹ and R⁴ are hydrogen, R², R³ and-X-Y have one of the meanings as defined in a single line of table A and Q is Q.37, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.37, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.37, where R^{q13} is CH₃ and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.38, where R^{q11}, R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.38, where R^{q11} is F and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.38, where R^{q11} is Cl and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.38, where R^{q11} is CH₃ and R^{q12} and R^{q13} are H.

Particularly preferred are moreover compounds (I) in which for a single compound R¹ and R⁴ are hydrogen, R², R³ and-X-Y have one of the meanings as defined in a single line of table A and Q is Q.38, where R^{q12} is F and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.38, where R^{q12} is Cl and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.38, where R^{q12} is CH₃ and R^{q11} and R^{q13} are H.

Particularly preferred are moreover compounds (I) in which for a single compound R¹ and R⁴ are hydrogen, R², R³ and-X-Y have one of the meanings as defined in a single line of table A and Q is Q.38, where R^{q13} is F and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.38, where R^{q13} is Cl and R^{q11} and R^{q12} are H.

Particularly preferred are moreover compounds (I) in which for a single compound R¹ and R⁴ are hydrogen, R², R³ and-X-Y have one of the meanings as defined in a single line of table A and Q is Q.38, where R^{q13} is CH₃ and R^{q11} and R^{q12} are H.

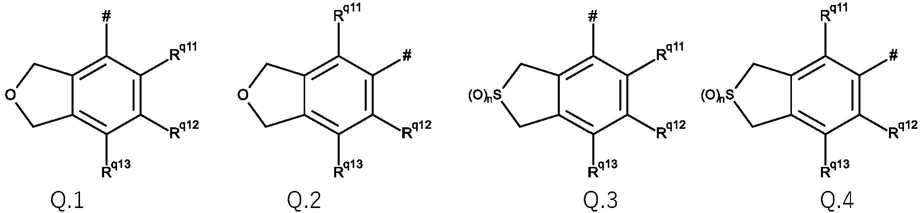

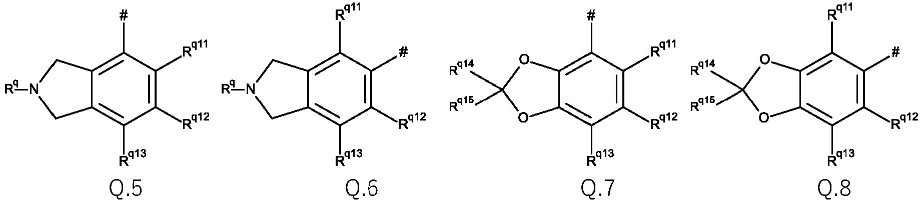

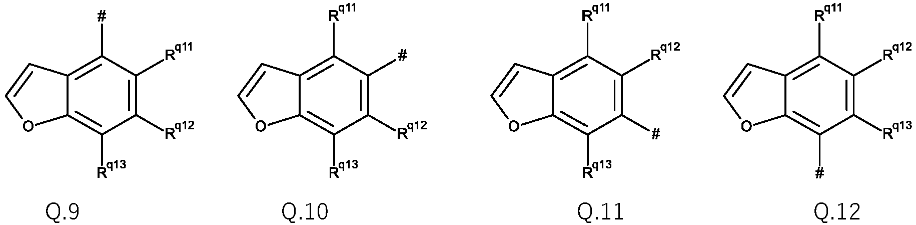

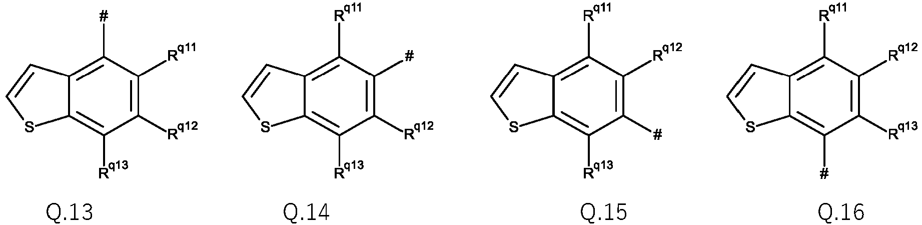

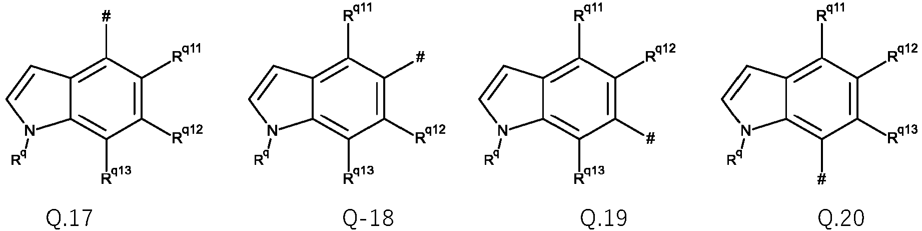

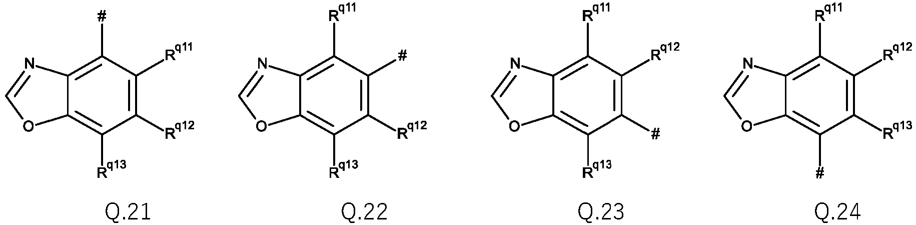

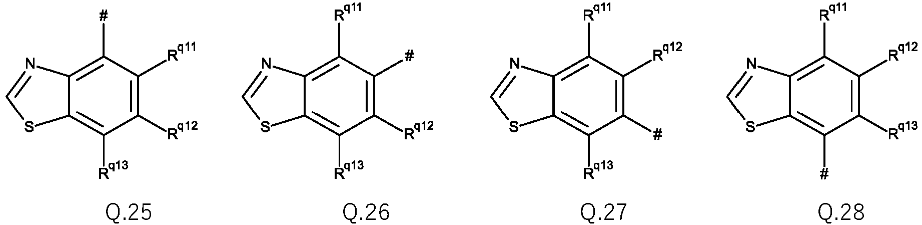

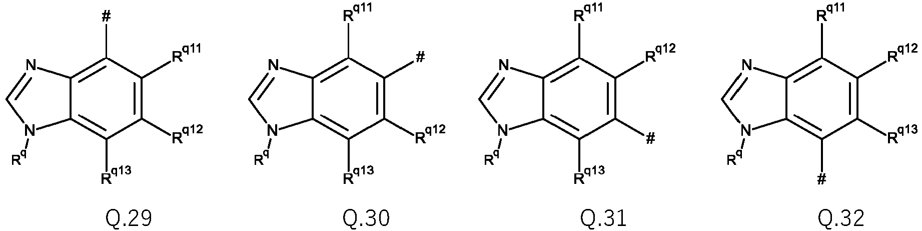

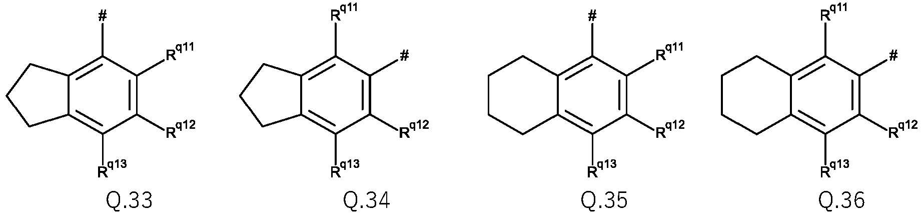

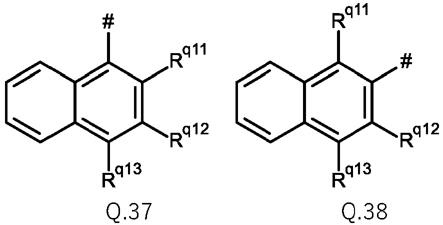

In the above rings Q.1 to Q.38 # denotes the attachment point to NR¹.

The compounds of formula (I) according to the invention can be prepared by standard processes of organic chemistry, for example by the following processes:

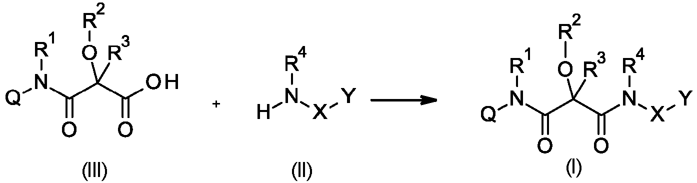

The compounds of formula (I) can be prepared according to methods or in analogy to methods that are described in the prior art. The synthesis takes advantage of starting materials that are commercially available or may be prepared according to conventional procedures starting from readily available compounds.

Compounds of the formula (I) can be prepared from the carboxylic acids (III) and commercially available amines (II) using an organic base and a coupling reagent. Thus, compounds of formula (I) can be synthesized from the corresponding carboxylic acids (leq.) using a coupling reagent (1-2 eq.), for example T₃P (propanephosphonic acid anhydride) or HATU (O-(7-azabenzotriazole-1-yl)-N,N,N',N'-tetramethyluronium-hexafluorphosphate), an organic base (1-3 eq.) and the amines (II) (1-3 eq.). The reaction is typically carried out in an organic solvent. Preferably an aprotic organic solvent is used. Most preferably tetrahydrofuran (THF), N,N-dimethylformamide (DMF) or acetonitrile (ACN) are used. The reaction is carried out at temperatures between 0° C and reflux. Preferably the reaction is carried out at room temperature. Preferably the organic base is triethylamine or N,N-diisopropylethylamine.

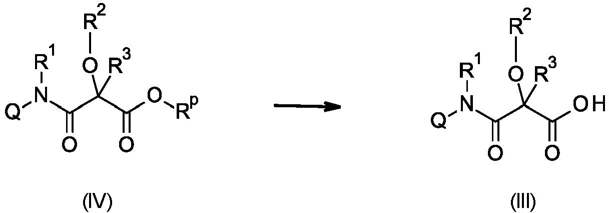

The carboxylic acids (III) are commercially available or can be prepared from the corresponding esters (IV) (wherein R^{P} is alkyl or benzyl). If R^{P} is alkyl, esters (IV) may be cleaved using aqueous alkali metal hydroxides. Preferably lithium hydroxide, sodium hydroxide or potassium hydroxide (1-2 eq.) are employed. The reaction is typically carried out in mixtures of water and an organic solvent. Preferably the organic solvent is THF, methanol or acetonitrile. The reaction is carried out at temperatures between 0° C and 100° C. Preferably the reaction is carried at room temperature. If R^{p} is benzyl in (IV), then the ester may be cleaved using palladium on charcoal (0.001-1eq.) as catalyst and hydrogen gas at temperatures between 0° C and reflux. Preferably the reaction is carried out at room temperature. Typically, an organic solvent is employed. Preferably THF, methanol or ethanol are employed.

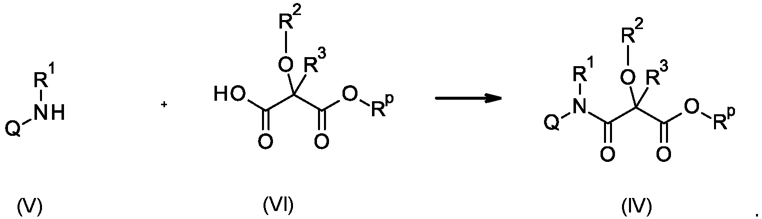

Compounds of the formula (IV) can be prepared from the carboxylic acids (VI) and commercially available amines (V) using a base and a coupling reagent. Thus, compounds of formula (IV) can be synthesized from the corresponding carboxylic acids (leq.) using a coupling reagent (1-2 eq.), for example T₃P (propanephosphonic acid anhydride) or HATU (*O*-(7-azabenzotriazole-1-yl)-N,N,N,N-tetramethyluronium-hexafluorphosphate), an organic base (1-3 eq.) and the amines (V) (1-3 eq.). The reaction is typically carried out in an organic solvent. Preferably an aprotic organic solvent is used. Most preferably tetrahydrofuran (THF), N,N-dimethylformamide (DMF) or acetonitrile (ACN) are used. The reaction is carried out at temperatures between 0° C to refluxing temperatures. Preferably the reaction is carried out at room temperature. Preferably the organic base is triethylamine or *N,N*-diisopropylethylamine.

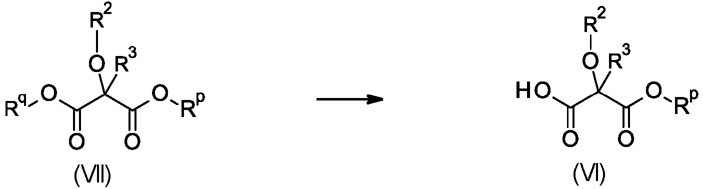

Carboxylic acid (VI) may be prepared from the corresponding diester by selective cleavage of one ester group. If R^{q} is an alkyl ester, selective ester cleavage may be achieved using an aqueous base. Preferably an alkali metal hydroxide is used. Most preferably lithium hydroxide, sodium hydroxide or potassium hydroxide are used. The reaction is typically carried out in mixtures of water and an organic solvent. Preferably THF, methanol or acetonitrile are employed. The reaction is carried out at temperatures between 0° C and 100° C, preferably at room temperature. Alternatively, trimethyltin hydroxide (e.g. 1eq.) in 1,2-dichlorethane at room temperature to reflux may be used (as described in Angew. Chem. Int. Ed, 2005, 44: 1378-1382), preferably at reflux. If R^{q} is benzyl in (VII), then the ester may be cleaved using palladium on charcoal (0.001-1eq.) as catalyst and hydrogen gas at temperatures between 0° C and reflux. Preferably the reaction is carried out at room temperature. Typically, an organic solvent is employed. Preferably THF, methanol or ethanol are employed.

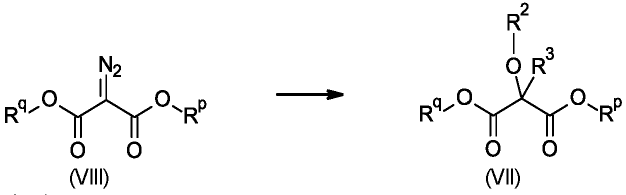

The diesters (VII) are either commercially available or may be prepared from the corresponding diazo-compounds (VIII) using dirhodiumtetraacetat ([Rh(OAc)₂]₂) (0.001-0.1 eq.) and the alcohol HO-R⁷, yielding alkoxy malonates (VII) (R⁸=H). The reaction is typically carried out in an organic solvent, preferably in toluene at temperatures between 0° to 100° C. Preferably the reaction is done at 60° C as described in Angew. Chem. Int. Ed. 2014, 53, 14230-14234. Diazo compounds (VIII), if not commercially available, may be prepared as described in Angew. Chem. Int. Ed. 2014, 53, 14230-14234.

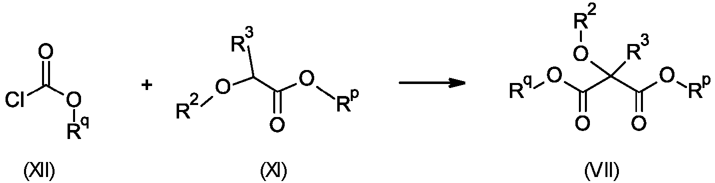

Alternatively, diesters (VII) may be synthesized from a commercially available monoester (XI), a base and a chloroformate (XII) (1-3 eq.) as described in Bioorganic & Medicinal Chemistry Letters, 12(11), 1501-1505; 2002. The reaction is typically carried out in an organic solvent, preferably in tetrahydrofuran. Suitable temperatures range between -78° C and 25° C. Preferably the reaction is allowed to warm from -78° C to 25° C over a period of 16 h. Preferably lithiumdiisopropylamide (leq.) is used as a base.

Alternatively diesters (VII), wherein R⁸ is fluorine, can be prepared from the corresponding non-fluorinated malonates using 1-chloromethyl-4-fluoro-1,4-diazoni-abicyclo[2.2.2]octanebis(tetrafluoroborate) (Selectfluor) as described in WO12/129384. Water and/or an organic solvent are used. Preferably the reaction is carried out in acetonitrile. The reaction is carried out at a temperature between 0° C and reflux temperature, preferably at 60° C using 1 to 4 equivalents of 1-chloromethyl-4-fluoro-1,4-diazoniabicyclo[2.2.2]octanebis(tetrafluoroborate) (Selectfluor). Alternatively, N-Fluorobenzenesulfonimide (CAS 133745-75-2) may be employed (see for example Differding, E., & Ofner, H. (1991). N-Fluorobenzenesulfonimide: A practical reagent for electrophilic fluorinations. Synlett, 1991(03)) 187-189).

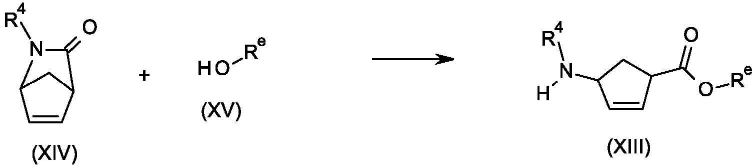

Amines of the formula (XIII) can be prepared from the lactames (XIV), which are either commercially available or may be prepared by alkylation as described in Org. Process Res. Dev. 2018, 22, 337-343, and commercially available alcohols (XV) using thionyl chloride (2eq.) as described in Tetrahedron Lett. 2001, 42, 1347-1350. The reaction is typically carried out in the coupling alcohols (XV) as the solvent. The reaction is carried out at temperatures between 0° C to refluxing temperatures. Preferably the reaction is carried out at room temperature.

To widen the spectrum of action, the compounds of formula (I) may be mixed with many representatives of other herbicidal or growth-regulating active ingredient groups and then applied concomitantly. Suitable components for combinations are, for example, herbicides from the classes of the acetamides, amides, aryloxyphenoxypropionates, benzamides, benzofuran, benzoic acids, benzothiadiazinones, bipyridylium, carbamates, chloroacetamides, chlorocarboxylic acids, cyclohexanediones, dinitroanilines, dinitrophenol, diphenyl ether, glycines, imidazolinones, isoxazoles, isoxazolidinones, nitriles, N-phenylphthalimides, oxadiazoles, oxazolidinediones, oxyacetamides, phenoxycarboxylic acids, phenylcarbamates, phenylpyrazoles, phenylpyrazolines, phenylpyridazines, phosphinic acids, phosphoroamidates, phosphorodithioates, phthalamates, pyrazoles, pyridazinones, pyridines, pyridinecarboxylic acids, pyridinecarboxamides, pyrimidinediones, pyrimidinyl(thio)benzoates, quinolinecarboxylic acids, semicarbazones, sulfonylaminocarbonyltriazolinones, sulfonylureas, tetrazolinones, thiadiazoles, thiocarbamates, triazines, triazinones, triazoles, triazolinones, triazolocarboxamides, triazolopyrimidines, triketones, uracils, ureas.

It may furthermore be beneficial to apply the compounds of formula (I) alone or in combination with other herbicides, or else in the form of a mixture with other crop protection agents, for example together with agents for controlling pests or phytopathogenic fungi or bacteria. Also of interest is the miscibility with mineral salt solutions, which are employed for treating nutritional and trace element deficiencies. Other additives such as non-phytotoxic oils and oil concentrates may also be added.

In one embodiment of the present invention the combinations according to the present invention comprise at least one compound of formula (I) (compound A or component A) and at least one further active compound selected from herbicides B (compound B), preferably herbicides B of class b1) to b15), and safeners C (compound C).

In another embodiment of the present invention the combinations according to the present invention comprise at least one compound of formula (I) and at least one further active compound B (herbicide B).

Examples of herbicides B which can be used in combination with the *compounds* A of formula (I) according to the present invention are:
b1) from the group of the lipid biosynthesis inhibitors:
   ACC-herbicides such as alloxydim, alloxydim-sodium, butroxydim, clethodim, clodinafop, clodinafop-propargyl, cycloxydim, cyhalofop, cyhalofop-butyl, diclofop, diclofop-methyl, fenoxaprop, fenoxaprop-ethyl, fenoxaprop-P, fenoxaprop-P-ethyl, fluazifop, fluazifop-butyl, fluazifop-P, fluazifop-P-butyl, haloxyfop, haloxyfop-methyl, haloxyfop-P, haloxyfop-P-methyl, metamifop, pinoxaden, profoxydim, propaquizafop, quizalofop, quizalofop-ethyl, quizalofop-tefuryl, quizalofop-P, quizalofop-P-ethyl, quizalofop-P-tefuryl, sethoxydim, tepraloxydim, tralkoxydim, 4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2 ' ,4'-dichloro-4-cyclopropyl- [1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2 ' ,4'-Dichloro -4-cyclopropyl- [1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); and non ACC herbicides such as benfuresate, butylate, cycloate, dalapon, dimepiperate, EPTC, esprocarb, ethofumesate, flupropanate, molinate, orbencarb, pebulate, prosulfocarb, TCA, thi-obencarb, tiocarbazil, triallate and vernolate;
b2) from the group of the ALS inhibitors:
   sulfonylureas such as amidosulfuron, azimsulfuron, bensulfuron, bensulfuron-methyl, chlorimuron, chlorimuron-ethyl, chlorsulfuron, cinosulfuron, cyclosulfamuron, ethametsulfuron, ethametsulfuron-methyl, ethoxysulfuron, flazasulfuron, flucetosulfuron, flupyrsulfuron, flupyrsulfuron-methyl-sodium, foramsulfuron, halosulfuron, halosulfuron-methyl, imazosulfuron, iodosulfuron, iodosulfuron-methyl-sodium, iofensulfuron, iofensulfuron-sodium, mesosulfuron, metazosulfuron, metsulfuron, metsulfuron-methyl, nicosulfuron, orthosulfamuron, oxasulfuron, primisulfuron, primisulfuron-methyl, propyrisulfuron, prosulfuron, pyrazosulfuron, pyrazosulfuron-ethyl, rimsulfuron, sulfometuron, sulfometuron-methyl, sulfosulfuron, thifensulfuron, thifensulfuron-methyl, triasulfuron, tribenuron, tribenuron-methyl, trifloxysulfuron, triflusulfuron, triflusulfuron-methyl and tritosulfuron,
   imidazolinones such as imazamethabenz, imazamethabenz-methyl, imazamox, imazapic, imazapyr, imazaquin and imazethapyr, triazolopyrimidine herbicides and sulfonanilides such as cloransulam, cloransulam-methyl, diclosulam, flumetsulam, florasulam, metosulam, penoxsulam, pyrimisulfan and pyroxsulam, pyrimidinylbenzoates such as bispyribac, bispyribac-sodium, pyribenzoxim, pyriftalid, pyriminobac, pyriminobac-methyl, pyrithiobac, pyrithiobac-sodium, 4-[[[2-[(4,6-dimethoxy-2-pyrimidinyl)oxy]phenyl]methyl]amino]-benzoic acid-1-methylethyl ester (CAS 420138-41-6), 4-[[[2-[(4,6-dimethoxy-2-pyrimidinyl)oxy]phenyl]methyl]amino]-benzoic acid propyl ester (CAS 420138-40-5), N-(4-bromophenyl)-2-[(4,6-dimethoxy-2-pyrimidinyl)oxy]benzenemethanamine (CAS 420138-01-8),
   sulfonylaminocarbonyl-triazolinone herbicides such as flucarbazone, flucarbazone-sodium, propoxycarbazone, propoxycarbazone-sodium, thiencarbazone and thiencarbazone-methyl; and triafamone;
   among these, a preferred embodiment of the invention relates to those compositions comprising at least one imidazolinone herbicide;
b3) from the group of the photosynthesis inhibitors:
   amicarbazone, inhibitors of the photosystem II, e.g. 1-(6-tert-butylpyrimidin-4-yl)-2-hydroxy-4-methoxy-3-methyl-2H-pyrrol-5-one (CAS 1654744-66-7), 1-(5-tert-butylisoxazol-3-yl)-2-hydroxy-4-methoxy-3-methyl-2H-pyrrol-5-one (CAS 1637455-12-9), 1-(5-tert-butylisoxazol-3-yl)-4-chloro-2-hydroxy-3-methyl-2H-pyrrol-5-one (CAS 1637453-94-1), 1-(5-tert-butyl-1-methyl-pyrazol-3-yl)-4-chloro-2-hydroxy-3-methyl-2H-pyrrol-5-one (CAS 1654057-29-0), 1-(5-tert-butyl-1-methyl-pyrazol-3-yl)-3-chloro-2-hydroxy-4-methyl-2H-pyrrol-5-one (CAS 1654747-80-4), 4-hydroxy-1-methoxy-5-methyl-3-[4-(trifluoromethyl)-2-pyridyl]imidazolidin-2-one; (CAS 2023785-78-4), 4-hydroxy-1,5-dimethyl-3-[4-(trifluoromethyl)-2-pyridyl]imidazolidin-2-one (CAS 2023785-79-5), 5-ethoxy-4-hydroxy-1-methyl-3-[4-(trifluoromethyl)-2-pyridyl]imidazolidin-2-one (CAS 1701416-69-4), 4-hydroxy-1-methyl-3-[4-(trifluoromethyl)-2-pyridyl]imidazolidin-2-one (CAS 1708087-22-2), 4-hydroxy-1,5-dimethyl-3-[1-methyl-5-(trifluoromethyl)pyrazol-3-yl]imidazolidin-2-one (CAS 2023785-80-8), 1-(5-tert-butylisoxazol-3-yl)-4-ethoxy-5-hydroxy-3-methyl-imidazolidin-2-one (CAS 1844836-64-1), triazine herbicides, including of chlorotriazine, triazinones, triazindiones, methylthiotriazines and pyridazinones such as ametryn, atrazine, chloridazone, cyanazine, desmetryn, dimethametryn,hexazinone, metribuzin, prometon, prometryn, propazine, simazine, simetryn, terbumeton, terbuthylazin, terbutryn and trietazin, aryl urea such as chlorobromuron, chlorotoluron, chloroxuron, dimefuron, diuron, fluometuron, isoproturon, isouron, linuron, metamitron, methabenzthiazuron, metobenzuron, metoxuron, monolinuron, neburon, siduron, tebuthiuron and thiadiazuron, phenyl carbamates such as desmedipham, karbutilat, phenmedipham, phenmedipham-ethyl, nitrile herbicides such as bromofenoxim, bromoxynil and its salts and esters, ioxynil and its salts and esters, uraciles such as bromacil, lenacil and terbacil, and bentazon and bentazon-sodium, pyridate, pyridafol, pentanochlor and propanil and inhibitors of the photosystem I such as diquat, diquat-dibromide, paraquat, paraquat-dichloride and paraquat-dimetilsulfate. Among these, a preferred embodiment of the invention relates to those compositions comprising at least one aryl urea herbicide. Among these, likewise a preferred embodiment of the invention relates to those compositions comprising at least one triazine herbicide. Among these, likewise a preferred embodiment of the invention relates to those compositions comprising at least one nitrile herbicide;
b4) from the group of the protoporphyrinogen-IX oxidase inhibitors:
   acifluorfen, acifluorfen-sodium, azafenidin, bencarbazone, benzfendizone, bifenox, butafenacil, carfentrazone, carfentrazone-ethyl, chlomethoxyfen, chlorphthalim, cinidon-ethyl, cyclopyranil, fluazolate, flufenpyr, flufenpyr-ethyl, flumiclorac, flumiclorac-pentyl, flumioxazin, fluoroglycofen, fluoroglycofen-ethyl, fluthiacet, fluthiacet-methyl, fomesafen, halosafen, lactofen, oxadiargyl, oxadiazon, oxyfluorfen, pentoxazone, profluazol, pyraclonil, pyraflufen, pyraflufen-ethyl, saflufenacil, sulfentrazone, thidiazimin, tiafenacil, trifludimoxazin, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100), N-ethyl-3-(2,6-dichloro-4-trifluoromethylphenoxy)-5-methyl-14-pyrazole-1-carboxamide (CAS 452098-92-9), N-tetrahydrofurfuryl-3-(2,6-dichloro-4-trifluoromethylphenoxy)-5-methyl-14-pyrazole-1-carboxamide (CAS 915396-43-9), N-ethyl-3-(2-chloro-6-fluoro-4-trifluoromethylphenoxy)-5-methyl-1*H*-pyrazole-1-carboxamide (CAS 452099-05-7), N-tetrahydrofurfuryl-3-(2-chloro-6-fluoro-4-trifluoromethylphenoxy)-5-methyl-1H-pyrazole-1-carboxamide (CAS 452100-03-7), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione (CAS 451484-50-7), 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0), 1-methyl-6-trifluoromethyl-3-(2,2,7-tri-fluoro-3-oxo-4-prop-2-ynyl-3,4-dihydro-2H-benzo[1,4]oxazin-6-yl)-1H-pyrimidine-2,4-dione (CAS 1304113-05-0), methyl (*E*)-4-[2-chloro-5-[4-chloro-5-(difluoromethoxy)-1*H*-methyl-pyrazol-3-yl]-4-fluoro-phenoxy]-3-methoxy-but-2-enoate (CAS 948893-00-3), and 3-[7-chloro-5-fluoro-2-(trifluoromethyl)-1H-benzimidazol-4-yl]-1-methyl-6-(trifluoromethyl)-1H-pyrimidine-2,4-dione (CAS 212754-02-4), 2-[2-chloro-5-[3-chloro-5-(trifluoromethyl)-2-pyridinyl]-4-fluorophenoxy]-2-methoxy-acetic acid methyl ester (CAS 1970221-16-9), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy]-acetic acid methyl ester (CAS 2158274-96-3), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy] acetic acid ethyl ester (CAS 158274-50-9), methyl 2-[[3-[2-chloro-5-[4-(difluoromethyl)-3-methyl-5-oxo-1,2,4-triazol-1-yl]-4-fluoro-phenoxy]-2-pyridyl]oxy]acetate (CAS 2271389-22-9), ethyl 2-[[3-[2-chloro-5-[4-(difluoromethyl)-3-methyl-5-oxo-1,2,4-triazol-1-yl]-4-fluoro-phenoxy]-2-pyridyl]oxy]acetate (CAS 2230679-62-4), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy]-acetic acid methyl ester (CAS 2158275-73-9), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy] acetic acid ethyl ester (CAS 2158274-56-5), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy]-N-(methylsulfonyl)-acetamide (CAS 2158274-53-2), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy]-N-(methylsulfonyl)-acetamide (CAS 2158276-22-1);
b5) from the group of the bleacher herbicides:
   PDS inhibitors: beflubutamid, diflufenican, fluridone, flurochloridone, flurtamone, norflurazon, picolinafen, and 4-(3-trifluoromethylphenoxy)-2-(4-trifluoromethylphenyl)pyrimidine (CAS 180608-33-7), HPPD inhibitors: benzobicyclon, benzofenap, bicyclopyrone, clomazone, fenquinotrione, isoxaflutole, mesotrione, oxotrione (CAS 1486617-21-3), pyrasulfotole, pyrazolynate, pyrazoxyfen, sulcotrione, tefuryltrione, tembotrione, tolpyralate, topramezone , bleacher, unknown target: aclonifen, amitrole flumeturon 2-chloro-3-methylsulfanyl-N-(1-methyltetrazol-5-yl)-4-(trifluoromethyl)benzamide (CAS 1361139-71-0), bixlozone and 2-(2,5-dichlorophenyl)methyl-4,4-dimethyl-3-isoxazolidinone (CAS 81778-66-7);
b6) from the group of the EPSP synthase inhibitors:
   glyphosate, glyphosate-isopropylammonium, glyposate-potassium and glyphosate-trimesium (sulfosate);
b7) from the group of the glutamine synthase inhibitors:
   bilanaphos (bialaphos), bilanaphos-sodium, glufosinate, glufosinate-P and glufosinate-ammonium;
b8) from the group of the DHP synthase inhibitors:
   asulam;
b9) from the group of the mitosis inhibitors:
   compounds of group K1: dinitroanilines such as benfluralin, butralin, dinitramine, ethalfluralin, fluchloralin, oryzalin, pendimethalin, prodiamine and trifluralin, phosphoramidates such as amiprophos, amiprophos-methyl, and butamiphos, benzoic acid herbicides such as chlorthal, chlorthal-dimethyl, pyridines such as dithiopyr and thiazopyr, benzamides such as propyzamide and tebutam; compounds of group K2: carbetamide, chlorpropham, flamprop, flamprop-isopropyl, flamprop-methyl, flamprop-M-isopropyl, flamprop-M-methyl and propham ; among these, compounds of group K1, in particular dinitroanilines are preferred;
b10) from the group of the VLCFA inhibitors:
   chloroacetamides such as acetochlor, alachlor, amidochlor, butachlor, di-methachlor, dimethenamid, dimethenamid-P, metazachlor, metolachlor, metolachlor-S, pethoxamid, pretilachlor, propachlor, propisochlor and thenylchlor, oxyacetanilides such as flufenacet and mefenacet, acetanilides such as diphena-mid, naproanilide, napropamide and napropamide-M, tetrazolinones such fentraza-mide, and other herbicides such as anilofos, cafenstrole, fenoxasulfone, ipfencarba-zone, piperophos, pyroxasulfone and isoxazoline compounds of the formulae II.1, II.2, II.3, II.4, II.5, II.6, II.7, II.8 and II.9 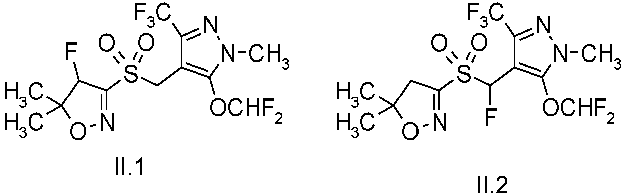 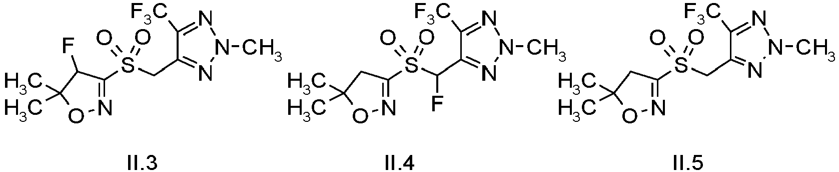 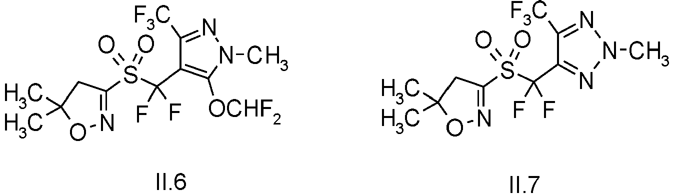 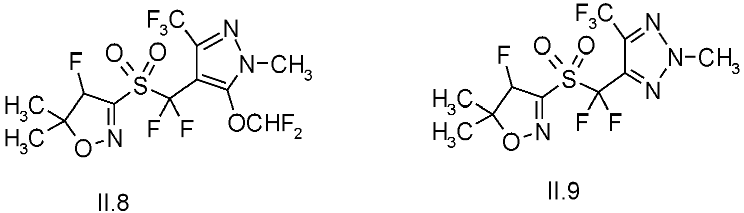
   the isoxazoline compounds of the formula (II) are known in the art, e.g. from WO 2006/024820, WO 2006/037945, WO 2007/071900 and WO 2007/096576;
   among the VLCFA inhibitors, preference is given to chloroacetamides and oxyacetamides;
b11) from the group of the cellulose biosynthesis inhibitors:
   chlorthiamid, dichlobenil, flupoxam, indaziflam, isoxaben, triaziflam and 1-cyclohexyl-5-pentafluorphenyloxy-1⁴-[1,2,4,6]thiatriazin-3-ylamine (CAS 175899-01-1);
b12) from the group of the decoupler herbicides:
   dinoseb, dinoterb and DNOC and its salts;
b13) from the group of the auxinic herbicides:
   2,4-D and its salts and esters such as clacyfos, 2,4-DB and its salts and esters, aminocyclopyrachlor and its salts and esters, aminopyralid and its salts such as aminopyralid-dimethylammonium, aminopyralid-tris(2-hydroxypropyl)ammonium and its esters, benazolin, benazolin-ethyl, chloramben and its salts and esters, clomeprop, clopyralid and its salts and esters, dicamba and its salts and esters, dichlorprop and its salts and esters, dichlorprop-P and its salts and esters, flopyrauxifen, fluroxypyr, fluroxypyr-butometyl, fluroxypyr-meptyl, halauxifen and its salts and esters (CAS 943832-60-8); MCPA and its salts and esters, MCPA-thioethyl, MCPB and its salts and esters, mecoprop and its salts and esters, mecoprop-P and its salts and esters, picloram and its salts and esters, quinclorac, quinmerac, TBA (2,3,6) and its salts and esters, triclopyr and its salts and esters, florpyrauxifen, florpyrauxifen-benzyl (CAS 1390661-72-9) and 4-amino-3-chloro-5-fluoro-6-(7-fluoro-1H-indol-6-yl)picolinic acid (CAS 1629965-65-6);
b14) from the group of the auxin transport inhibitors: diflufenzopyr, diflufenzopyr-sodium, naptalam and naptalam-sodium;
b15) from the group of the other herbicides: bromobutide, chlorflurenol, chlorflurenol-methyl, cinmethylin, cumyluron, cyclopyrimorate (CAS 499223-49-3) and its salts and esters, dalapon, dazomet, difenzoquat, difenzoquat-metilsulfate, dimethipin, DSMA, dymron, endothal and its salts, etobenzanid, flurenol, flurenol-butyl, flurprimidol, fosamine, fosamine-ammonium, indanofan, maleic hydrazide, mefluidide, metam, methiozolin, methyl azide, methyl bromide, methyl-dymron, methyl iodide, MSMA, oleic acid, oxaziclomefone, pelargonic acid, pyributicarb, quinoclamine tetflupyrolimet, and tridiphane.

Moreover, it may be useful to apply the compounds of formula (I) in combination with safeners. Safeners are chemical compounds which prevent or reduce damage on useful plants without having a major impact on the herbicidal action of the compounds of the formula (I) towards undesired vegetation. They can be applied either before sowings (e.g. on seed treatments, shoots or seedlings) or in the pre-emergence application or post-emergence application of the useful plant. The safeners and the compounds of formula (I) and optionally the herbicides B can be applied simultaneously or in succession.

In another embodiment of the present invention the combinations according to the present invention comprise at least one compound of formula (I) and at least one safener C (component C).

Examples of safeners are e.g. (quinolin-8-oxy)acetic acids, 1-phenyl-5-haloalkyl-1H-1,2,4-triazol-3-carboxylic acids, 1-phenyl-4,5-dihydro-5-alkyl-1H-pyrazol-3,5-dicarboxylic acids, 4,5-dihydro-5,5-diaryl-3-isoxazol carboxylic acids, dichloroacetamides, alpha-oximinophenylacetonitriles, acetophenonoximes, 4,6-dihalo-2-phenylpyrimidines, N-[[4-(aminocarbonyl)phenyl]sulfonyl]-2-benzoic amides, 1,8-naphthalic anhydride, 2-halo-4-(haloalkyl)-5-thiazol carboxylic acids, phosphorthiolates and N-alkyl-O-phenylcarbamates and their agriculturally acceptable salts and their agriculturally acceptable derivatives such amides, esters, and thioesters, provided they have an acid group.

Examples of safener compounds C are benoxacor, cloquintocet, cyometrinil, cyprosulfamide, dichlormid, dicyclonon, dietholate, fenchlorazole, fenclorim, flurazole, fluxofenim, furilazole, isoxadifen, mefenpyr, mephenate, naphthalic anhydride, oxabetrinil, 4-(dichloroacetyl)-1-oxa-4-azaspiro[4.5]decane (MON4660, CAS 71526-07-3), 2,2,5-trimethyl-3-(dichloroacetyl)-1,3-oxazolidine (R-29148, CAS 52836-31-4), metcamifen and BPCMS (CAS 54091-06-4).

The active compounds B of groups b1) to b15) and the active compounds C are known herbicides and safeners, see, for example, The Compendium of Pesticide Common Names (http://www.alanwood.net/pesticides/); Farm Chemicals Handbook 2000 volume 86, Meister Publishing Company, 2000; B. Hock, C. Fedtke, R. R. Schmidt, Herbizide [Herbicides], Georg Thieme Verlag, Stuttgart 1995; W. H. Ahrens, Herbicide Handbook, 7th edition, Weed Science Society of America, 1994; and K. K. Hatzios, Herbicide Handbook, Supplement for the 7th edition, Weed Science Society of America, 1998. 2,2,5-Trimethyl-3-(dichloroacetyl)-1,3-oxazolidine [CAS No. 52836-31-4] is also referred to as R-29148. 4-(Dichloroacetyl)-1-oxa-4-azaspiro[4.5]decane [CAS No. 71526-07-3] is also referred to as AD-67 and MON 4660.

The assignment of the active compounds to the respective mechanisms of action is based on current knowledge. If several mechanisms of action apply to one active compound, this substance was only assigned to one mechanism of action.

The invention also relates to formulations comprising at least an auxiliary and at least one compound of formula (I) according to the invention.

A formulation comprises a pesticidally effective amount of a compound of formula (I). The term "effective amount" denotes an amount of the combination or of the compound of formula (I), which is sufficient for controlling undesired vegetation, especially for controlling undesired vegetation in crops (i.e. cultivated plants) and which does not result in a substantial damage to the treated crop plants. Such an amount can vary in a broad range and is dependent on various factors, such as the undesired vegetation to be controlled, the treated crop plants or material, the climatic conditions and the specific compound of formula (I) used.

The compounds of formula (I), their salts, amides, esters or thioesters can be converted into customary types of formulations, e. g. solutions, emulsions, suspensions, dusts, powders, pastes, granules, pressings, capsules, and mixtures thereof. Examples for formulation types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further formulation types are defined in the "Catalogue of pesticide formulation types and international coding system", Technical Monograph No. 2, 6th Ed. May 2008, CropLife International.

The formulations are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetting agents, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compounds of formula (I) on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), inorganic clays (organically modified or unmodified), polycarboxylates, and silicates.

Suitable bactericides are bronopol and isothiazolinone derivatives such as alkyli-sothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for formulation types and their preparation are:
i) Water-soluble concentrates (SL, LS) 10-60 wt% of a compound of formula (I) or a combination comprising at least one compound of formula (I) (component A) and at least one further compound selected from the herbicidal compounds B (component B) and safeners C (component C) according to the invention and 5-15 wt% wetting agent (e.g. alcohol alkoxylates) are dissolved in water and/or in a water-soluble solvent (e.g. alcohols) ad 100 wt%. The active substance dissolves upon dilution with water.
ii) Dispersible concentrates (DC) 5-25 wt% of a compound of formula (I) or a combination comprising at least one compound of formula (I) (component A) and at least one further compound selected from the herbicidal compounds B (component B) and safeners C (component C) according to the invention and 1-10 wt% dispersant (e. g. polyvinylpyrrolidone) are dissolved in organic solvent (e.g. cyclohexanone) ad 100 wt%. Dilution with water gives a dispersion.
iii) Emulsifiable concentrates (EC) 15-70 wt% of compound of formula (I) or a combination comprising at least one compound of formula (I) (component A) and at least one further compound selected from the herbicidal compounds B (component B) and safeners C (component C) according to the invention and 5-10 wt% emulsifiers (e.g. calcium dodecylbenzenesulfonate and castor oil ethoxylate) are dissolved in water-insoluble organic solvent (e.g. aromatic hydrocarbon) ad 100 wt%. Dilution with water gives an emulsion.
iv) Emulsions (EW, EO, ES) 5-40 wt% of compound of formula (I) or a combination comprising at least one compound of formula (I) (component A) and at least one further compound selected from the herbicidal compounds B (component B) and safeners C (component C) according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into water ad 100 wt% by means of an emulsifying machine and made into a homogeneous emulsion. Dilution with water gives an emulsion.
v) Suspensions (SC, OD, FS) In an agitated ball mill, 20-60 wt% of a compound of formula (I) or a combination comprising at least one compound of formula (I) (component A) and at least one further compound selected from the herbicidal compounds B (component B) and safeners C (component C)according to the invention are comminuted with addition of 2-10 wt% dispersants and wetting agents (e.g. sodium lignosulfonate and alcohol ethoxylate), 0,1-2 wt% thickener (e.g. xanthan gum) and water ad 100 wt% to give a fine active substance suspension. Dilution with water gives a stable suspension of the active substance. For FS type formulation up to 40 wt% binder (e.g. polyvinylalcohol) is added.
vi) Water-dispersible granules and water-soluble granules (WG, SG) 50-80 wt% of a compound of formula (I) or a combination comprising at least one compound of formula (I) (component A) and at least one further compound selected from the herbicidal compounds B (component B) and safeners C (component C)according to the invention are ground finely with addition of dispersants and wetting agents (e.g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS) 50-80 wt% of a compound of formula (I) or a combination comprising at least one compound of formula (I) (component A) and at least one further compound selected from the herbicidal compounds B (component B) and safeners C (component C) according to the invention are ground in a rotor-stator mill with addition of 1-5 wt% dispersants (e.g. sodium lignosulfonate), 1-3 wt% wetting agents (e.g. alcohol ethoxylate) and solid carrier (e.g. silica gel) ad 100 wt%. Dilution with water gives a stable dispersion or solution of the active substance.
viii) Gel (GW, GF) In an agitated ball mill, 5-25 wt% of a compound of formula (I) or a combination comprising at least one compound of formula (I) (component A) and at least one further compound selected from the herbicidal compounds B (component B) and safeners C (component C) according to the invention are comminuted with addition of 3-10 wt% dispersants (e.g. sodium lignosulfonate), 1-5 wt% thickener (e.g. carboxymethylcellulose) and water ad 100 wt% to give a fine suspension of the active substance. Dilution with water gives a stable suspension of the active substance.
iv) Microemulsion (ME) 5-20 wt% of a compound of formula (I) or a combination comprising at least one compound of formula (I) (component A) and at least one further compound selected from the herbicidal compounds B (component B) and safeners C (component C) according to the invention are added to 5-30 wt% organic solvent blend (e.g. fatty acid dimethylamide and cyclohexanone), 10-25 wt% surfactant blend (e.g. alcohol ethoxylate and arylphenol ethoxylate), and water ad 100 %. This mixture is stirred for 1 h to produce spontaneously a thermodynamically stable microemulsion.
iv) Microcapsules (CS) An oil phase comprising 5-50 wt% of a compound of formula (I) or a combination comprising at least one compound of formula (I) (component A) and at least one further compound selected from the herbicidal compounds B (component B) and safeners C (component C) according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound of formula (I) according to the invention, 0-40 wt% water insoluble organic solvent (e.g. aromatic hydrocarbon), and an isocyanate monomer (e.g. diphenylmethene-4,4'-diisocyanate) are dispersed into an aqueous solution of a protective colloid (e.g. polyvinyl alcohol). The addition of a polyamine (e.g. hexamethylenediamine) results in the formation of polyurea microcapsules. The monomers amount to 1-10 wt%. The wt% relate to the total CS formulation.
ix) Dustable powders (DP, DS) 1-10 wt% of a compound of formula (I) or a combination comprising at least one compound of formula (I) (component A) and at least one further compound selected from the herbicidal compounds B (component B) and safeners C (component C) according to the invention are ground finely and mixed intimately with solid carrier (e.g. finely divided kaolin) ad 100 wt%.
x) Granules (GR, FG) 0.5-30 wt% of a compound of formula (I) or a combination comprising at least one compound of formula (I) (component A) and at least one further compound selected from the herbicidal compounds B (component B) and safeners C (component C) according to the invention is ground finely and associated with solid carrier (e.g. silicate) ad 100 wt%. Granulation is achieved by extrusion, spray-drying or the fluidized bed.
xi) Ultra-low volume liquids (UL) 1-50 wt% of a compound of formula (I) or a combination comprising at least one compound of formula (I) (component A) and at least one further compound selected from the herbicidal compounds B (component B) and safeners C (component C) according to the invention are dissolved in organic solvent (e.g. aromatic hydrocarbon) ad 100 wt%.

The formulation types i) to xi) may optionally comprise further auxiliaries, such as 0,1-1 wt% bactericides, 5-15 wt% anti-freezing agents, 0,1-1 wt% anti-foaming agents, and 0,1-1 wt% colorants.

The formulations and/or combinations generally comprise between 0.01 and 95%, preferably between 0.1 and 90%, and in particular between 0.5 and 75%, by weight of the compounds of formula (I).

The compounds of formula (I) are employed in a purity of from 90% to 100%, preferably from 95% to 100% (according to NMR spectrum).

Solutions for seed treatment (LS), suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The formulations in question give, after two-to-tenfold dilution, active substance concentrations of from 0.01 to 60% by weight, preferably from 0.1 to 40% by weight, in the ready-to-use preparations. (nach unten verschoben)

Methods for applying compounds of formula (I), formulations and /or combinations thereof, on to plant propagation material, especially seeds, include dressing, coating, pelleting, dusting, soaking and in-furrow application methods of the propagation material. Preferably, compounds of formula (I), formulations and /or combinations thereof, respectively, are applied on to the plant propagation material by a method such that germination is not induced, e. g. by seed dressing, pelleting, coating and dusting.

Various types of oils, wetting agents, adjuvants, fertilizer, or micronutrients, and further pesticides (e.g. herbicides, insecticides, fungicides, growth regulators, safeners) may be added to the compounds of formula (I), the formulations and/or the combinations comprising them as premix or, if appropriate not until immediately prior to use (tank mix). These agents can be admixed with the formulations according to the invention in a weight ratio of 1:100 to 100:1, preferably 1:10 to 10:1.

The user applies the compounds of formula (I) according to the invention, the formulations and/or the combinations comprising them usually from a pre-dosage device, a knapsack sprayer, a spray tank, a spray plane, or an irrigation system. Usually, the formulation is made up with water, buffer, and/or further auxiliaries to the desired application concentration and the ready-to-use spray liquor or the formulation according to the invention is thus obtained. Usually, 20 to 2000 liters, preferably 50 to 400 liters, of the ready-to-use spray liquor are applied per hectare of agricultural useful area.

According to one embodiment, either individual components of the formulation according to the invention or partially premixed components, e. g. components comprising compounds of formula (I) and optionally active substances from the groups B and/or C), may be mixed by the user in a spray tank and further auxiliaries and additives may be added, if appropriate.

In a further embodiment, individual components of the formulation according to the invention such as parts of a kit or parts of a binary or ternary mixture may be mixed by the user himself in a spray tank and further auxiliaries may be added, if appropriate.

In a further embodiment, either individual components of the formulation according to the invention or partially premixed components, e. g components comprising compounds of formula (I) and optionally active substances from the groups B and/or C), can be applied jointly (e.g. after tank mix) or consecutively.

The compounds of formula (I), are suitable as herbicides. They are suitable as such, as an appropriate formulation or in combination with at least one further compound selected from the herbicidal active compounds B (component B) and safeners C (component C).

The compounds of formula (I), or the formulations and /or combinations comprising the compounds of formula (I), control undesired vegetation on non-crop areas very efficiently, especially at high rates of application. They act against broad-leaved weeds and grass weeds in crops such as wheat, rice, maize, soya and cotton without causing any significant damage to the crop plants. This effect is mainly observed at low rates of application.

The compounds of the invention are useful for controlling for example following weeds: *Abutilon theophrasti* (ABUTH), *Alopercurus myosuroides* (ALOMY), *Amaranthus retroflexus* (AMARE), *Apera spica-venti* (APESV), *Avena fatua* (AVEFA), *Echinocloa crus-galli*(ECHCG), *Setaria faberi* (SETFA), *Setaria viridis* (SETVI), to name just a few representative examples.

The compounds of formula (I), or the formulations and/or the combinations comprising them, are applied to the plants mainly by spraying the leaves. Here, the application can be carried out using, for example, water as carrier by customary spraying techniques using spray liquor amounts of from about 100 to 1000 l/ha (for example from 300 to 400 l/ha). The compounds of formula (I), or the formulations and/or the combinations comprising them, may also be applied by the low-volume or the ultra-low-volume method, or in the form of microgranules.

Application of the compounds of formula (I), or the formulations and/or the combinations comprising them, can be done before, during and/or after, preferably during and/or after, the emergence of the undesired vegetation.

Application of the compounds of formula (I), or the formulations and/or the combinations can be carried out before or during sowing.

The compounds of formula (I), or the formulations and/or the combinations comprising them, can be applied pre-, post-emergence or pre-plant, or together with the seed of a crop plant. It is also possible to apply the compounds of formula (I), or the formulations and/or the combinations comprising them, by applying seed, pretreated with the compounds of formula (I), or the formulations and/or the combinations comprising them, of a crop plant. If the active ingredients are less well tolerated by certain crop plants, application techniques may be used in which the combinations are sprayed, with the aid of the spraying equipment, in such a way that as far as possible they do not come into contact with the leaves of the sensitive crop plants, while the active ingredients reach the leaves of undesired vegetation growing underneath, or the bare soil surface (post-directed, lay-by).

In a further embodiment, the compounds of formula (I), or the formulations and/or the combinations comprising them, can be applied by treating seed. The treatment of seeds comprises essentially all procedures familiar to the person skilled in the art (seed dressing, seed coating, seed dusting, seed soaking, seed film coating, seed multilayer coating, seed encrusting, seed dripping and seed pelleting) based on the compounds of formula (I), or the formulations and/or the combinations prepared therefrom. Here, the combinations can be applied diluted or undiluted.

The term "seed" comprises seed of all types, such as, for example, corns, seeds, fruits, tubers, seedlings and similar forms. Here, preferably, the term seed describes corns and seeds. The seed used can be seed of the crop plants mentioned above, but also the seed of transgenic plants or plants obtained by customary breeding methods.

When employed in plant protection, the amounts of active substances applied, i.e. the compounds of formula (I), component B and, if appropriate, component C without formulation auxiliaries, are, depending on the kind of effect desired, from 0.001 to 2 kg per ha, preferably from 0.005 to 2 kg per ha, more preferably from 0.05 to 0.9 kg per ha and in particular from 0.1 to 0.75 kg per ha.

In another embodiment of the invention, the application rate of the compounds of formula (I), component B and, if appropriate, component C, is from 0.001 to 3 kg/ha, preferably from 0.005 to 2.5 kg/ha and in particular from 0.01 to 2 kg/ha of active substance (a.s.).

In another preferred embodiment of the invention, the rates of application of the compounds of formula (I) according to the present invention (total amount of compounds of formula (I)) are from 0.1 g/ha to 3000 g/ha, preferably 10 g/ha to 1000 g/ha, depending on the control target, the season, the target plants and the growth stage.

In another preferred embodiment of the invention, the application rates of the compounds of formula (I) are in the range from 0.1 g/ha to 5000 g/ha and preferably in the range from 1 g/ha to 2500 g/ha or from 5 g/ha to 2000 g/ha.

In another preferred embodiment of the invention, the application rate of the compounds of formula (I) is 0.1 to 1000 g/ha, preferably1 to 750 g/ha, more preferably 5 to 500 g/ha.

The required application rates of herbicidal compounds B are generally in the range of from 0.0005 kg/ha to 2.5 kg/ha and preferably in the range of from 0.005 kg/ha to 2 kg/ha or 0.01 kg/ha to 1.5 kg/h of a.s.

The required application rates of safeners C are generally in the range of from 0.0005 kg/ha to 2.5 kg/ha and preferably in the range of from 0.005 kg/ha to 2 kg/ha or 0.01 kg/ha to 1.5 kg/h of a.s.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

In another embodiment of the invention, to treat the seed, the amounts of active substances applied, i.e. the compounds of formula (I), component B and, if appropriate, component C are generally employed in amounts of from 0.001 to 10 kg per 100 kg of seed.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

In case of combinations according to the present invention it is immaterial whether the compounds of formula (I), and the further component B and/or the component C are formulated and applied jointly or separately.

In the case of separate application, it is of minor importance, in which order the application takes place. It is only necessary, that the compounds of formula (I), and the further component B and/or the component C are applied in a time frame that allows simultaneous action of the active ingredients on the plants, preferably within a time-frame of at most 14 days, in particular at most 7 days.

Depending on the application method in question, the compounds of formula (I), or the formulations and /or combinations comprising them, can additionally be employed in a further number of crop plants for eliminating undesired vegetation. Examples of suitable crops are the following:
*Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, lpomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis and Prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera* and *Zea mays.*

Preferred crops are *Arachis hypogaea, Beta vulgaris spec. altissima, Brassica napus var. napus, Brassica oleracea, Citrus limon, Citrus sinensis, Coffea arabica (Coffea canephora, Coffea liberica), Cynodon dactylon, Glycine max, Gossypium hirsutum, (Gossypium arboreum, Gossypium herbaceum, Gossypium vitifolium), Helianthus annuus, Hordeum vulgare, Juglans regia, Lens culinaris, Linum usitatissimum, Lycopersicon lycopersicum, Malus spec., Medicago sativa, Nicotiana tabacum (N.rustica), Olea europaea, Oryza sativa, Phaseolus lunatus, Phaseolus vulgaris, Pistacia vera, Pisum sativum, Prunus dulcis, Saccharum officinarum, Secale cereale, Solanum tuberosum, Sorghum bicolor (s. vulgare), Triticale, Triticum aestivum, Triticum durum, Vicia faba, Vitis vinifera* and *Zea mays.*

Especially preferred crops are crops of cereals, corn, soybeans, rice, oilseed rape, cotton, potatoes, peanuts or permanent crops.

The compounds of formula (I) according to the invention, or the formulations and /or combinations comprising them, can also be used in crops which have been modified by mutagenesis or genetic engineering in order to provide a new trait to a plant or to modify an already present trait.

The term "crops" as used herein includes also (crop) plants which have been modified by mutagenesis or genetic engineering in order to provide a new trait to a plant or to modify an already present trait.

Mutagenesis includes techniques of random mutagenesis using X-rays or mutagenic chemicals, but also techniques of targeted mutagenesis, in order to create mutations at a specific locus of a plant genome. Targeted mutagenesis techniques frequently use oligonucleotides or proteins like CRISPR/Cas, zinc-finger nucleases, TALENs or meganucleases to achieve the targeting effect.

Genetic engineering usually uses recombinant DNA techniques to create modifications in a plant genome which under natural circumstances cannot readily be obtained by cross breeding, mutagenesis or natural recombination. Typically, one or more genes are integrated into the genome of a plant in order to add a trait or improve a trait. These integrated genes are also referred to as transgenes in the art, while plant comprising such transgenes are referred to as transgenic plants. The process of plant transformation usually produces several transformation events, which differ in the genomic locus in which a transgene has been integrated. Plants comprising a specific transgene on a specific genomic locus are usually described as comprising a specific "event", which is referred to by a specific event name. Traits which have been introduced in plants or have been modified include in particular herbicide tolerance, insect resistance, increased yield and tolerance to abiotic conditions, like drought.

Herbicide tolerance has been created by using mutagenesis as well as using genetic engineering. Plants which have been rendered tolerant to acetolactate synthase (ALS) inhibitor herbicides by conventional methods of mutagenesis and breeding comprise plant varieties commercially available under the name Clear-field^{®}. However, most of the herbicide tolerance traits have been created via the use of transgenes.

Herbicide tolerance has been created to glyphosate, glufosinate, 2,4-D, dicamba, oxynil herbicides, like bromoxynil and ioxynil, sulfonylurea herbicides, ALS inhibitor herbicides and 4-hydroxyphenylpyruvate dioxygenase (HPPD) inhibitors, like isoxaflutole and mesotrione.

Transgenes which have been used to provide herbicide tolerance traits comprise: for tolerance to glyphosate: cp4 epsps, epsps grg23ace5, mepsps, 2mepsps, gat4601, gat4621 and goxv247, for tolerance to glufosinate: pat and bar, for tolerance to 2,4-D: aad-1 and aad-12, for tolerance to dicamba: dmo, for tolerance to oxynil herbicies: bxn, for tolerance to sulfonylurea herbicides: zm-hra, csr1-2, gm-hra, S4-HrA, for tolerance to ALS inhibitor herbicides: csr1-2, for tolerance to HPPD inhibitor herbicides: hppdPF, W336 and avhppd-03.

Transgenic corn events comprising herbicide tolerance genes are for example, but not excluding others, DAS40278, MON801, MON802, MON809, MON810, MON832, MON87411, MON87419, MON87427, MON88017, MON89034, NK603, GA21, MZHG0JG, HCEM485, VCO-Ø1981-5, 676, 678, 680, 33121, 4114, 59122, 98140, Bt10, Bt176, CBH-351, DBT418, DLL25, MS3, MS6, MZIR098, T25, TC1507 and TC6275.

Transgenic soybean events comprising herbicide tolerance genes are for example, but not excluding others, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHTØH2, W62, W98, FG72 and CV127.

Transgenic cotton events comprising herbicide tolerance genes are for example, but not excluding others, 19-51a, 31707, 42317, 81910, 281-24-236, 3006-210-23, BXN10211, BXN10215, BXN10222, BXN10224, MON1445, MON1698, MON88701, MON88913, GHB119, GHB614, LLCotton25, T303-3 and T304-40.

Transgenic canola events comprising herbicide tolerance genes are for example, but not excluding others, MON88302, HCR-1, HCN10, HCN28, HCN92, MS1, MS8, PHY14, PHY23, PHY35, PHY36, RF1, RF2 and RF3.

Insect resistance has mainly been created by transferring bacterial genes for insecticidal proteins to plants. Transgenes which have most frequently been used are toxin genes of *Bacillus spec.* and synthetic variants thereof, like cry1A, cry1Ab, cry1Ab-Ac, cry1Ac, cry1A.105, cry1F, cry1Fa2, cry2Ab2, cry2Ae, mcry3A, ecry3.1Ab, cry3Bb1, cry34Ab1, cry35Ab1, cry9C, vip3A(a), vip3Aa20. However, also genes of plant origin have been transferred to other plants. In particular genes coding for protease inhibitors, like CpTI and pinII. A further approach uses transgenes in order to produce double stranded RNA in plants to target and downregulate insect genes. An example for such a transgene is dvsnf7.

Transgenic corn events comprising genes for insecticidal proteins or double stranded RNA are for example, but not excluding others, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098.

Transgenic soybean events comprising genes for insecticidal proteins are for example, but not excluding others, MON87701, MON87751 and DAS-81419.

Transgenic cotton events comprising genes for insecticidal proteins are for example, but not excluding others, SGK321, MON531, MON757, MON1076, MON15985, 31707, 31803, 31807, 31808, 42317, BNLA-601, Event1, COT67B, COT102, T303-3, T304-40, GFM Cry1A, GK12, MLS 9124, 281-24-236, 3006-210-23, GHB119 and SGK321.

Increased yield has been created by increasing ear biomass using the transgene athb17, being present in corn event MON87403, or by enhancing photosynthesis using the transgene bbx32, being present in the soybean event MON87712.

Crops comprising a modified oil content have been created by using the transgenes: gm-fad2-1, Pj.D6D, Nc.Fad3, fad2-1A and fatb1-A. Soybean events comprising at least one of these genes are: 260-05, MON87705 and MON87769.

Tolerance to abiotic conditions, in particular to tolerance to drought, has been created by using the transgene cspB, comprised by the corn event MON87460 and by using the transgene Hahb-4, comprised by soybean event IND-ØØ41Ø-5.

Traits are frequently combined by combining genes in a transformation event or by combining different events during the breeding process. Preferred combination of traits are herbicide tolerance to different groups of herbicides, insect tolerance to different kind of insects, in particular tolerance to lepidopteran and coleopteran insects, herbicide tolerance with one or several types of insect resistance, herbicide tolerance with increased yield as well as a combination of herbicide tolerance and tolerance to abiotic conditions.

Plants comprising singular or stacked traits as well as the genes and events providing these traits are well known in the art. For example, detailed information as to the mutagenized or integrated genes and the respective events are available from websites of the organizations "International Service for the Acquisition of Agri-bio-tech Applications (ISAAA)" (http://www.isaaa.org/gmapprovaldatabase) and the "Center for Environmental Risk Assessment (CERA)" (http://cera-gmc.org/GMCropDatabase), as well as in patent applications, like EP3028573 and WO2017/011288.

The use of the compounds of formula (I) or formulations or combinations comprising them according to the invention on crops may result in effects which are specific to a crop comprising a certain gene or event. These effects might involve changes in growth behavior or changed resistance to biotic or abiotic stress factors. Such effects may in particular comprise enhanced yield, enhanced resistance or tolerance to insects, nematodes, fungal, bacterial, mycoplasma, viral or viroid pathogens as well as early vigor, early or delayed ripening, cold or heat tolerance as well as changed amino acid or fatty acid spectrum or content.

Furthermore, plants are also covered that contain by the use of recombinant DNA techniques a modified amount of ingredients or new ingredients, specifically to improve raw material production, e.g., potatoes that produce increased amounts of amylopectin (e.g. Amflora^{®} potato, BASF SE, Germany).

Furthermore, it has been found that the compounds of formula (I) according to the invention, or the formulations and /or combinations comprising them, are also suitable for the defoliation and/or desiccation of plant parts of crops such as cotton, potato, oilseed rape, sunflower, soybean or field beans, in particular cotton. In this regard, formulations and /or combinations for the desiccation and/or defoliation of crops, processes for preparing these formulations and /or combinations and methods for desiccating and/or defoliating plants using the compounds of formula (I) have been found.

As desiccants, the compounds of formula (I) are particularly suitable for desiccating the above-ground parts of crop plants such as potato, oilseed rape, sunflower and soybean, but also cereals. This makes possible the fully mechanical harvesting of these important crop plants.

Also of economic interest is to facilitate harvesting, which is made possible by concentrating within a certain period of time the dehiscence, or reduction of adhesion to the tree, in citrus fruit, olives and other species and varieties of pernicious fruit, stone fruit and nuts. The same mechanism, i.e. the promotion of the development of abscission tissue between fruit part or leaf part and shoot part of the plants is also essential for the controlled defoliation of useful plants, in particular cotton.

Moreover, a shortening of the time interval in which the individual cotton plants mature leads to an increased fiber quality after harvesting.

### A Synthesis Examples

Chemical bonds, drawn as bars in chemical formulae, indicate the relative stereochemistry on the ring system.

### Example 1: Synthesis of 3-[[(1R,4S)-4-isopropoxycarbonylcyclopent-2-en-1-yl]amino]-2-methoxy-3-oxo-propanoic acid (Inter A)

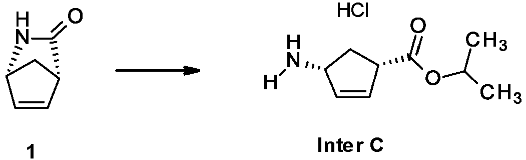

To a solution of (1R,4S)-2-azabicyclo[2.2.1]hept-5-en-3-one (CAS 79200-56-9) (33.3 g, 305 mmol) (3.0 g, 24 mmol) in isopropanol (100 mL) thionyl chloride (72.6 g, 610 mmol) were added at 0 ° C. After stirring for 2 h and letting the mixture warm up to room temperature, the solvent was removed under reduced pressure to afford Inter C (54 g, 86%) as a colorless salt.

¹H NMR (400 MHz, D₂O) δ 6.23 (ddd, *J* = 5.7, 2.4, 1.6 Hz, 1H), 5.99 (dt, *J* = 5.7, 2.3 Hz, 1H), 5.03 (hept, *J* = 6.2 Hz, 1H), 4.42 (ddt, *J* = 6.7, 4.9, 1.9 Hz, 1H), 3.75 (dddd, *J* = 10.4, 4.1, 3.3, 2.0 Hz, 1H), 2.70 (dt, *J =* 14.4, 8.5 Hz, 1H), 2.08 (dt, *J =* 14.4, 5.2 Hz, 1H), 1.28 (dd, *J* = 6.3, 3.0 Hz, 6H).

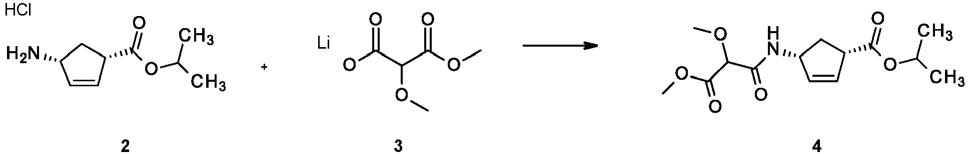

To a solution of the lithium 2,3-dimethoxy-3-oxo-propanoate (**3**) (37.5 g, 243 mmol) in dimethylformamide (DMF, 250 mL) the hydrochloride salt of isopropyl (1S,4R)-4-aminocyclopent-2-ene-1-carboxylate (**2**) (50 g, 243 mmol) (CAS 229613-83-6) was added. To the resulting solution was added HATU (101 g, 729 mmol) (CAS: 148893-10-1) and then diisopropylethylamine (124 mL). After stirring overnight, the reaction was quenched with water (50 mL) and sat. aqueous bicarbonate solution (50 mL) The aqueous layer was separated and extracted with ethyl acetate (3 x 100 mL). The combined organic phases were dried (sodium sulfate), filtered and evaporated under reduced pressure. The crude product (**4**) (72 g, 99%) was obtained as a 1:1 mixture of diastereoisomers and was used in the next step without further purification.

¹H NMR (500 MHz, chloroform-d) δ 7.10 - 6.98 (m, 1H), 5.98 - 5.79 (m, 2H), 5.13 - 4.97 (m, 2H), 4.34 - 4.25 (m, 1H), 3.83 (s, 3H), 3.56 - 3.44 (m, 4H), 2.55 - 2.41 (m, 1H), 1.96 - 1.85 (m, 1H), 1.29 - 1.23 (m, 6H).

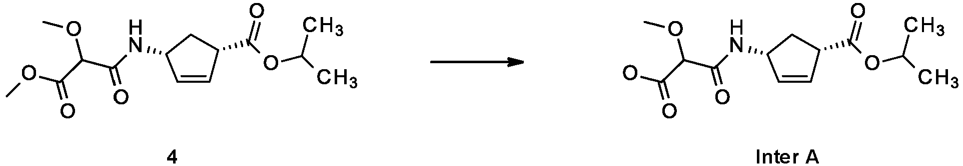

To a solution of isopropyl (1S,4R)-4-[(2,3-dimethoxy-3-oxo-propanoyl)amino]cyclopent-2-ene-1-carboxylate (**4**) (21.1 g, 70.5 mmol) in 1,2-dichloroethane (200 mL) was added trimethyltin hydroxide (Me₃SnOH) (25.5 g, 141 mmol) at room temperature. The reaction mixture was stirred at room temperature for 16 h, then the reaction mixture was extracted with saturated sodium bicarbonate solution in water (3 x 100 mL). The combined organic phases were adjusted to pH 1 using concentrated hydrogen chloride solution in water. The resulting mixture was extracted with ethyl acetate (3 x 100 mL). The organic phases were dried over sodium sulfate. The dried organic phase was filtered and concentrated under reduced pressure to afford the 3-[[(1R,4S)-4-isopropoxycarbonylcyclopent-2-en-1-yl]amino]-2-methoxy-3-oxo-propanoic acid (20 g, quantitative) as a mixture of diastereoisomers (1:1).

¹H NMR (500 MHz, chloroform-d) δ 9.64 (s, 1H), 7.65 - 7.43 (m, 1H), 6.05 - 5.79 (m, 2H), 5.13 - 4.96 (m, 2H), 4.32 (s, 1H), 3.70 - 3.60 (m, 3H), 3.55 - 3.45 (m, 1H), 2.55 - 2.41 (m, 1H), 2.01 - 1.89 (m, 1H), 1.31 - 1.21 (m, 6H).

### Example 2: Synthesis of isopropyl (1S,4R)-4-[[3-(benzofuran-4-ylamino)-2-methoxy-3-oxo-propanoyl]amino]cyclopent-2-ene-1-carboxylate (Cpd. I.6 in below table 1):

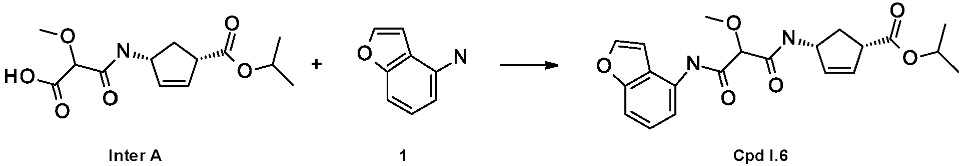

1-Propanephosphonic anhydride solution (T₃P) (1.14 g, 1.79 mmol, 50% in ethyl acetate) and triethylamine (0.29 mL, 2.1 mmol) were added to a solution of 3-[[(1R,4S)-4-isopropoxycarbonylcyclopent-2-en-1-yl]amino]-2-methoxy-3-oxo-propanoic acid **(Inter A)** (300 mg, 1.05 mmol) and benzofuran-4-amine (1) (168 mg, 1.26 mmol) in tetrahydrofuran (30 mL). After stirring at room temperature overnight, the mixture was poured into ice water and extracted with ethyl acetate (3x). The extracts were washed with brine, dried, concentrated, purified by column chromatography using ethyl acetate as solvent to yield the title compound Cpd I.6 (150 mg, 36% yield) as a 1:1 mixture of diastereoisomers.

¹H NMR (500 MHz, Chloroform-d) δ 9.44 - 9.25 (m, 1H), 7.86 - 7.74 (m, 1H), 7.64 - 7.51 (m, 1H), 7.42 - 7.18 (m, 3H), 6.93 - 6.76 (m, 1H), 6.00 - 5.78 (m, 2H), 5.13 - 5.05 (m, 1H), 5.01 (h, *J* = 6.2 Hz, 1H), 4.43 - 4.35 (m, 1H), 3.75 - 3.62 (m, 3H), 3.51 - 3.43 (m, 1H), 2.56 - 2.42 (m, 1H), 2.01 - 1.89 (m, 1H), 1.28 - 1.18 (m, 6H).

### Example 3: Synthesis of isopropyl (1S,4R)-4-[[3-[(2,2-difluoro-1,3-benzodioxol-4-yl)amino]-2-methoxy-3-oxo-propanoyl]amino]cyclopent-2-ene-1-carboxylate (Cpd. I.8 in below table 1):

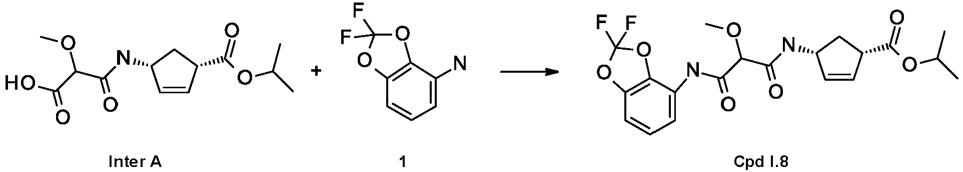

According to the procedure used in Example 2, 1-propanephosphonic anhydride solution (T₃P) (1.14 g, 1.79 mmol, 50% in ethyl acetate) was added to a solution of 3-[[(1R,4S)-4-isopropoxycarbonylcyclopent-2-en-1-yl]amino]-2-methoxy-3-oxo-propanoic acid **(Inter A)** (300 mg, 1.05 mmol) and 2,2-difluoro-1,3-benzodioxol-4-amine (**1**) (0.22 g, 1.26 mmol) in tetrahydrofuran (30 mL). After stirring at room temperature overnight, the mixture was poured into ice water and extracted with ethyl acetate (3x). The combined extracts were washed with brine, dried, concentrated, purified by column chromatography using ethyl acetate as solvent to yield the title compound Cpd I.8 (180 mg, 39% yield) as a 1:1 mixture of diastereoisomers.

¹H NMR (500 MHz, chloroform-d) δ 9.14 - 9.04 (m, 1H), 7.92 - 7.87 (m, 1H), 7.31 - 7.23 (m, 1H), 7.08 - 7.00 (m, 1H), 6.86 - 6.80 (m, 1H), 6.00 - 5.80 (m, 2H), 5.14 - 5.07 (m, 1H), 5.06 - 4.98 (m, 1H), 4.36 - 4.31 (m, 1H), 3.71 - 3.63 (m, 3H), 3.53 - 3.45 (m, 1H), 2.98 - 2.84 (m, 1H), 2.53 - 2.42 (m, 1H), 1.98 - 1.89 (m, 1H), 1.28 - 1.23 (m, 6H).

### Example 4: Synthesis of isopropyl (1S,4R)-4-[[3-(1,3-benzothiazol-4-ylamino)-2-methoxy-3-oxo-propanoyl]amino]cyclopent-2-ene-1-carboxylate (Cpd. I.9 in below table 1):

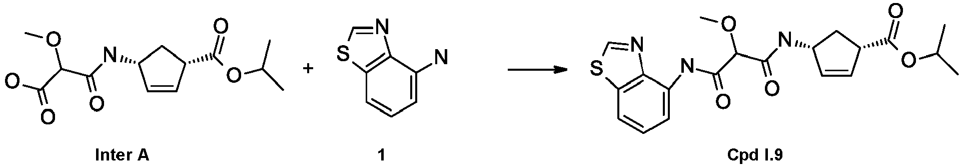

According to the procedure used in Example 2, 1-propanephosphonic anhydride solution (T₃P) (1.14 g, 1.79 mmol, 50% in ethyl acetate) was added to a solution of 3-[[(1R,4S)-4-isopropoxycarbonylcyclopent-2-en-1-yl]amino]-2-methoxy-3-oxo-propanoic acid **(Inter A)** (300 mg, 1.05 mmol) and 1,3-benzothiazol-4-amine (1) (0.19 g, 1.26 mmol) in tetrahydrofuran (30 mL). After stirring at room temperature overnight, the mixture was poured into ice water and extracted with ethyl acetate (3x). The combined extracts were washed with brine, dried, concentrated, purified by column chromatography using ethyl acetate as solvent to yield the title compound Cpd I.9 (300 mg, 68% yield) as a 1:1 mixture of diastereoisomers.

¹H NMR (400 MHz, chloroform-d) δ 9.49 - 9.32 (m, 1H), 8.98 (d, *J* = 1.5 Hz, 1H), 7.96 - 7.89 (m, 1H), 7.85 - 7.78 (m, 1H), 7.50 - 7.37 (m, 2H), 5.99 - 5.80 (m, 2H), 5.15 - 5.06 (m, 1H), 5.05 - 4.95 (m, 1H), 4.48 - 4.39 (m, 1H), 3.70 - 3.63 (m, 3H), 3.52 - 3.44 (m, 1H), 2.56 - 2.42 (m, 1H), 2.01 - 1.91 (m, 1H), 1.28 - 1.20 (m, 6H).

### Example 5: Synthesis of isopropyl (1S,4R)-4-[[2-methoxy-3-[(1-methylindol-4-yl)amino]-3-oxo-propanoyl]amino]cyclopent-2-ene-1-carboxylate (Cpd. I.10 in below table 1):

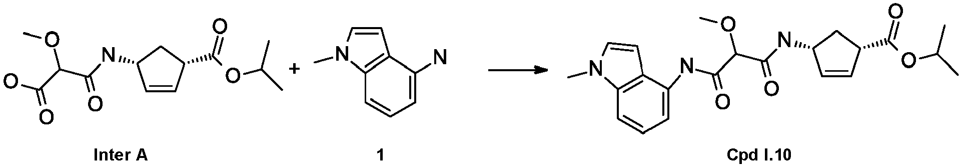

According to the procedure used in Example 2, 1-propanephosphonic anhydride solution (T₃P) (1.14 g, 1.79 mmol, 50% in ethyl acetate) was added to a solution of 3-[[(1R,4S)-4-isopropoxycarbonylcyclopent-2-en-1-yl]amino]-2-methoxy-3-oxo-propanoic acid (**Inter A**) (300 mg, 1.05 mmol) and 1-methylindol-4-amine (1) (184 mg, 1.26 mmol) in tetrahydrofuran (30 mL). After stirring at room temperature overnight, the mixture was poured into ice water and extracted with ethyl acetate (3x). The combined extracts were washed with brine, dried, concentrated, purified by column chromatography using ethyl acetate as solvent to yield the title compound Cpd I.10 (300 mg, 68% yield) as a 1:1 mixture of diastereoisomers.

¹H NMR (400 MHz, chloroform-d) δ 9.30 - 9.17 (m, 1H), 7.89 - 7.78 (m, 1H), 7.35 - 7.25 (m, 1H), 7.18 (t, *J* = 7.9 Hz, 1H), 7.09 (d, *J =* 8.3 Hz, 1H), 7.01 (d, *J =* 3.3 Hz, 1H), 6.51 (d, *J* = 3.2 Hz, 1H), 5.97 - 5.78 (m, 2H), 5.13 - 5.05 (m, 1H), 5.05 - 4.96 (m, 1H), 4.36 (d, *J* = 6.1 Hz, 1H), 3.73 (s, 3H), 3.71 - 3.65 (m, 3H), 3.50 - 3.42 (m, 1H), 2.55 - 2.42 (m, 1H), 1.98 - 1.86 (m, 1H), 1.27 - 1.21 (m, 6H).

### Example 5: Synthesis of isopropyl (1S,4R)-4-[[3-(1,3-benzoxazol-4-ylamino)-2-methoxy-3-oxo-propanoyl]amino]cyclopent-2-ene-1-carboxylate (Cpd. I.11 in below table 1):

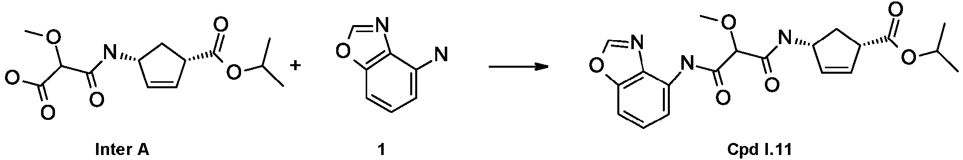

According to the procedure used in Example 2, 1-propanephosphonic anhydride solution (T₃P) (1.14 g, 1.79 mmol, 50% in ethyl acetate) was added to a solution of 3-[[(1R,4S)-4-isopropoxycarbonylcyclopent-2-en-1-yl]amino]-2-methoxy-3-oxo-propanoic acid **(Inter A)** (300 mg, 1.05 mmol) and 1,3-benzoxazol-4-amine (**1**) (169 mg, 1.26 mmol) in tetrahydrofuran (30 mL). After stirring at room temperature overnight, the mixture was poured into ice water and extracted with ethyl acetate (3x). The combined extracts were washed with brine, dried, concentrated, purified by column chromatography using ethyl acetate as solvent to yield the title compound Cpd I.11 (150 mg, 36% yield) as a 1:1 mixture of diastereoisomers.

¹H NMR (400 MHz, Chloroform-d) δ 9.84 (s, 1H), 8.29 (d, *J =* 7.9 Hz, 1H), 8.09 (s, 1H), 7.41 - 7.22 (m, 3H), 6.00 - 5.79 (m, 2H), 5.19 - 5.08 (m, 1H), 5.02 (h, *J* = 6.3 Hz, 1H), 4.46 - 4.37 (m, 1H), 3.75 - 3.63 (m, 3H), 3.53 - 3.43 (m, 1H), 2.50 (tt, *J* = 14.3, 8.4 Hz, 1H), 1.94 (tt, *J* = 14.1, 3.8 Hz, 1H), 1.31 - 1.18 (m, 6H).

High Performance Liquid Chromatography: HPLC-column Kinetex XB C18 1,7µ (50 x 2,1 mm); eluent: acetonitrile / water + 0.1% trifluoroacetic acid (gradient from 5:95 to 100 : 0 in 1.5 min at 60° C, flow gradient from 0.8 to 1.0 ml/min in 1.5 min).

In analogy to the examples described above, the following compounds of formula (I), wherein R¹ and R⁴ are hydrogen, were prepared, starting from commercially available diesters and using commercially available amines:

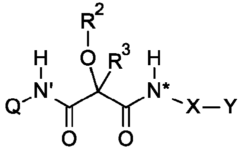

**Table 1**

| HPLC/MS = MassChargeRatio | | | | | |
|---|---|---|---|---|---|
| Cpd | Q-N' | R² | R³ | N*-X-Y | HPLC/MS |
| I.1 | 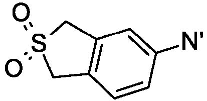 | CH₃ | H | 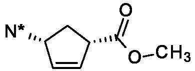 | 423.2 |
| I.2 | 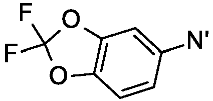 | CH₃ | H | 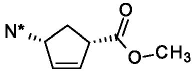 | 413.2 |
| I.3 | 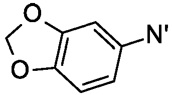 | CH₃ | H | 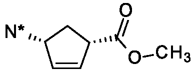 | 377.2 |
| I.4 | 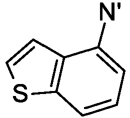 | CH₃ | H | 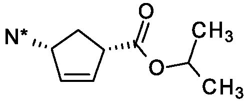 | 417.0 |
| I.5 | 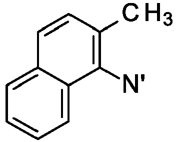 | CH₃ | H | 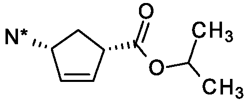 | 425.1 |
| I.6 | 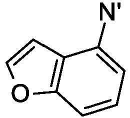 | CH₃ | H | 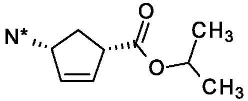 | 401.1 |
| I.7 | 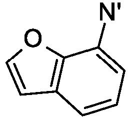 | CH₃ | H | 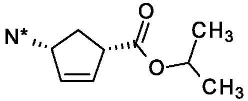 | 401.1 |
| I.8 | 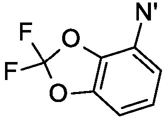 | CH₃ | H | 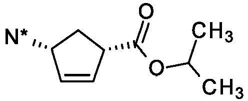 | 441.0 |
| I.9 | 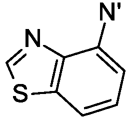 | CH₃ | H | 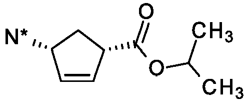 | 418.3 |
| I.10 | 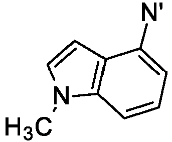 | CH₃ | H | 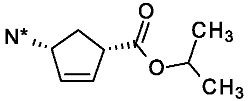 | 414.4 |
| I.11 | 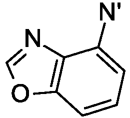 | CH₃ | H | 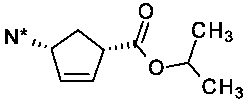 | 402.3 |
| I.12 | 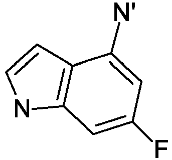 | CH₃ | H | 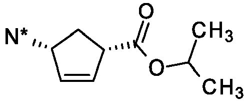 | 418.0 |
| I.13 | 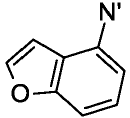 | CH₃ | H | 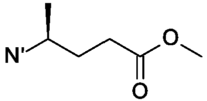 | 363.2 |
| I.14 | 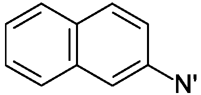 | CH₃ | H | 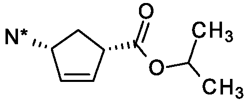 | 411.2 |

### B Biological examples

The herbicidal activity of the compounds of formula (I) was demonstrated by the following greenhouse experiments:
The culture containers used were plastic flowerpots containing loamy sand with approximately 3.0% of humus as the substrate. The seeds of the test plants were sown separately for each species.

For the pre-emergence treatment, the active ingredients, which had been suspended or emulsified in water, were applied directly after sowing by means of finely distributing nozzles. The containers were irrigated gently to promote germination and growth and subsequently covered with transparent plastic hoods until the test plants had rooted. This cover caused uniform germination of the test plants, unless this had been impaired by the active ingredients.

For the post-emergence treatment, the test plants were first grown to a height of 3 to 15 cm, depending on the plant habit, and only then treated with the active ingredients which had been suspended or emulsified in water. For this purpose, the test plants were either sown directly and grown in the same containers, or they were first grown separately as seedlings and transplanted into the test containers a few days prior to treatment.

Depending on the species, the test plants were kept at 10 -25° C or 20 - 35° C, respectively.

The test period extended over 2 to 4 weeks. During this time, the test plants were tended, and their response to the individual treatments was evaluated.

Evaluation was carried out using a scale from 0 to 100. 100 means no emergence of the test plants, or complete destruction of at least the aerial moieties, and 0 means no damage, or normal course of growth. A good herbicidal activity is given at values of 65 to < 90 and a very good herbicidal activity is given at values of 90 to 100.

The test plants used in the greenhouse experiments were of the following species:

| Bayer code | Scientific name |
|---|---|
| ABUTH | *Abutilon theophrasti* |
| ALOMY | *Alopercurus myosuroides* |
| AMARE | *Amaranthus retroflexus* |
| APESV | *Apera spica-venti* |
| AVEFA | *Avena fatua* |
| ECHCG | *Echinocloa crus-galli* |
| SETFA | *Setaria faberi* |
| SETVI | *Setaria viridis* |

At an application rate of 0.125 kg/ha, applied by the pre-emergence method:
- compound I.1 showed good herbicidal activity against ECHCG
- compound I.14 showed good herbicidal activity against SETFA

At an application rate of 0.250 kg/ha, applied by the pre-emergence method:
- compounds I.10, I.11 showed good herbicidal activity against ABUTH
- compounds I.6 showed very good herbicidal activity against APESV
- compounds I.4, I.7, I.8 showed good herbicidal activity against APESV
- compounds I.6, I.9 showed good herbicidal activity against ECHCG
- compounds I.6, I.8 showed good herbicidal activity against SETFA

At an application rate of 0.250 kg/ha, applied by the post-emergence method:
- compounds I.4, I.11 showed very good herbicidal activity against ABUTH
- compounds I.2, I.7, I.8 showed good herbicidal activity against ABUTH
- compounds I.2, I.3, I.6, I.7 showed good herbicidal activity against ALOMY
- compounds I.11 showed very good herbicidal activity against AMARE
- compound 1.13 showed good herbicidal activity against AMARE
- compounds I.2, I.4 showed very good herbicidal activity against AVEFA
- compounds I.3, I.8 showed good herbicidal activity against AVEFA
- compounds I.6 showed very good herbicidal activity against ECHCG
- compounds I.6, I.7, I.8 showed very good herbicidal activity against SETVI
- compounds I.4, I.9 showed good herbicidal activity against SETVI

## Claims

1. Compounds of the formula (I) 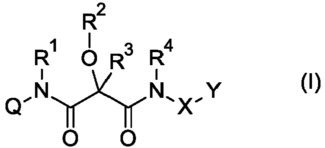 wherein the substituents have the following meanings:
Q is selected from bicyclic condensed ring systems of formula Q1 to Q13: 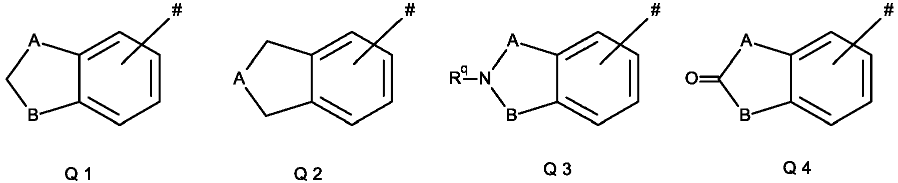 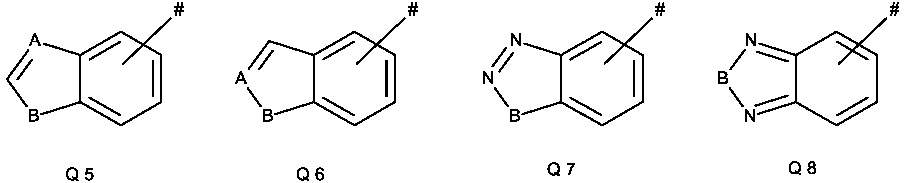 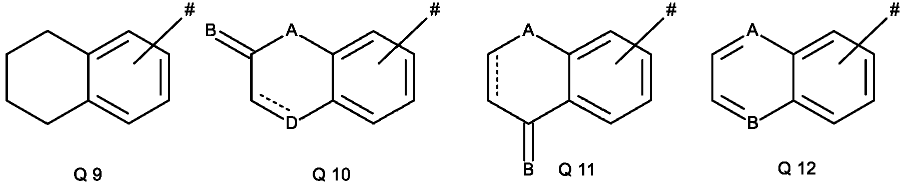 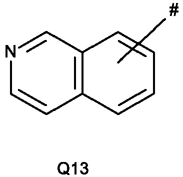
where
| | |
|---|---|
| in Q1: | A is CH₂, NR^{q}, O, S, S(O) or S(O)₂; and |
| | B is CH₂, NR^{q}, O, S, S(O) or S(O)₂; |
| in Q2: | A is NR^{q}, O, S, S(O) or S(O)₂; |
| in Q3: | A is CH₂, C(=O), NR^{q}, O, S, S(O) or S(O)₂; and |
| | B is CH₂, C(=O), NR^{q}, O, S, S(O) or S(O)₂; |
| in Q4: | A is CH₂, NR^{q}, O, S, S(O) or S(O)₂; and |
| | B is CH₂, NR^{q}, O, S, S(O) or S(O)₂; |
| in Q5: | A is CH or N; and |
| | B is CH₂, NR^{q}, O, S, S(O) or S(O)₂; |
| in Q6: | A is CH or N; and |
| | B is CH₂, NR^{q}, O, S, S(O) or S(O)₂; |
| in Q7: | B is CH₂, NR^{q}, O, S, S(O) or S(O)₂; |
| in Q8: | B is CH₂, NR^{q}, O, S, S(O) or S(O)₂; |
| in Q10: | the dashed line indicates a single bond or a double bond |
| | A is NR^{q} or O; |
| | B is O; and |
| | D is CH₂, NR^{q} or O if the dashed line indicates a single bond; and is CH or N the dashed line indicates a double bond; |
| in Q11: | the dashed line indicates a single bond or a double bond |
| | A is NR^{q} or O; and |
| | B is O; |
| in Q12: | A is CH or N; and |
| | B is CH or N; |
where
R^{a} is hydrogen, (C₁-C₃)-alkyl, (C₁-C₃)-haloalkyl, (C₁-C₃)-alkoxy, (C₁-C₃)-haloalkoxy, phenyl-(C₁-C₃)-alkyl, (C₁-C₄)-alkylcarbonyl or (C₁-C₄)-alkoxycarbonyl; and
# is the attachment point to NR¹;
where rings Q1 to Q13 carry k substituents R^{Q1}; where R^{Q1} is selected from the group consisting of halogen, cyano, (C₁-C₃)-alkyl, (C₁-C₃)-haloalkyl, (C₁-C₃)-alkoxy and (C₁-C₃)-haloalkoxy; where R^{q} is hydrogen or (C₁-C₃)-alkyl; and where k is 0, 1, 2 or 3;
R¹ is hydrogen;
R² is (C₁-C₆)-alkyl;
R³ is hydrogen or halogen;
R⁴ is hydrogen;
X is a bond; and Y is Z; where Z is a five- or six-membered saturated or partly unsaturated carbocyclic ring which is substituted by m radicals CO₂R^{e}, where R^{e} is hydrogen or (C₁-C₆)-alkyl and m is 1 or 2; or
X is a divalent unit (X¹); and Y is (C₁-C₄)-alkyl which is substituted by m radicals CO₂R^{e}, where R^{e} is hydrogen or (C₁-C₆)-alkyl and m is 1 or 2;
where the divalent unit (X¹) has the following formula: 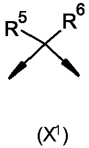
where R⁵ and R⁶ are independently of each other hydrogen or (C₁-C₆)-alkyl;
and the agriculturally acceptable salts, the stereoisomers and the tautomers thereof.

2. The compounds as claimed in claim 1, where
R² is methyl or ethyl; preferably methyl; and
R³ is hydrogen.

3. The compounds as claimed in any of claims 1 or 2, where Z is a five- or six-membered partly unsaturated carbocyclic ring which is substituted by m radicals CO₂R^{e}, where R^{e} is hydrogen or (C₁-C₆)-alkyl and m is 1 or 2.

4. The compounds as claimed in claim 3, where Z is a five-membered partly unsaturated carbocyclic ring which is substituted by one radical CO₂R^{e}, where R^{e} is hydrogen or (C₁-C₆)-alkyl.

5. The compounds as claimed in any of claims 1 or 2, wherein
X is a divalent unit (X¹), where one of R⁵ and R⁶ hydrogen and the other is hydrogen or methyl; and
Y is is (C₁-C₄)-alkyl which is substituted by m radicals CO₂R^{e}, where R^{e} is hydrogen or (C₁-C₆)-alkyl; where m is 1 or 2 and preferably 1.

6. The compounds as claimed in any of claims 1 to 5, where m is 1.

7. The compounds as claimed in any of claims 1 to 6, where Q is a bicyclic condensed ring system of formula Q1, Q2, Q5 or Q12.

8. The compounds as claimed in claim 7,
where Q is a bicyclic condensed ring system of formula Q1, Q2, Q5 or Q12,
where in Q1 A and B are O; in Q2 A is S, S(O) or S(O)₂, preferably S(O)₂; in Q5 A is CH or N and B is NR^{q}, O or S; and in Q12 A and B are CH; where rings Q1, Q2, Q5 and Q12 carry k substituents R^{Q1}; where R^{Q1} is selected from the group consisting of halogen and (C₁-C₃)-alkyl; where R^{q} is hydrogen or (C₁-C₃)-alkyl; and where k is 0, 1 or 2.

9. The compounds as claimed in any of claims 1 to 8, where
Q is a bicyclic condensed ring system of formula Q1, Q2, Q5 or Q12; where in Q1 A and B are O; in Q2 A is S, S(O) or S(O)₂, preferably S(O)₂; in Q5 A is CH or N and B is NR^{q}, O or S; and in Q12 A and B are CH; where rings Q1, Q2, Q5 and Q12 carry k substituents R^{Q1}; where R^{Q1} is selected from the group consisting of halogen and (C₁-C₃)-alkyl; where R^{q} is hydrogen or (C₁-C₃)-alkyl; and where k is 0, 1 or 2;
R¹ hydrogen;
R² is methyl or ethyl;
R³ is hydrogen;
R⁴ is hydrogen;
X is a bond; and Y is Z; where Z is a five-membered partly unsaturated carbocyclic ring which is substituted by one radical CO₂R^{e}, where R^{e} is (C₁-C₆)-alkyl; or
X is a divalent unit (X¹), where R⁵ and R⁶ are independently of each other hydrogen or methyl; and Y is (C₁-C₄)-alkyl which is substituted by one radical CO₂R^{e}, where R^{e} is (C₁-C₆)-alkyl.

10. A composition comprising at least one compound as claimed in any one of claims 1 to 9, and at least one auxiliary, which is customary for formulating crop protection compounds.

11. The composition as claimed in claim 10, comprising a further herbicide.

12. The use of a compound as claimed in any one of claims 1 to 9, or a composition as claimed in claims 10 or 11 for controlling unwanted vegetation.

13. A method for controlling unwanted vegetation which comprises allowing a herbicidally effective amount of at least one compound as claimed in any one of claims 1 to 9, or a composition as claimed in claim 10 or 11 to act on plants, their seed and/or their habitat.

## Patentansprüche

1. **Verbindungen der Formel (I)** 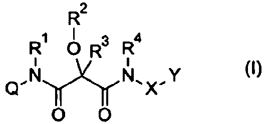 wobei die Substituenten die folgenden Bedeutungen haben:
Q ist ausgewählt aus bicyclischen kondensierten Ringsystemen der Formel QI bis Q13: 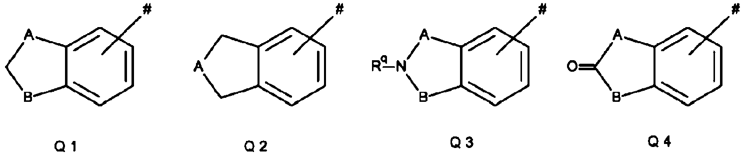 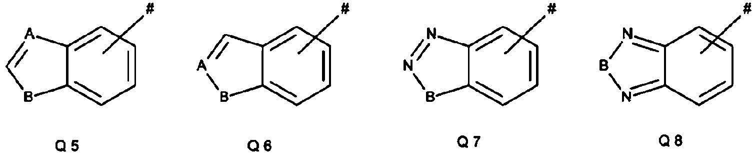 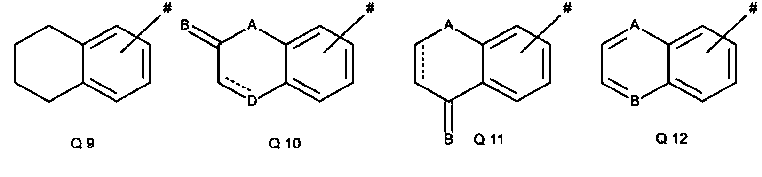 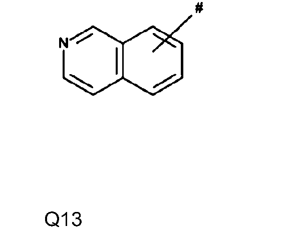
wobei
| | |
|---|---|
| in Q1: | A ist CH₂, NR^{q}, O, S, S(O) oder S(O)₂ ist; und |
| | B ist CH₂, NR^{q}, O, S, S(O) oder S(O)₂ ist; in |
| Q2: | A NR^{q}, O, S, S(O) oder S(O)₂ ist; |
| in Q3: | A ist CH₂, C(=O), NR^{q}, O, S, S(O) oder S(O)₂; und |
| | B ist CH₂, C(=O), NR^{q}, O, S, S(O) oder S(O)₂; |
| in Q4: | A ist CH2, C(=O), NRq, O, S, S(O) oder S(O)₂; und |
| | B ist CH₂, NR^{q}, O, S, S(O) oder S(O)₂; |
| in Q5: | A ist CH oder N; und |
| | B ist CH₂, NR^{q}, O, S, S(O) oder S(O)₂; |
| in Q6: | A ist CH oder N; und |
| | B ist CH₂, NR^{q}, O, S, S(O) oder S(O)₂; |
| in Q7: | B istCH₂, NR^{q}, O, S, S(O) oder S(O)₂; |
| in Q8: | B ist CH₂, NR^{q}, O, S, S(O) oder S(O)₂; |
| in QI0: | die gestrichelte Linie eine Einfachbindung oder eine Doppelbindung anzeigt |
| | A NR^{q} oder O ist; |
| | B O ist; und |
| | D CH₂, NR^{q} oder O ist, wenn die gestrichelte Linie eine Einfachbindung anzeigt; und CH oder N ist, wenn die gestrichelte Linie eine Doppelbindung anzeigt; |
| inQ11: | die gestrichelte Linie eine Einfachbindung oder eine Doppelbindung anzeigt |
| | A NR^{q} oder O ist; und |
| | B O ist; |
| in Q12: | A CH oder N ist; und |
| | B CH oder N ist; |
wobei
R^{q} Wasserstoff, (C₁-C₃)-Alkyl, (C₁-C₃)-Halogenalkyl, (C₁-C₃)-Alkoxy, (C₁-C₃)-Halogenalkoxy, Phenyl-(C₁-C₃)-alkyl, (C₁-C₄)-Alkylcarbonyl oder (C₁-C₄)-Alkoxycarbonyl ist; und
# die Verknüpfungsstelle mit NR¹ ist;
wobei die Ringe QI bis Q13 k Substituenten RQ1 tragen; wobei RQ1 ausgewählt ist aus der Gruppe bestehend aus Halogen, Cyano, (C₁-C₃)-Alkyl, (C₁-C₃)-Halogenalkyl, (C₁-C₃)-Alkoxy und (C₁-C₃)-Halogenalkoxy; wobei R^{q} Wasserstoff oder (C₁-C₃)-Alkyl ist; und wobei k 0, 1, 2 oder 3 ist;
R¹ Wasserstoff ist;
R² (C₁-C₆)-Alkyl ist;
R³ Wasserstoff oder Halogen ist;
R⁴ Wasserstoff ist;
X eine Bindung ist; und Y Z ist; wobei Z ein fünf- oder sechsgliedriger gesättigter
oder teilweise ungesättigter carbocyclischer Ring ist, der durch m Reste CO₂R^{e} substituiert ist, wobei R^{e} Wasserstoff oder (C₁-C₆)-Alkyl ist und m 1 oder 2 ist; oder
X eine zweiwertige Einheit (X¹) ist; und Y (C₁-C₄)-Alkyl ist, das durch m Reste CO₂R^{e} substituiert ist, wobei R^{e} Wasserstoff oder (C₁-C₆)-Alkyl ist und m 1 oder 2 ist; wobei die zweiwertige Einheit (X1) die folgende Formel hat: 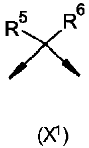 wobei R⁵ und R⁶ unabhängig voneinander Wasserstoff oder (C₁-C₆)-Alkyl sind;
sowie die landwirtschaftlich akzeptablen Salze, die Stereoisomere und die **Tautomere davon.**

2. **Die Verbindungen nach Anspruch 1, wobei** R²
**Methyl oder Ethyl ist; vorzugsweise Methyl; und** R³ **Wasserstoff ist.**

3. Die Verbindungen nach einem der Ansprüche 1 oder 2, wobei Z ein fünf- oder sechsgliedriger teilweise ungesättigter carbocyclischer Ring ist, der durch m Reste CO2R^{e} substituiert ist, wobei R^{e} Wasserstoff oder (C₁-C₆)-Alkyl ist und m 1 oder 2 ist.

4. Die Verbindungen nach Anspruch 3, wobei Z ein fünfgliedriger teilweise ungesättigter carbocyclischer Ring ist, der durch einen Rest CO₂R^{e} substituiert ist, wobei R^{e} Wasserstoff oder (C₁-C₆)-Alkyl ist.

5. Die Verbindungen nach einem der Ansprüche 1 oder 2, wobei
X eine zweiwertige Einheit (X¹) ist, wobei einer von R⁵ und R⁶ Wasserstoff ist und der andere Wasserstoff oder Methyl ist; und
Y (C₁-C₄)-Alkyl ist, das durch m Reste CO₂R^{e} substituiert ist, wobei R^{e} Wasserstoff oder (C₁-C₆)-Alkyl ist; wobei m 1 oder 2 und vorzugsweise 1 ist.

6. Die Verbindungen nach einem der Ansprüche 1 bis 5, wobei m 1 ist.

7. Die Verbindungen nach einem der Ansprüche 1 bis 6, wobei Q ein bicyclisches kondensiertes Ringsystem der Formel QI, Q2, Q5 oder Q12 ist.

8. Die Verbindungen nach Anspruch 7,
wobei Q ein bicyclisches kondensiertes Ringsystem der Formel QI, Q2, Q5 oder Q12 ist, wobei in QI A und B O sind; in Q2 A S, S(O) oder S(O)₂, vorzugsweise S(O)₂, ist; in Q5 A CH oder N ist und B NR^{q}, O oder S ist; und in Q12 A und B CH sind; wobei die Ringe QI, Q2, Q5 und Q12 k Substituenten RQ1 tragen; wobei RQ1 ausgewählt ist aus der Gruppe bestehend aus Halogen und (C₁-C₃)-Alkyl; wobei R^{q} Wasserstoff oder (C₁-C₃)-Alkyl ist; und wobei k 0, 1 oder 2 ist.

9. Die Verbindungen nach einem der Ansprüche 1 bis 8, wobei
Q ein bicyclisches kondensiertes Ringsystem der Formel QI, Q2, Q5 oder Q12 ist; wobei in QI A und B O sind; in Q2 A S, S(O) oder S(O)₂, vorzugsweise S(O)₂, ist; in Q5 A CH oder N ist und B NR^{q}, 0 oder S ist; und in Q12 A und B CH sind; wobei die Ringe QI, Q2, Q5 und Q12 k Substituenten RQ1 tragen; wobei Roi ausgewählt ist aus der Gruppe bestehend aus Halogen und (C₁-C₃)-Alkyl; wobei R^{q} Wasserstoff oder (C₁-C₃)-Alkyl ist; und wobei k 0, 1 oder 2 ist;
R¹ Wasserstoff;
R² Methyl oder Ethyl ist;
R³ Wasserstoff ist;
R⁴ Wasserstoff ist;
X eine Bindung ist; und Y Z ist; wobei Z ein fünfgliedriger teilweise ungesättigter carbocyclischer Ring ist, der durch einen Rest CO₂Re substituiert ist, wobei R^{e} (C₁-C₆)-Alkyl ist; oder
X eine zweiwertige Einheit (X¹) ist, wobei R⁵ und R⁶ unabhängig voneinander Wasserstoff oder Methyl sind; und Y (C₁-C₄)-Alkyl ist, das durch einen radikal CO₂R^{e}, wobei R^{e} ein (C₁-C₆)-Alkylrest ist.

10. Eine Zusammensetzung, die mindestens eine Verbindung nach einem der Ansprüche 1 bis 9 und mindestens ein Hilfsmittel umfasst, das zur Formulierung von Pflanzenschutzmitteln üblich ist.

11. Die Zusammensetzung nach Anspruch 10, die ein weiteres Herbizid umfasst.

12. Die Verwendung einer Verbindung nach einem der Ansprüche 1 bis 9 oder einer Zusammen setzung nach Anspruch 10 oder 11 zur Bekämpfung unerwünschter Vegetation.

13. Ein Verfahren zur Bekämpfung unerwünschter Vegetation, das umfasst, eine herbizid wirksame Menge mindestens einer Verbindung nach einem der Ansprüche 1 bis 9 oder einer Zusammensetzung nach Anspruch 10 oder 11 auf Pflanzen, ihr Saatgut und/oder ihren Lebensraum einwirken zu lassen.

## Revendications

1. Composés de formule (I) 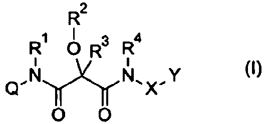 dans laquelle les substituants ont les significations suivantes :
Q est choisi parmi des systèmes cycliques bicycliques condensés de formule QI à Q13 : 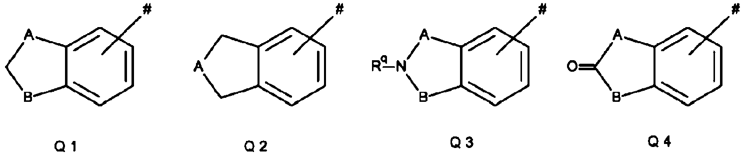 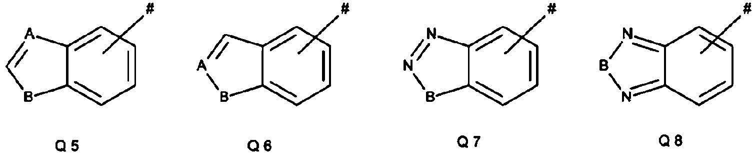 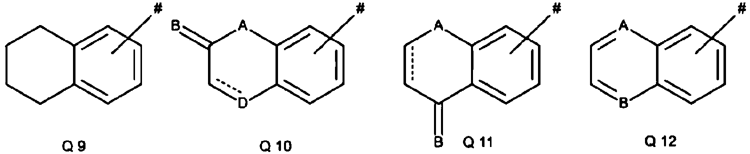 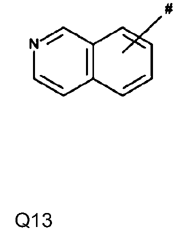 où
| | |
|---|---|
| dans Q1 : | A est CH₂, NR^{q}, O, S, S(O) ou S(O)₂ ; et |
| | B est CH₂, NR^{q}, O, S, S(O) ou S(O)₂; |
| dans Q2 : | A est NR^{q}, O, S, S(O) ou S(O)₂ ; |
| dans Q3 : | A est CH₂, C(=O), NR^{q}, O, S, S(O) ou S(O)₂ ; et |
| | B est CH₂, C(=O), NR^{q}, O, S, S(O) ou S(O)₂ ; |
| dans Q4 : | Aest CH2, C(=O), NR^{q}, O, S, S(O) ou S(O)2; |
| | et |
| | B est CH₂, NR^{q}, O, S, S(O) ou S(O)₂; |
| dans Q5 : | A est CH ou N ; et |
| | B est CH₂, NR^{q}, O, S, S(O) ou S(O)₂; |
| dans Q6 : | A est CH ou N ; et |
| | B est CH₂, NR^{q}, O, S, S(O) ou S(O)₂; |
| dans Q7 : | B est CH₂, NR^{q}, O, S, S(O) ou S(O)₂; |
| dans Q8 : | B est CH₂, NR^{q}, O, S, S(O) ou S(O)₂ ; |
| dansQI0: | le trait en pointillé indique une liaison simple ou une liaison double |
| | A est NR^{q} ou O; |
| | B est O ; et |
| | D est CH₂, NR^{q} ou O si le trait en pointillé indique une liaison simple ; et |
| | est CH ou N si le trait en pointillé indique une liaison double ; |
| dansQ11: | le trait en pointillé indique une liaison simple ou une liaison double |
| | A est NR^{q} ou O ; et |
| | B est O ; |
| dans Q12: | A est CH ou N ; et |
| | B est CH ou N ; |
où
R^{q} est hydrogène, (C₁-C₃)-alkyle, (C₁-C₃)-haloalkyle, (C₁-C₃)-alcoxy, (C₁-C₃)-haloalcoxy, phényl-(C₁-C₃)-alkyle, (C₁-C₄)-alkylcarbonyle ou (C₁-C₄)-alcoxycarbonyle ; et
# est le point d'attachement à NR¹ ;
où les cycles QI à Q13 portent k substituants RQ1 ; où RQ1 est choisi dans le groupe constitué par halogène, cyano, (C₁-C₃)-alkyle, (C₁-C₃)-haloalkyle, (C₁-C₃)-alcoxy et (C₁-C₃)-haloalcoxy ; où R^{q} est hydrogène ou (C₁-C₃)-alkyle ; et où k est 0, 1, 2 ou 3 ;
R¹ est hydrogène ;
R² est (C₁-C₆)-alkyle ;
R³ est hydrogène ou halogène ;
R⁴ est hydrogène;
X est une liaison ; et Y est Z ; où Z est un cycle carbocyclique saturé ou partiellement insaturé à cinq ou six chaînons qui est substitué par m radicaux CO2Re, où R^{e} est hydrogène ou (C₁-C₆)-alkyle et m est 1 ou ou
X est une unité divalente (X¹) ; et Y est (C₁-C₄)-alkyle substitué par m radicaux CO2R^{e}, où R^{e} est hydrogène ou (C1-C6)-alkyle et m est 1 ou 2 ;
où l'unité divalente (X1) a la formule suivante : 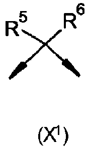
où R⁵ et R⁶ sont indépendamment l'un de l'autre hydrogène ou (C₁-C₆)-alkyle ;
ainsi que les sels agricolement acceptables, les stéréoisomères et les tautomères de ceux-ci.

2. Les composés selon la revendication 1, où R² est
méthyle ou éthyle ; de préférence méthyle ; et R³
est hydrogène.

3. Les composés selon l'une quelconque des revendications 1 ou 2, où Z est un cycle carbocyclique partiellement insaturé à cinq ou six chaînons qui est substitué par m radicaux CO2R^{e}, où R^{e} est hydrogène ou (C₁-C₆)-alkyle et m est 1 ou 2.

4. Les composés selon la revendication 3, où Z est un cycle carbocyclique partiellement insaturé à cinq chaînons substitué par un radical CO₂R^{e}, où R° est hydrogène ou (C₁-C₆)-alkyle.

5. Les composés selon l'une quelconque des revendications 1 ou 2, dans lesquels
X est une unité divalente (X¹), où l'un de R⁵ et R⁶ est hydrogène et l'autre est hydrogène ou méthyle ; et
Y Y est (C₁-C₄)-alkyle substitué par m radicaux CO₂R^{e}, où R^{e} est hydrogène ou (C₁-C₆)-alkyle ; où m est 1 ou 2 et de préférence 1.

6. Les composés selon l'une quelconque des revendications 1 à 5, où m est 1.

7. Les composés selon l'une quelconque des revendications 1 à 6, où Q est un système cyclique bicyclique condensé de formule QI, Q2, Q5 ou Q12.

8. Les composés selon la revendication 7,
où Q est un système cyclique bicyclique condensé de formule QI, Q2, Q5 ou Q12, où dans QI A et B sont O ; dans Q2 A est S, S(O) ou S(O)₂, de préférence S(O)₂ ; dans Q5 A est CH ou N et B est NR^{q}, O ou S ; et dans Q12 A et B sont CH ; où les cycles QI, Q2, Q5 et Q12 portent k substituants RQ1 ; où RQ1 est choisi dans le groupe constitué par halogène et (C₁-C₃)-alkyle ; où R^{q} est hydrogène ou (C₁-C₃)-alkyle ; et où k est 0, 1 ou 2.

9. Les composés selon l'une quelconque des revendications 1 à 8, où
Q est un système cyclique bicyclique condensé de formule QI, Q2, Q5 ou Q12 ; où dans QI A et B sont O ; dans Q2 A est S, S(O) ou S(O)₂, de préférence S(O)₂ ; dans Q5 A est CH ou N et B est NR^{q}, 0 ou S; et dans Q12 A et B sont CH ; où les cycles QI, Q2, Q5 et Q12 portent k substituants RQ1 ; où Roi est choisi dans le groupe constitué par halogène et (C₁-C₃)-alkyle ; où R^{q} est hydrogène ou (C₁-C₃)-alkyle ; et où k est 0, 1 ou 2 ;
R¹ hydrogène ;
R² est méthyle ou éthyle ;
R³ est hydrogène ;
R⁴ est hydrogène ;
X est une liaison ; et Y est Z ; où Z est un cycle carbocyclique partiellement insaturé à cinq chaînons substitué par un radical CO₂Re, où R^{e} est (C₁-C₆)-alkyle ; ou
X est une unité divalente (X¹), où R⁵ et R⁶ sont indépendamment l'un de l'autre hydrogène ou méthyle ; et Y est (C1-C4)-alkyle substitué par un radical CO₂R^{e}, où R^{e} est un groupement alkyle (C₁-C₆).

10. Une composition comprenant au moins un composé selon l'une quelconque des revendications 1 à 9, et au moins un auxiliaire, qui est habituel pour la formulation de produits phytosanitaires.

11. La composition selon la revendication 10, comprenant un herbicide supplémentaire.

12. L'utilisation d'un composé selon l'une quelconque des revendications 1 à 9, ou d'une composition selon les revendications 10 ou 11 pour lutter contre la végétation indésirable.

13. Un procédé pour lutter contre la végétation indésirable qui comprend le fait de permettre à une quantité herbicidement efficace d'au moins un composé selon l'une quelconque des revendications 1 à 9, ou d'une composition selon la revendication 10 ou 11 d'agir sur des plantes, leurs semences et/ou leur habitat.
